# NEUE EUROPÄISCHE PATENTSCHRIFT

(11) **EP 0 624 155 B2**
(45) Veröffentlichungstag und Bekanntmachung des Hinweises auf die Entscheidung über den Einspruch: **11.12.2002**
(45) Hinweis auf die Patenterteilung: 06.05.1998
(21) Anmeldenummer: 93902227.3
(22) Anmeldetag: 18.01.1993
(51) Int. Cl.: C07C 271/28, C07C 313/32, C07C 321/28, C07C 275/64, C07C 259/06, C07C 251/58, C07C 233/18, C07C 233/91, C07D 213/64, C07D 239/34, C07D 239/38

(54) **CARBAMATE UND DIESE ENTHALTENDE PFLANZENSCHUTZMITTEL**
CARBAMATES AND PLANT-PROTECTING AGENTS CONTAINING THEM
CARBAMATES ET AGENTS PHYTOSANITAIRES LES CONTENANT

(30) Priorität: 29.01.1992 DE 4202386; 26.06.1992 DE 4221007; 09.10.1992 DE 4234081; 09.10.1992 DE 4234028; 09.10.1992 DE 4234012; 09.10.1992 DE 4234067
(43) Veröffentlichungstag der Anmeldung: 17.11.1994
(73) Patentinhaber: BASF Aktiengesellschaft, 67063 Ludwigshafen (DE)
(72) Erfinder: MUELLER, Bernd, D-6710 Frankenthal (DE); SAUTER, Hubert, D-6800 Mannheim 1 (DE); ROEHL, Franz, D-6707 Schifferstadt (DE); DOETZER, Reinhard, D-6940 Weinheim (DE); LORENZ, Gisela, D-6730 Neustadt (DE); AMMERMANN, Eberhard, D-6148 Heppenheim (DE)
(86) Internationale Anmeldenummer: EP9300104
(87) Internationale Veröffentlichungsnummer: WO93015046

(56) Entgegenhaltungen:
- EP-A- 0 041 613
- EP-A- 0 051 871
- EP-A- 0 065 668
- EP-A- 0 081 207
- EP-A- 0 093 604
- EP-A- 0 093 620
- EP-A- 0 100 190
- EP-A- 0 110 442
- EP-A- 0 126 628
- EP-A- 0 498 396
- WO-A-80/00344
- GB-A- 574 995
- US-A- 3 116 995
- Patent Abstracts of Japan, Band 8, Nr 193, C-241, Zusammenfassung von JP-A- 59-84804 (SUMITOMO KAGAKU KOGYO K.K. et al.)
- Patent Abstracts of Japan, Band 8, Nr 130, C-229, Zusammenfassung von JP-A-59-42307 (SUMITOMO KAGAKU KOGYO K.K. et al.)
- Chem. Abstracts Data Base, 113:58892 & Chem. Lett. 4, 581-2, 1990
- Chem. Abstracts Data Base, 113:97142 & Synth. Commmun., 20(6), 887-92, 1990
- Chem. Abstracts Data Base, 101:1055508 & Carcinogesis 4(1), 67-75, 1983
- Chem. Abstracts Data Base, 75:63351 & Zh. Org. Khim.,7(5), 923-9, 1971
- Chem. Abstracts Data Base, 111:128750 & Toxicol. Lett. 48(1), 75-81, 1989
- Chem. Abstracts Data Base, 106:45410 & Drug Metab. Dispos., 14(4), 487-93, 1986
- Chem. Abstracts Data Base, 71:112578 & Biol. Aktiv. Soedin., 70-6, 1986
- Diss Abstr., Int. B 1977, 37(8), 3900-1
- J. Med. Chem., 1979, 981

## Beschreibung

Die vorliegende Erfindung betrifft Carbamate und ihre Verwendung als Pflanzenschutzmittel, insbesondere zur Bekämpfung von Pilzen.

Es ist bekannt Anilinderivate, z.B. den N-Phenyl-carbaminsäure-i-propylester oder den entsprechenden 3-Chlorphenylester (GB 574 995) oder den N-3,4-Dichlorphenyl-carbaminsäure-methylester (BE 612 550), als Pflanzenschutzmittel zu verwenden. Ihre fungizide Wirkung ist jedoch unbefriedigend.

Ferner sind aus EP-A 65 668 substituierte Phenylharnstoffderivate und aus EP-A 126 628 2-Halogenphenyl-carbaminsäurealkylester und die entsprechenden Thioderivate als Schädlingsbekämpfungsmittel bekannt. EP-A 93 620, EP-A 100 190 und die nachveröffentlichte EP-A 498 396 beschreiben substituierte N-Phenylcarbamate mit fungizider Wirkung.

Weiterhin sind einzelne Phenylcarbamate aus Chem. Lett., 4, 581 (1990); Zh. Org. Khim., 7, 923 (1971); Diss. Abstr. Int. B 1977, 37(8), 3900-1; und Carcinogensis, 4, 67 (1983) bekannt.

Es wurde nun überraschend gefunden, daß Carbamate der Formel I 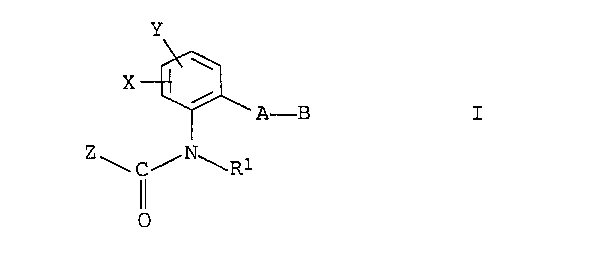 in der die Substituenten die folgenden Bedeutungen haben:
- Z: Methoxy, Amino, Methylamino, Dimethylamino, Methyl, Ethyl, Trifluormethyl oder Trichlormethyl;
- X,Y: Wasserstoff, Fluor, Chlor, Brom, Trifluormethyl, Cyano, Nitro, Alkyl, Alkenyl, Alkinyl, Alkoxy, Alkenyloxy, Alkinyloxy oder
- X,Y: sind gemeinsam zu einem ggf. substituierten aromatischen oder heteroaromatischen, alicyclischen oder heterocyclischen, partiell oder vollständig hydrierten Ring kondensiert;
- A: eine direkte Bindung, O, S, CR²=CR³, CHR²O, CHR²S, CHR²-ON=CR⁴, CR²=NO, ON=CR⁴, C≡C, CHR²-CHR³, CHR²O-CO oder OCHR²;
R²,R³ Wasserstoff, Alkyl, Alkenyl, Alkinyl oder Cycloalkyl;
R⁴ Wasserstoff, Cyano, Alkyl, Alkenyl, Alkinyl, Alkoxy oder Cycloalkyl;
- B: Wasserstoff,
ggf. subst. Alkyl, Alkenyl, Alkinyl, Cycloalkyl, Cycloalkenyl, Cycloalkinyl, Aryl, Hetaryl, Heterocyclyl, Arylalkyl, Hetarylalkyl, Cycloalkylalkyl oder Cycloalkenylalkyl;
- R¹: SR⁵, Alkoxy, Alkenyloxy, Alkinyloxy, Cycloalkoxy, Cycloalkenyloxy oder Alkoxycarbonyloxy;
R⁵ Alkyl, Cyclopropyl, Cyclopropylmethyl oder Cyclobutyl;
wobei B nicht Wasserstoff bedeutet, wenn A für eine direkte Bindung steht und Verbindungen mit folgenden Substituentenkombinationen ausgenommen bleiben:
- X,Y: Wasserstoff, Z Methyl, R¹ Methoxy, A-B Methoxy;
- X,Y: Wasserstoff, Z Amino, R¹ Ethoxycarbonyloxy, A-B Methyl;
- X,Y: 4,6-Dimethyl, Z Methyl, R¹ Methoxy, A-B Methyl;
- X,Y: Wasserstoff, Z Methyl, R¹ Methoxy, A-B Phenyl;
und ihre pflanzenverträglichen Säureadditionsprodukte und Basenadditionsprodukte, eine gute fungizide Wirkung haben.

Säuren für Säureadditionsprodukte sind z. B. Mineralsäuren, wie beispielsweise Chlorwasserstoffsäure, Bromwasserstoffsäure, Phosphorsäure, Schwefelsäure, Salpetersäure, oder aber Carbonsäuren, wie Ameisensäure, Essigsäure, Oxalsäure, Malonsäure, Milchsäure, Äpfelsäure, Bernsteinsäure, Weinsäure, Zitronensäure, Salicylsäure, p-Toluolsulfonsäure, Dodecylbenzolsulfonsäure, aber auch allgemein Protonen-acide Verbindungen, z. B. Saccharin.

Basen für Basenadditionsprodukte sind z. B. Kalium-, Natrium-, -hydroxid, -Carbonat, Ammoniumhydroxid.

Die neuen Verbindungen der Formel I können bei der Herstellung als Gemische von Stereoisomeren (E/Z-Isomere, Diastereomere, Enantiomere) anfallen, die in üblicher Weise, z. B. durch Kristallisation oder Chromatographie, in die Einzelkomponenten getrennt werden können. Sowohl die einzelnen Isomeren als auch ihre Gemische können als Fungizide verwendet werden und werden von der Erfindung erfaßt.

Die oben genannten Alkyle können substituiert sein und besitzen 1 bis 6 Kohlenstoffatome.

Die oben genannten Alkenyle und Alkinyle können substituiert sein und besitzen 2 bis 6 Kohlenstoffatome.

Die oben genannten Cycloalkyle besitzen 3 bis 10 Kohlenstoffatome und sind gegebenenfalls substituiert, beispielsweise mit 1 bis 4 gleichen oder verschiedenen Substituenten R⁶.

Die oben genannten Aryle besitzen 6, 10 oder 14 Kohlenstoffatome und sind gegebenenfalls substituiert, beispielsweise mit 1 bis 4 gleichen oder verschiedenen Substituenten R⁶.

Die oben genannten Hetaryle besitzen 5 bis 14 Ringatome, davon 1 bis 4 Heteroatome aus der Gruppe N, O, S, sind ungesättigt und gegebenenfalls substituiert, beispielsweise mit 1 bis 4 gleichen oder verschiedenen Substituenten R⁶.

Die oben genannten Heterocyclylen besitzen 5 bis 14 Ringatome, davon 1 bis 4 Heteroatome aus der Gruppe N, O, S sind gesättigt oder partiell ungesättigt und sind gegebenenfalls substituiert, beispielsweise mit 1 bis 4 gleichen oder verschiedenen Substituenten R⁶.

Die oben genannten Cycloalkenyle besitzen 5 bis 14 Kohlenstoffatome und sind gegebenenfalls substituiert, beispielsweise mit 1 bis 4 gleichen oder verschiedenen Substituenten R⁶.

Zwei benachbarte Substituenten R⁶ können zusammen mit den Kohlenstoffatomen, deren Substituenten sie sind, einen carbocyclischen hydrierten, partiell ungesättigten oder aromatischen Ring mit 3 bis 14 Kohlenstoffatomen oder auch einen heterocyclischen hydrierten, partiell ungesättigten oder heteroaromatischen Ring mit 3 bis 14 Ringatomen, davon 1 bis 4 Heteroatome aus der Gruppe N, O, S bilden.

R⁶ kann gegebenenfalls substituiert sein, beispielsweise mit 1 bis 4 gleichen oder verschiedenen Substituenten R⁷ und R⁶ bedeutet beispielsweise Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Alkoxy, Alkenyloxy, Alkinyloxy, Cycloalkyloxy, Aryloxy, Hetaryloxy, Heterocyclyloxy, Cycloalkenyloxy, Alkoximino, Alkenyloximino, Alkinyloximino, Cycloalkyloximino, Cycloalkenyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Alkylaminocarbonyl, Dialkylaminocarbonyl, Alkenylaminocarbonyl, Dialkenylaminocarbonyl, Alkylthio, Alkenylthio, Alkinylthio, Cycloalkylthio, Arylthio, Hetarylthio, Heterocyclylthio, Cycloalkenylthio, Alkylamino, Alkenylamino, Alkinylamino, Cycloalkylamino, Arylamino, Hetarylamino, Heterocyclylamino, Cycloalkenylamino, Alkylcarbonyl, Alkenylcarbonyl, Alkinylcarbonyl, Cycloalkylcarbonyl, Arylcarbonyl, Hetarylcarbonyl, Heterocyclylcarbonyl Cycloalkenylcarbonyl, Alkylsulfoxyl, Alkenylsutfoxyl, Alkinylsulfoxyl, Cycloalkylsulfoxyl, Arylsulfoxyl, Hetarylsulfoxyl, Heterocyclylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Alkylsulfinyl, Alkenylsulfinyl, Alkinylsulfinyl, Cycloatkylsulfinyl, Arylsulfinyl, Hetarylsulfinyl, Heterocyclylsulfinyl oder Cycloalkenylsulfinyl.

R⁷ ist ein beliebiger Substituent und bedeutet beispielsweise Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Alkoxy, Alkenyloxy, Alkinyloxy, Cycloalkyloxy, Aryloxy, Hetaryloxy, Heterocyclyloxy, Cycloalkenyloxy, Alkoximino, Alkenyloximino, Alkinyloximino, Cycloalkyloximino, Cycloalkenyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Alkylaminocarbonyl, Dialkyl- aminocarbonyl, Alkenylaminocarbonyl, Dialkenylaminocarbonyl, Alkylthio, Alkenythio, Alkinylthio, Cycloalkylthio, Arylthio, Hetarylthio, Heterocyclylthio, Cycloalkenylthio, Alkylamino, Alkenylamino, Alkinylamino, Cycloalkylamino, Arylamino, Hetarylamino, Heterocyclylamino, Cycloalkenylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Alkylsutfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl oder Cycloalkenylsulfinyl.

Die vorstehend genannten Alkyle können substituiert sein, besitzen bevorzugt 1 bis 6 Kohlenstoffatome und bedeuten insbesondere Methyl, Ethyl, Propyl, n-Propyl, i-Propyl, Butyl, n-Butyl, i-Butyl, t-Butyl, s-Butyl, Pentyl oder Hexyl.

Die vorstehend genannten Alkenyle können substituiert sein, besitzen bevorzugt 2 - 6 Kohlenstoffatome und bedeuten insbesondere Ethenyl, Propenyl, Propen-(1)-yl, Propen-(2)-yl, propen-(1)-yl-(2),Butenyl, Buten-(1)-yl, Buten-(2)-yl, Buten-(3)-yl, Buten-(1)-yl-(3), Buten-(2)-yl-(2), Buten-(1)-yl-(2),2-Methyl-propenyl-(1), 2-Methyl-propenyl-(2), Pentenyl oder Hexenyl.

Die vorstehend genannten Alkinyle können substituiert sein, besitzen bevorzugt 2 - 6 Kohlenstoffatome und bedeuten insbesondere Ethinyl, Propinyl, Propin-(1)-yl,Propin-(3)-yl, Butinyl, Butin-(1)-yl, Butin-(2)-yl, Butin-(3)-yl, 1-Methylpropin-(2)-yl, Pentinyl oder Hexinyl.

Die vorstehend genannten Halogene bedeuten Fluor, Chlor, Brom oder lod.

Die vorstehend genannten Cycloalkylreste bedeuten bevorzugt Cyclopropyl, Cyclobutyl, Cyclopentyl, Cyclohexyl, Cycloheptyl, Cyclooctyl, Cyclononyl, Cyclodecyl, Bornanyl, Norbornanyl, Dicyclohexyl, Bicyclo[3,3,0]octyl, Bicyclo [3,2,1]octyl, Bicyclo[2,2,2]octyl oder Bicyclo[3,3,1]nonyl.

Die vorstehend genannten Cycloalkenylreste bedeuten bevorzugt Cyclopropenyl, Cyclobutenyl, Cyclopentenyl, Cyclohexenyl, Cycloheptenyl, Cyclooctenyl, Cyclononenyl, Cyclodecenyl, Bornenyl, Norbomenyl, Bicyclo[3,3,0]octenyl, Bicyclo[3,2,1]octenyl, Bicyclo[2,2,2]octenyl oder Bicyclo[3,3,1]nonenyl.

Die vorstehend genannten Haloalkylreste bedeuten bevorzugt C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl oder Pentafluorethyl.

Die vorstehend genannten Haloalkoxyreste bedeuten bevorzugt C₁-C₄-Halogenalkoxy, besonders C₁-C₂-Halogenalkyloxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 22-Dichlor-2-fluorethyloxy, 2,2,2-Tri-chlorethyloxy oder Pentafluorethyloxy.

Die vorstehend genannten Aryle bedeuten bevorzugt Phenyl, 1 -Naphthyl, 2-Naphtyl, 1-Anthracenyl, 2-Anthracenyl oder 9-Anthracenyl.

Die vorstehend genannten Hetaryle bedeuten bevorzugt Furyl, 2-Furyl, 3-Furyl, Thienyl, 2-Thienyl, 3-Thienyl, Pyrrolyl, 1-Pyrrolyl, 2-Pyrrolyl, 3-Pyrrolyl, Isoxazolyl, 3-Isoxazolyl, 4-Isoxazolyl, 5-Isoxazolyl, Isothiozolyl, 3-Isothiozolyl, 4-Isothiozolyl, 5-Isothiozolyl, Pyrazolyl, 1-Pyrazolyl, 3-Pyrazolyl, 4-Pyrazolyl, 5-Pyrazolyl, Oxazolyl, 2-Oxazolyl, 4-Oxazolyl, 5-Oxazolyl, Thiazolyl, 2-Thiazolyl, 4-Thiazolyl, 5-Thiazolyl, Imidazolyl, 1-Imidiazolyl, 2-Imidazolyl, 4-Imidazolyl, 5-Imidazolyl, 1,2,3-Thiadiazolyl, 1,2,4-Thiadiazolyl, 1,2,5-Thiadiazolyl, 1,3,4-Thiadiazolyl, Tetrazolyl, 1,2,3,4-Thiatriazolyl, 1,2,3,4-Oxatriazolyl, Pyridyl, 2-Pyridyl, 4-Pyridyl, Pyridazinyl, 3-Pyridazinyl, 4-Pyridazinyl, Pyrimidinyl, 2-Pyrimidinyl, 4-Pyrimidinyl, 5-Pyrimidinyl, Pyrazinyl, 2-Pyrazinyl, 3-Pyrazinyl, 1,2,4-Triazinyl, 1,3,5-Triazinyl oder 1,2,4,5-Tetrazinyl.

Dabei können benachbarte Substituenten des Heteroaromaten kondensiert sein zu einem aromatischen oder heteroaromatischen Ring, so daß Hetaryl auch kondensierte Ringsysteme umfaßt wie z. B. Benzofuranyl, Isobenzofuranyl, 1-Benzothienyl, 2-Benzothienyl, Indolyl, Isoindolyl, Benzisoxazolyl, Benzoxazolyl, Benzisothiazolyl, Benzthiazolyl, 2-Benzthiazolyl, 4-Benzthiazolyl, 5-Benzthiazolyl, 6-Benzthiazolyl, 7-Benzthiazolyl, Indazolyl, Benzimidazolyl, Benzofurazanyl, Dibenzofuranyl, Dibenzothienyl, Acridinyl, Phenanthridinyl, Carbazolyl, Chinolinyl, Isochinolinyl, Phthalazinyl, Chinazolinyl, Chinoxalinyl, Cinnolinyl, 1,5-Naphthyridinyl, 1,6-Naphthyridinyl, 1,7-Naphthyridinyl, 1,8-Naphthyridinyl, Pteridinyl, Pyrrolopyridinyl, Pyrrolopyridazinyl, Pyrrolopyrimidinyl, Pyrrolopyrazinyl, Pyrrolotriazinyl, Furopyridinyl, Furopyridazinyl, Furopyrimidinyl, Furopyrazinyl, Furotriazinyl, Thienopyridinyl, Thienopyridazinyl, Thienopyrimidinyl, Thienopyrazinyl, Thienotriazinyl, Imidazopyridazinyl, Imidazopyrimidinyl, Imidazopyrazinyl, Pyrazolopyridinyl, Pyrazolopyridazinyl, Pyrazolopyrimidinyl, Pyrazolopyrazinyl, Isoxazolopyrazinyl, Oxazolopyridinyl, Oxazolopyridazinyl, Oxazolopyrimidinyl, Oxazolopyrazinyl, Thiazolopyridinyl, Thiazolopyridazinyl, Isothiazolopyrazinyl, Triazolopyridinyl, Triazolopyridazinyl, Triazolopyrimidinyl oder Triazolopyrazinyl.

Die vorstehend genannten Heterocyclylreste bedeuten bevorzugt 2-Tetrahydrofuranyl, Oxiranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-lsoxazolidinyl, 3-Isothiazolidinyl, 4-Isothiazolidinyl, 5-Isothiazoldinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,5-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,5-Dihydrofur-2-yl, 2,5-Dihydrofur-2-yl, 2,3-Dihydrofur-3-yl, 2,3-Dihydrothien-2-yl, 2,3-Dihydrothien-3-yl, 2,5-Dihydrothien-2-yl, 2,5-Di-hydrothieny-2-yl, 2,4-Pyrrolin-2-yl, 2,3-Pyrrolin-3-yl, 2,5-Pyrrolin-2-yl, 2,5-Pyrrolin-3-yl, 2,3-Isoxazolin-3-yl, 3,4-lsoxazolin-3-yl, 4,5-Isoxazolin-2-yl, 2,3-Isoxazolin-4-yl, 3,4-Isoxazolin-3-yl, 4,5-Isoxazolin-4-yl, 2,3-Isoxazolin-5-yl, 3,4-lsoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-lsothiazolin-4-yl, 4,5-lsothiazolin-4-yl, 2,3-Isothiazolin-5-yl, 3,4-lsothiazolin-5-yl, 4,5-lsothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydopyrazol-1-yl, 3,4-Dihydropyrazol-2-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 3,4-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 3-Tetrahydropyridazinyl, 4-Tetreahydropyridazinyl, 2-Tetrahydropyrimidinyl, 4-Tetrahydropyrimidinyl, 5-Te- trahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, Oxazol-2-in-2-yl, 2-Tetrahydropyranyl, 1,3-Dioxolan-2-yl, Thiazol-2-in-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl, 1,3-Dithian-2-yl, N-Morpholinyl oder Dihydrochinazolinyl.

Die Erfindung betrifft ferner Carbamate der Formel I 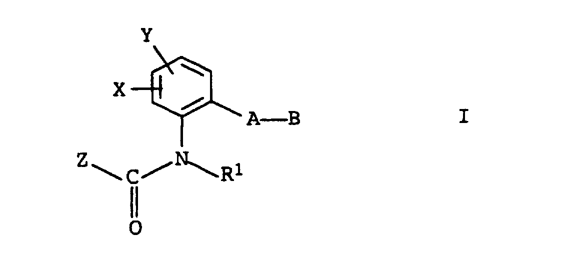 in der die Substituenten die folgenden Bedeutungen haben:
Z bedeutet Methoxy, NH₂, NHCH₃, CH₃,
X und Y bedeuten unabhängig voneinander Wasserstoff, F, Cl, Br, CF₃, CN, NO₂, Alkoxy, Akenyloxy, Alkinyloxy,
Alkyl, Alkenyl oder Alkinyl oder können zusammen zu einem Phenylring kondensiert sein,

- R¹: SR⁵, Alkoxy, Alkenyloxy, Alkinyloxy, Cycloalkoxy, Cycloalkenyloxy oder Alkoxycarbonyloxy;
R⁵ Alkyl, Cyclopropyl, Cyclopropylmethyl oder Cyclobutyl;
wobei B nicht Wasserstoff bedeutet, wenn A für eine direkte Bindung steht und Z Methoxy bedeutet,
A bedeutet -O-, -CR²=CR³⁻, -C≡C-, -CHR²-O-, -CHR²-S-, -CHR²-O-N=C(R⁴)-, -CR²=N-O- oder -O-N=C(R⁴)-B bedeutet
a) subst. Phenyl, wenn A -CR²=CR³, -C≡C-, -CHR²-O-, -CHR²-S-, -CHR²-O-N=C(R⁴)-, -CR²=N-O- oder -O-N=C (R⁴) ist,
b) oder B bedeutet ggf. subst. Cycloalkyl, ggf. subst. Cycloalkenyl, ggf. subst. Heterocyclyl, ggf. subst. Hetaryl, ggf. subst. Naphthyl, ggf. subst. Arylalkyl, ggf. subst. Hetarylalkyl, ggf. subst. Cycloalkylalkyl ggf. subst. Cycloalkenylalkyl oder ggf. subst. Anthracenyl
R² und R³ bedeuten unabhängig voneinander Wasserstoff, Alkyl, Alkenyl, Alkinyl oder Cycloalkyl,
R⁴ bedeutet CN, Alkyl, Alkenyl, Alkinyl oder Cycloalkyl,
R⁵ bedeutet Alkyl, Cyclopropyl, Cyclopropylmethyl oder Cyclobutyl und ihre pflanzenverträglichen Säureadditionsprodukte und Basenadditionsprodukte.

Die Erfindung betrifft ferner Carbamate der Formel II gestrichener Text ersetzt durch Seiten 10, 10a, eingegangen an 06.11.01 X und Y bedeuten unabhängig voneinander Wasserstoff, F, Cl, Br, CF₃, CN, NO₂, Alkoxy, Alkenyloxy, Alkinyloxy, Alkyl, Alkenyl oder Alkinyl oder können zusammen zu einem ggf. substituierten aromatischen oder heteroaromatischen, alicyclischen oder heterocyclischen, partiell oder vollständig hydrierten Ring kondensiert sein,
R^{y} kann ggf. substituiert sein und bedeutet Alkyl, Alkenyl, Alkinyl, Cycloalkyl, Cycloalkenyl oder -CO₂-Alkyl,
A bedeutet -O-, -S-, -CR²=CR³⁻, CHR²-O-, -CHR²-S-, -CHR²-O-N=C(R⁴)-, -CR²=N-O-, -O-N=C(R⁴)-, -C≡C-, -CHR²-CHR³-, -CHR²-O-CO-, -O-CHR²- oder eine Einfachbindung,
B kann ggf. substituiert sein und bedeutet Alkyl, Alkenyl, Alkinyl, Cycloalkyl, Cycloalkenyl, Cycloalkinyl, Aryl, Hetaryl, Heterocyclyl oder Wasserstoff,
R² und R³ bedeuten unabhängig voneinander Wasserstoff, Alkyl, Alkenyl, Alkinyl oder Cycloalkyl und R⁴ bedeutet Wasserstoff, Cyano, Alkyl, Alkenyl, Alkinyl, Cycloalkyl oder Alkoxy, und ihre pflanzenverträglichen Säureadditionsprodukte und Basenadditionsprodukte,

Die Erfindung betrifft ferner Carbamate der Formel VIII 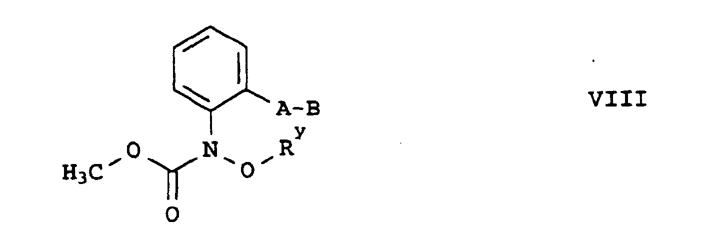 in der A und B die in Anspruch 1 und RY die in Anspruch 11 genannte Bedeutung besitzen.

Die Erfindung betrifft ferner Carbamate der Formel IX 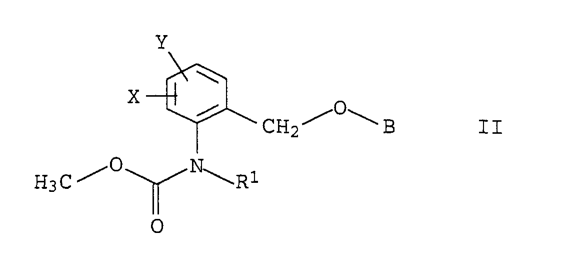 in der R¹ für SR⁵ steht und
X und Y bedeuten unabhängig voneinander Wasserstoff, F, Cl, Br, CF₃, CN, NO₂, Alkoxy, Akenyloxy, Alkinyloxy, Alkyl, Alkenyl oder Alkinyl oder können zusammen zu einem Phenylring kondensiert sein;
A bedeutet -O-, -CR²=CR³-, -C≡C-, -CHR²-O-, -CHR²-S-, -CHR²-O-N=C(R⁴)-, -CR²=N-O- oder -O-N=C(R⁴)-;
B bedeutet
a) subst. Phenyl, wenn A -CR²=CR³, -C≡C-, -CHR²-O-, -CHR²-S-, -CHR²-O-N=C(R⁴)-, -CR²=N-O- oder -O-N=C(R⁴) ist,
b) oder B bedeutet ggf. subst. Cycloalkyl, ggf. subst. Cycloalkenyl, ggf. subst. Heterocyclyl, ggf. subst. Hetaryl, ggf. subst. Naphthyl, ggf. subst. Arylalkyl, ggf. subst. Hetarylalkyl, ggf. subst. Cycloalkylalkyl, ggf. subst. Cycloalkenylalkyl oder ggf. subst. Anthracenyl;
R² und R³ bedeuten unabhängig voneinander Wasserstoff, Alkyl, Alkenyl, Alkinyl oder Cycloalkyl,
R⁴ bedeutet CN, Alkyl, Alkenyl, Alkinyl oder Cycloalkyl;
R⁵ bedeutet Alkyl, Cyclopropyl, Cyclopropylmethyl oder Cyclobutyl.

Die Erfindung betrifft ferner Carbamate der Formel III 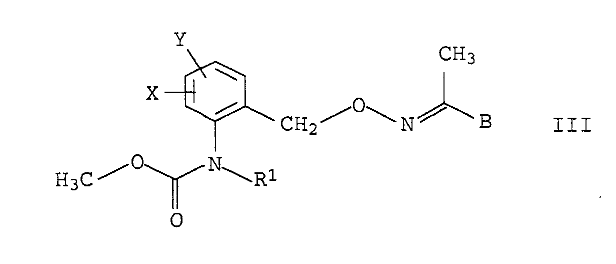 in der X, Y, R¹ und B die vorstehend für Formel II angegebene Bedeutung besitzen.

Die Erfindung betrifft ferner Carbamate der Formel I, in der A den Rest -CH=CH- bedeutet und X, Y, R¹ und B die vorstehend für Formel II angegebene Bedeutung besitzen.

Die Erfindung betrifft ferner Carbamate der Formel VII 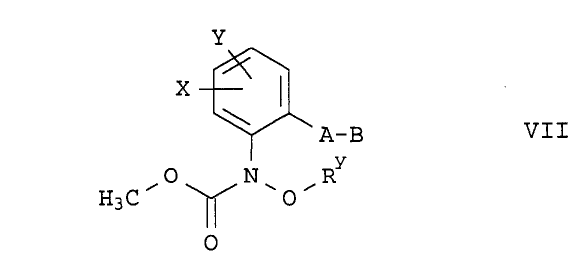 in der die Substituenten die folgenden Bedeutungen haben: 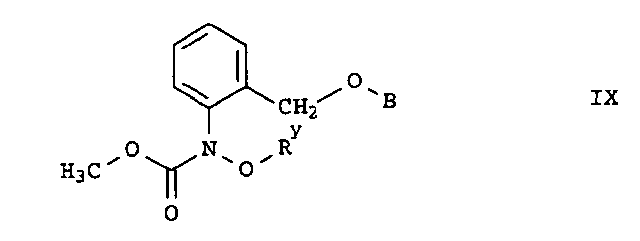 in der B die in Anspruch 1 und R^{y} die in Anspruch 11 genannte Bedeutung besitzen.

Die Erfindung betrifft ferner Carbamate der Formel X 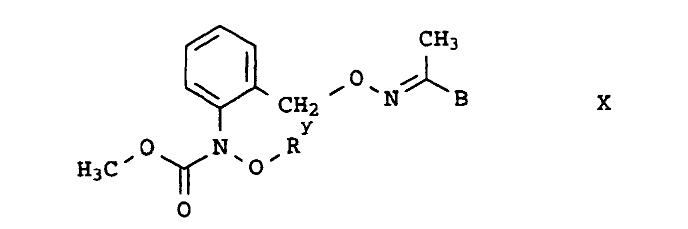 in der B die in Anspruch 1 und R^{y} die in Anspruch 11 genannte Bedeutung besitzen.

Die neuen Verbindungen können beispielsweise nach folgenden Verfahren hergestellt werden:

Die nach Standardverfahren erhältlichen Nitrobenzole 1 werden zu den Anilinen 2 reduziert, z. B. mit Wasserstoff oder Wasserstoffüberträgern wie z. B. Ammoniumformiat in Gegenwart geeigneter Katalysatoren wie Pd, Pt oder Ni, mit komplexen Reduktionsmitteln wie z. B. Collman's Reagenz (Na₂Fe(CO)₄) oder nach anderen literaturbekannten Methoden (J. March, Advanced Organic Chemistry, 3. Auflage 1985, S. 1103ff). Die Aniline 2 werden unter alkalischen Bedingungen mit Chlorameisensäuremethylester zu den Carbamaten 3 umgesetzt. Die Reaktion der Carbamate 3 unter alkalischen Bedingungen mit den entsprechenden Alkylierungsmitteln, Acylierungsmitteln bzw. R⁵-S-S(=O)₂-R⁵ liefert die Derivate 4 (Schema 1).

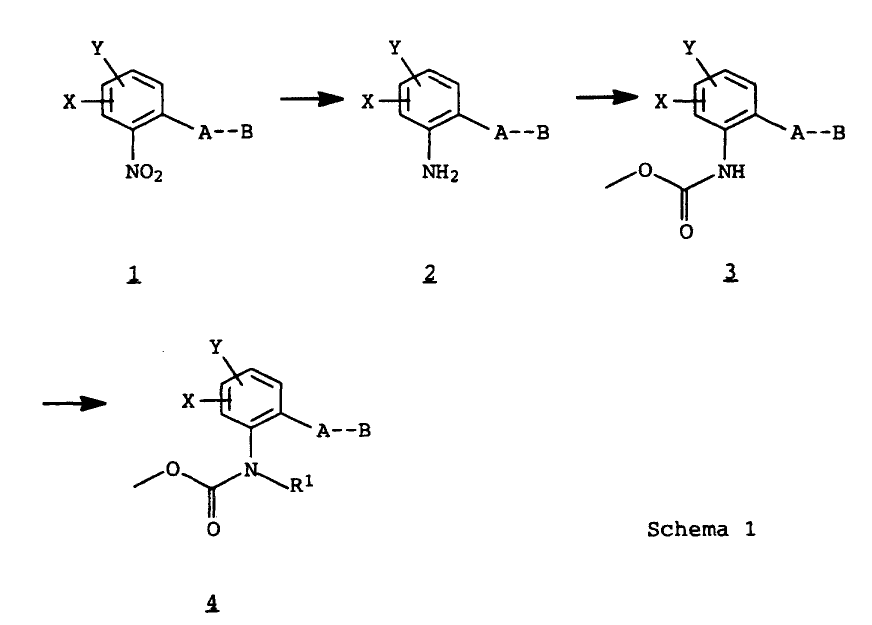

Analog Schema 1 können die Nitrobenzole 5 in die Carbamate 6 überführt werden. Durch saure Spaltung des Methylethers von 6 sind die Halogenderivate 7 (Z = Cl, Br) erhältlich (Schema 2).

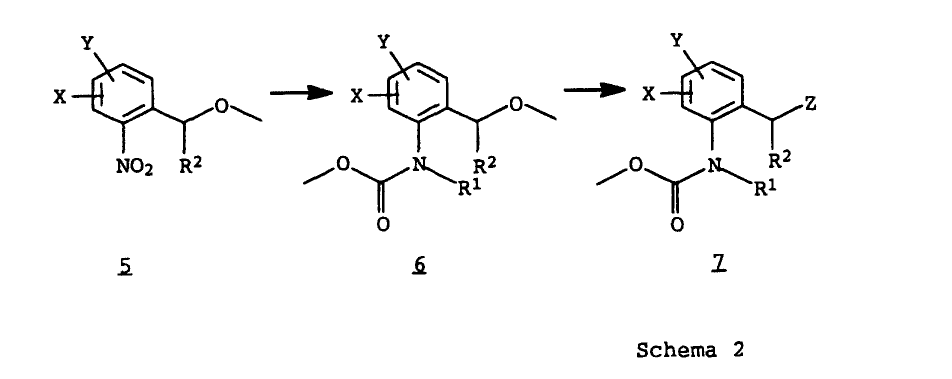

Alternativ sind die Halogenderivate 7 (Z= Cl, Br) durch radikalische Halogenierung aus den Derivaten 9 zugänglich. Die Carbamate 9 wiederum werden aus den entsprechenden Ausgangsmaterialien 8 analog Schema 1 hergestellt (Schema 3).

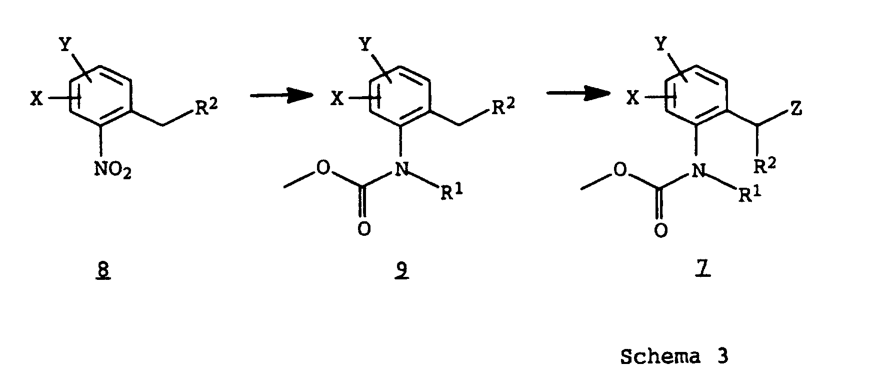

Die Halogenderivate 7 (Z = Cl, Br) können unter alkalischen Bedingungen in die Wirkstoffe 10 überführt werden. Alternativ werden die Verbindungen 7 durch Reaktion mit P(C₆H₅)₃ oder P(O-Alkyl)₃ zu den Phosphorverbindungen 11a und 11b bzw. oxidativ (z. B. mit N-Methylmorpholin-N-oxid) zu den Carbonylverbindungen 12 umgesetzt (Schema 4). 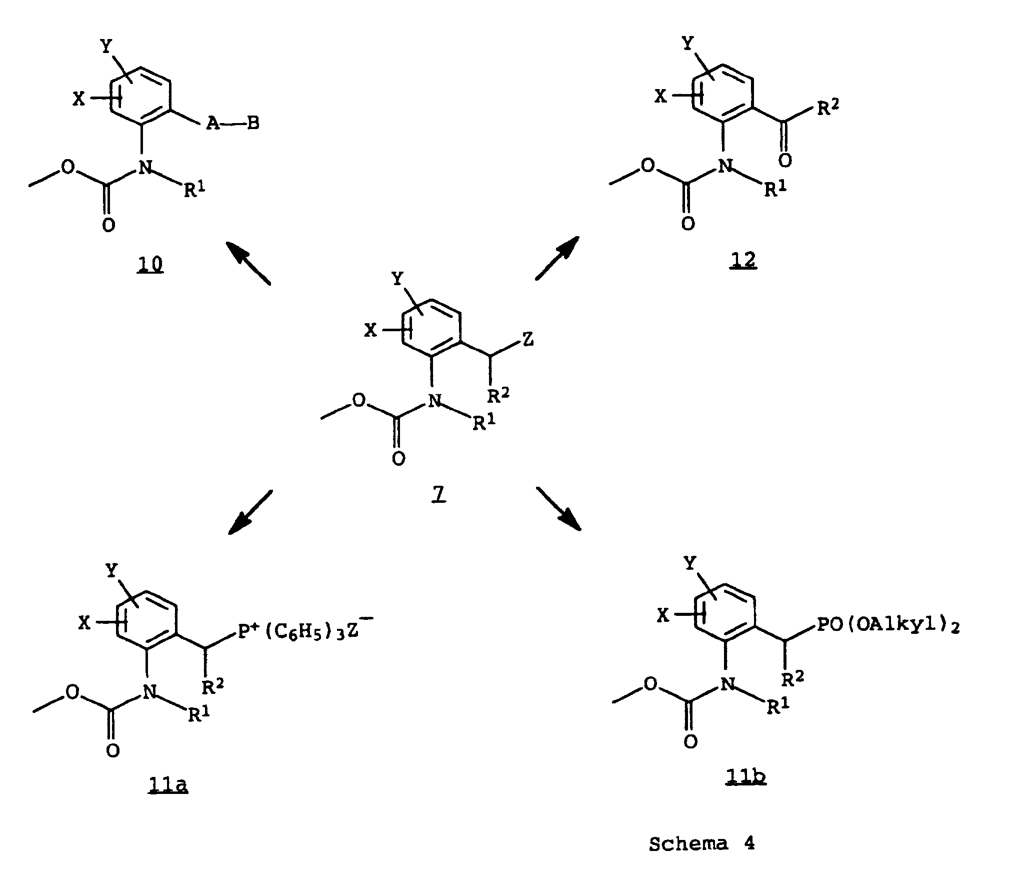
- A =: -CHR²-O-
-CHR²-S-
-CHR²-O-N=C(R⁴)-

Aus den Phosphoniumsalzen 11a oder Phosphonaten 11b bzw. Carbonylverbindungen 12 sind durch Wittig-Reaktion die entsprechenden Stilbene 13 zugänglich (Schema 5).

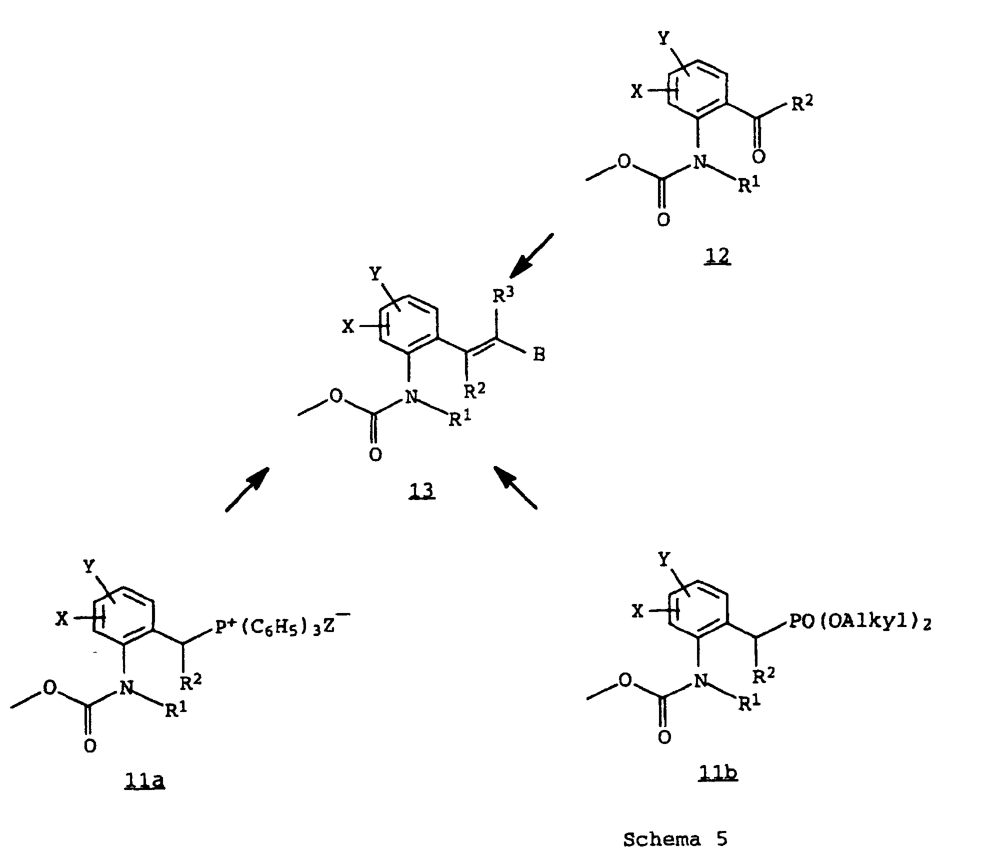

Durch partielle Reduktion der Nitroaromaten 21 (z.B. mit Zink (analog Bamberger et al., Ann. Chem. 316 (1901), 278) oder mit Wasserstoff in Gegenwart geeigneter Katalysatoren wie z.B. Platin (analog EP 85 890)) erhält man die Hydroxylamine 22, die unter alkalischen Bedingungen mit einem Acylierungsmittel (z.B. Propionylchlorid) bzw. Carbamoylierungsmittel (z.B. Methylisocyanat) zur Verbindung 23 und anschließend mit einem Elektrophil, z.B. mit einem Alkylierungsmittel, zu den Wirkstoffen 24 umgesetzt werden können (Schema 11), wobei R^{y} für Alkyl, Alkenyl, Alkinyl, Cycloalkyl, Cycloalkenyl oder Alkoxycarbonyl steht.

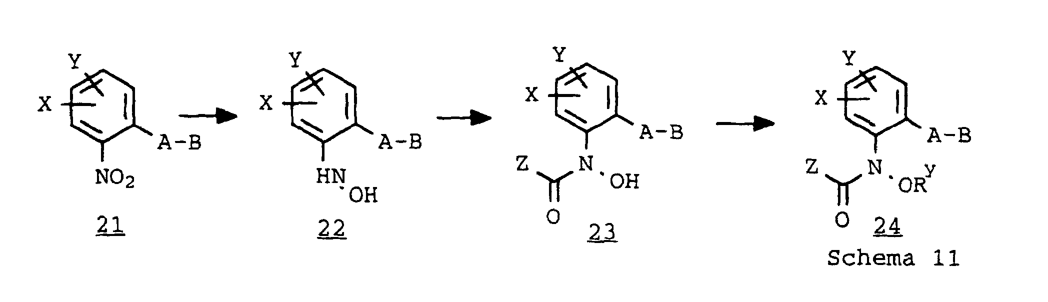

Außerdem kann das Hydroxylamin 25 (analog Bamberger et al., Ann. Chem. 316 (1901), 278; EP 85 890) zur Verbindung 26 acyliert bzw. aminoacyliert (z.B. mit Methylisocyanat) und anschließend zum Hydroxylaminderivat 27 alkyliert bzw. alkoxyacyliert (z.B. mit Chlorameisensäureestern) werden. Die radikalische Halogenierung von 27 z.B. mit N-Bromsuccinimid, Brom, Chlor oder SO₂Cl₂ in Gegenwart eines Radikalstarters, z.B. Azoisobutyrodinitril oder unter Bestrahlung mit UV-Licht liefert dann das Halogenid 28 (Hal = Cl, Br; Schema 12).

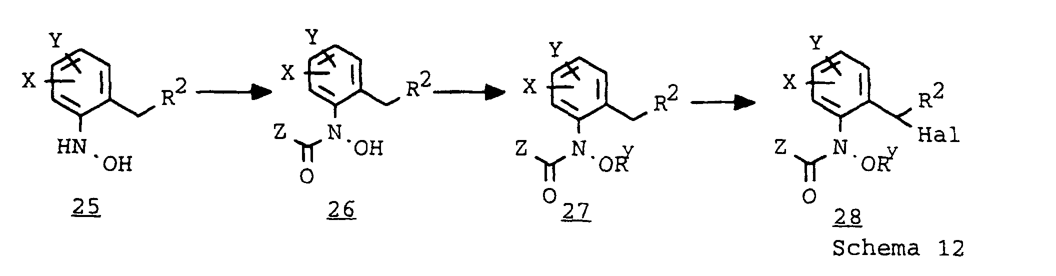

Die Halogenide 28 können dann mit den entsprechenden Nucleophilen in die Verbindungen 29 überführt werden (Schema 13).

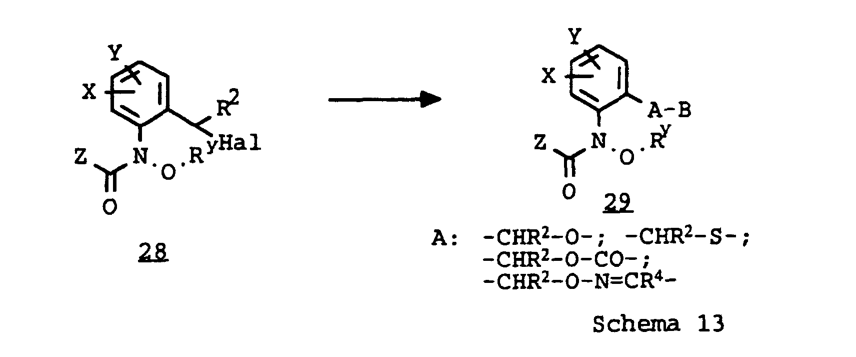

Außerdem können die Halogenide 28 radikalisch zum Dihalogenid 30 umgesetzt und anschließend mit H₂O/MeOH in Gegenwart von AgNO₃ in die Carbonylverbindung 31 überführt bzw. direkt mit N-Methylmorpholin-N-oxid zur Carbonylverbindung 11 umgesetzt werden. Außerdem sind aus den Halogeniden 8 die Phosphonate, Phosphoniumsalze oder Phosphinoxide 32 (P ist der jeweilige Phosphor-organische Rest) erhältlich (Schema 14).

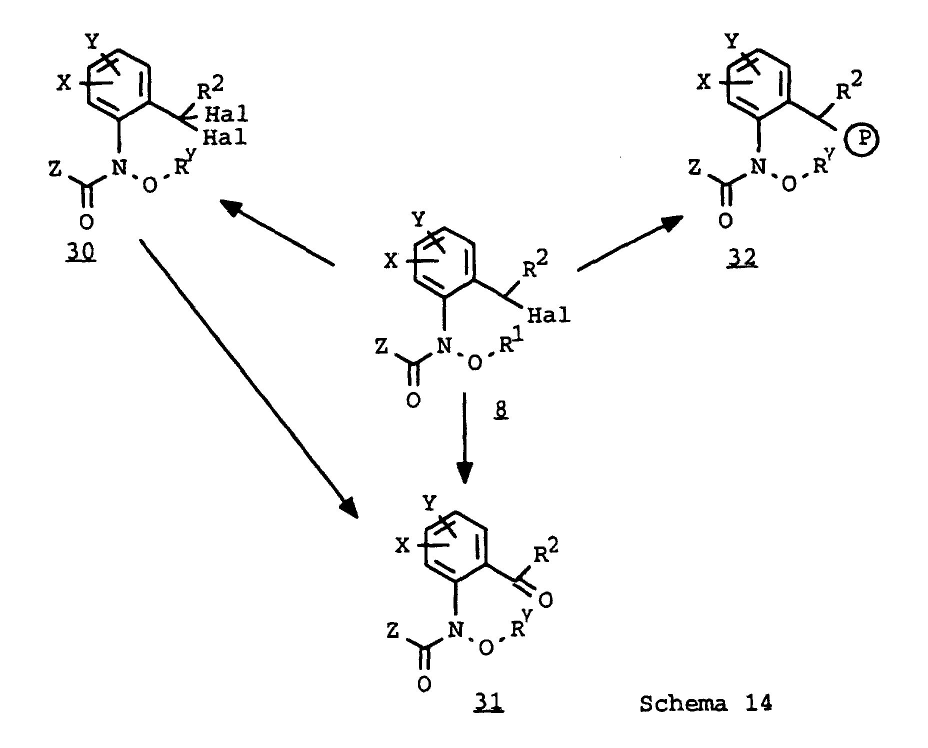

Die Carbonylverbindungen 31 können dann mit den entsprechenden Hydroxylaminen zu den Oximen 33 oder in einer Wittig-Reaktion zu den Olefinen 34 umgesetzt werden. Die Olefine 34 sind außerdem in einer Wittig-Reaktion ausgehend von den Phosphonaten, Phosphoniumsalzen bzw. Phosphinoxiden 32 erhältlich (Schema 15).

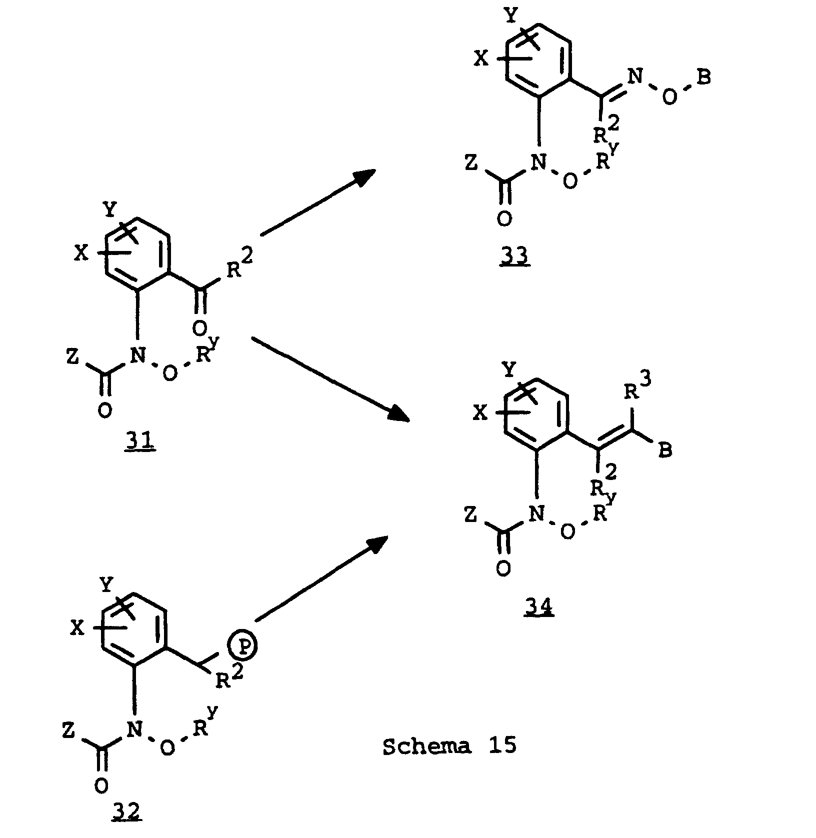

Aus den Olefinen 34 können dann durch Reduktion die gesättigten Verbindungen 35 bzw. im Fall R²=R³=H durch Halogenaddition (Hal = Cl, Br, J) und anschließender zweifacher Halogenwasserstoffabspaltung die Acetylene 36 hergestellt werden (Schema 16).

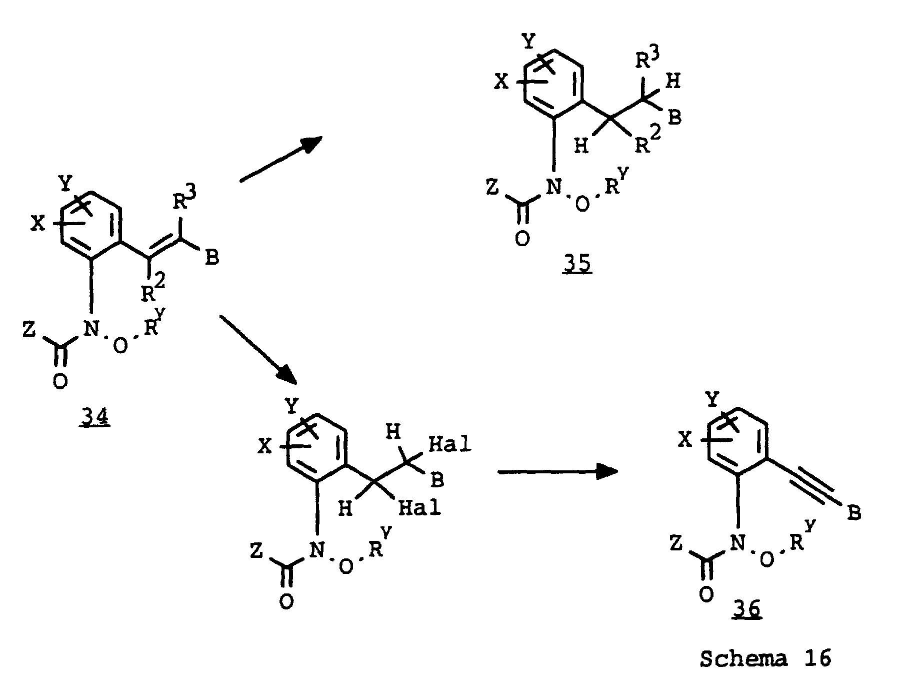

Alternativ können die Harnstoffe 39 durch Acylierung der Hydroxylamine 22 zu den Verbindungen 37, nachfolgende Alkylierung bzw. Acylierung zu den Verbindungen 38 und Substitution der nucleofugen Abgangsgruppe V (V=z.B. OCH₃, OCCl₃, CCl₃, O-Phenyl, O-p-Nitrophenyl) mit NH₃, H₂N-CH₃ oder HN(CH₃)₂ synthetisiert werden (Schema 17).

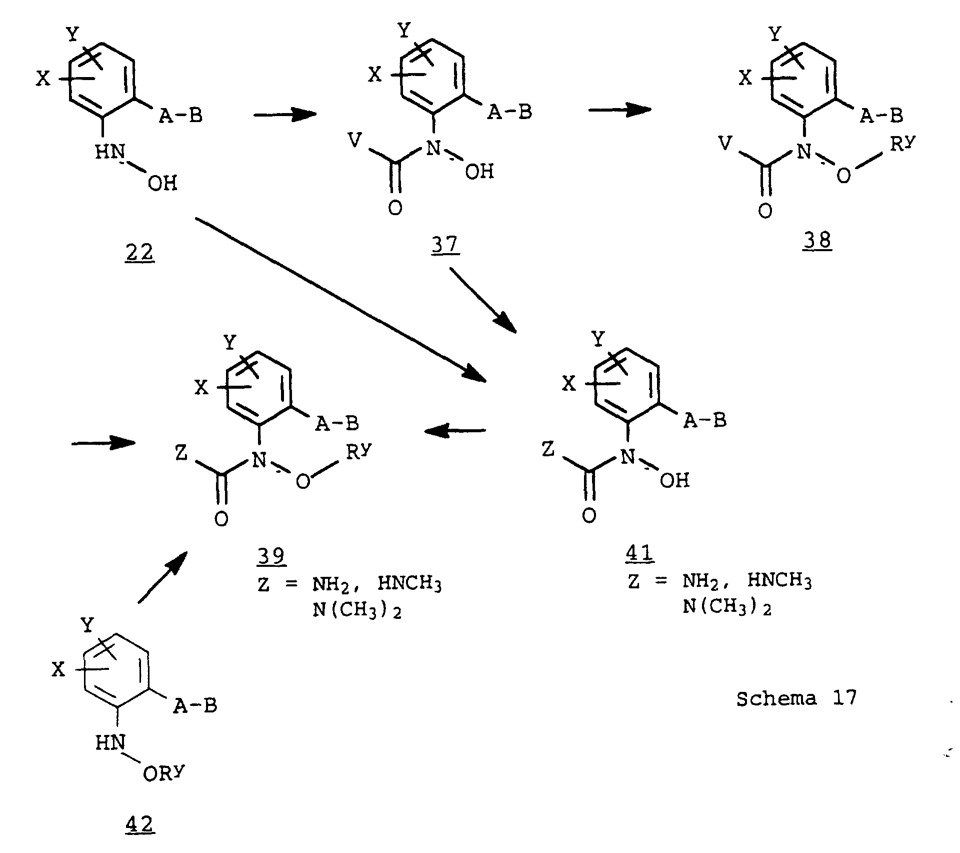

Alternativ hierzu können Harnstoffe der Formel 39 auch durch Alkylierung von Harnstoffen der Formel 41 erhalten werden, die ihrerseits aus 37 durch Umsetzung von 37 mit den entsprechenden Aminen oder aber direkt aus 22 durch Aminocarbonylierung (z.B. mit Dimethylcarbamoylchlorid oder Methylisocyanat) zugänglich sind (s. z.B. Houben-Weyl, Band E16a, S. 208).

Weiterhin können Harnstoffe der Formel 39 auch aus den N-Aryl-O-Alkylhydroxylaminen der Formel 42 in analoger Weise durch Aminocarbonylierung erhalten werden.

Die Verbindungen der Formel 42 ihrerseits sind nach Literaturbekannten Verfahren aus den Hydroxylaminen der Formel 2 erhältlich (s. z.B. Houben-Weyl, Band E16a, S. 271, 282-289).

Außerdem sind die Hydroxylamine 22 aus den Anilinen 43 durch Bildung der Imine 44, Oxidation der Verbindungen 44 mit m-Chlorperbenzoesäure und Umsetzung der Oxaziridine 45 mit Hydroxylamin, erhältlich (Schema 8; analog G. Grundke et al., Synthesis 1987, 1115).

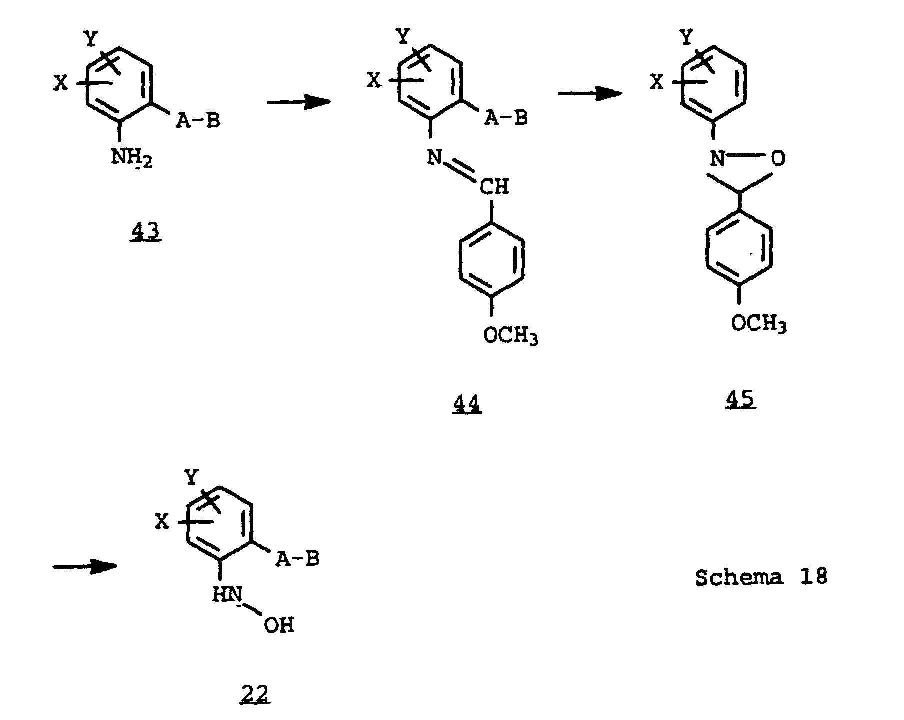

Die folgenden Beispiele erläutern die Herstellung der neuen Verbindungen.

### Beispiele

### Beispiel 1

N-[2-(2'-Methylphenoxymethyl)-phenyl]-N-methylthio-carbaminsäuremethylester (Tabelle 1, Nr. 89)

### a) 2- (2'-Methylphenoxymethyl)-nitrobenzol

75 g (0,347 mol) 2-Nitrobenzylbromid, 37 g (0,342 mol) o-Kresol und 56 g (0,405 mol) Kaliumcarbonat in 500 ml Dimethylformamid werden 5 Stunden bei Raumtemperatur gerührt. Anschließend verdünnt man die Reaktionsmischung mit Wasser und extrahiert die wässrige Phase dreimal mit Ether. Die etherische Phase wird getrocknet und eingeengt. Der kristalline Rückstand wird mit Methanol ausgerührt und abgesaugt. Man erhält 73 g (0,300 mol = 88 %) der Titelverbindung als farblosen Festkörper.
Fp = 83°C
¹H-NMR (CDCl₃; δ (ppm)):
8,15 (d, 1H, J = 8 Hz, Aromat); 7,95 (d, 1H, J = 8 Hz, Aromat); 7,7 (t, 1H, J = 8 Hz, Aromat); 7,45 (t, 1H, J = 8 Hz, Aromat); 7,15 (m, 2H, Aromat); 6,9 (m, 2H, Aromat); 5,45 (s, 2H, O-CH₂); 2,35 (s, 3H, CH₃)

### b) 2-(2'-Methylphenoxymethyl)-anilin

75 g (0,308 mol) 2-(2'-Methylphenoxymethyl)-nitrobenzol (Beispiel 1a) und 10 g 5 %ige Pt/C (Platin adsorbiert an Aktivkohle) in 50 ml Methanol werden unter einer H₂-Atmosphäre zwei Stunden kräftig gerührt. Dann gibt man weitere 2 g 5 %ige Pt/C hinzu und rührt über Nacht. Anschließend wird der Katalysator abgesaugt und durch 10 g frischen Katalysator ersetzt. Man rührt über Nacht, saugt ab und dampft das Filtrat i. Vak. ein. Der Rückstand wird säulenchromatographisch mit Hexan/EssigesterGemischen gereinigt. Man erhält 61 g (0,286 mol = 93 %) der Titelverbindung als farblosen Festkörper. Fp = 56°C
¹H-NMR (CDCl₃; δ (ppm)):
7,2 (m, 4H, Aromat); 6,95 (d, 1H, J = 8 Hz, Aromat); 6,9 (t, 1H, J = 6 Hz, Aromat); 6,7 (m, 2H, Aromat); 5,0 (s, 2H, O-CH₂); 4,05 (s, breit, 2H, NH₂); 2,2 (s, 3H, CH₃)

### c) N- [2-(2'-Methylphenoxymethyl)-phenyl]-carbaminsäuremethylester

10 g (47 mmol) 2-(2'-Methylphenoxymethyl)-anilin in 500 ml Methylenchlorid wird bei 20 - 30 °C tropfenweise mit 6 g (63 mmol) Chlorameisensäuremethylester versetzt. Man rührt 3 Stunden bei Raumtemperatur, wobei ein weißer Festkörper ausfällt, und rührt anschließend die Reaktionsmischung mit 20 ml 10 %iger Natronlauge. Die organische Phase wird über Kieselgel abgesaugt, eingeengt und der zurückbleibende Rückstand wird mit Methanol ausgerührt und abgesaugt. Man erhält 10,5 g (39 mmol = 82 %) der Titelverbindung als farblosen Festkörper.
Fp = 111°C
¹H-NMR (CDCl₃; δ (ppm)):
8,0 (d, breit, 1H, Aromat); 7,7 (s, breit, 1H, Aromat); 7,7 (s, breit, 1H, NH); 6,8 - 7,5 (m, 6H, Aromat); 5,0 (s, 2H, O-CH₂); 3,75 (s, 3H, O-CH₃); 2,25 (s, 3H, CH₃)

### d) N- [2-(2'-Methylphenoxymethyl)-phenyl]-N-methylthio-carbaminsäuremethylester (Tabelle 1, Nr. 89)

4,9 g (17,3 mmol) N-[2-(2'-Methylphenoxymethyl)-phenyl)-carbaminsäuremethylester (Beispiel 2c) in 80 ml Toluol wird portionsweise mit 0,5 g (20,8 mmol) Natriumhydrid versetzt. Nach beeendeter Gasentwicklung wird 2,4 g (19 mmol) Methanthiosulfonsäuremethylester hinzugegeben und über Nacht bei Raumtemperatur gerührt. Anschließend extrahiert man die Reaktionsmischung mit Wasser, trocknet über MgSO₄ und dampft i. Vak. ein. Der Rückstand wird säulenchromatographisch mit Hexan/Essigester-Gemischen an Kieselgel gereinigt. Man erhält 3 g (9,1 mmol = 53 %) der Titelverbindung als gelbes Öl.
¹H-NMR (CDCl₃; δ (ppm)): 7,65 (d, breit, 1H, Aromat); 7,35 (m, 2H, Aromat); 7,15 (m, 3H, Aromat); 6,85 (m, 2H, Aromat); 5,0 (m, 2H, O-CH₂); 3,75 (s, 3H, O-CH₃); 2,55 (s, 3H, S-CH₃); 2,3 (s, 3H, CH₃)

### Beispiel 2

### N-(2-Methylphenyl)-N-methoxy-carbaminsäuremethylester (Tabelle 2, Nr. 1)

### a) N-(2-Methylphenyl)-N-hydroxy-carbaminsäuremethylester

16,4 g N-(2-Methylphenyl)-hydroxylamin (Rohprodukt, erhalten nach Bamberger et al., Ann. Chem. 316 (1901), 278) und 12,9 g (0,163 mol) Pyridin in 100 ml Methylenchlorid werden bei 25-30°C tropfenweise mit 14,0 g (0,148 mol) Chlorkohlensäuremethylester versetzt. Man rührt über Nacht bei Raumtemperatur (20°C) und extrahiert die Reaktionsmischung anschließend mit verdünnter Satzsäure und Wasser. Die organische Phase wird über MgSO₄ getrocknet und eingeengt. Der Rückstand wird säulenchromatographisch mit Hexan/Essigester-Gemischen gereinigt. Man erhält 7 g (39mmol) der Titelverbindung als gelbes Öl.
¹H-NMR (CDCl₃; δ in ppm):
8,6 (s, breit, OH); 7,3 (m, 4H, Phenyl); 3,75 (s, 3H, OCH₃); 2,3 (s, 3H, CH₃)

### b) N-(2-Methylphenyl)-N-methoxy-carbaminsäuremethylester (Tabelle 2, Nr. 1)

6,6 g (36,5 mmol) der Hydroxylverbindung aus Beispiel 2a in 50 ml Dimethylformamid wird bei 20-30°C portionsweise mit 1,1 g (44,1 mmol) Natriumhydrid versetzt. Nach beendeter Gasentwicklung gibt man 5,7 g (40,1 mmol) Methyljodid hinzu und rührt über Nacht bei Raumtemperatur. Anschließend verdünnt man die Reaktionsmischung mit Wasser und extrahiert die wäßrige Phase dreimal mit Methyl-t-butylether. Die vereinigten organischen Phasen werden über MgSO₄ getrocknet und eingeengt. Der Rückstand wird säulenchromatographisch mit Hexan/ Essigester-Gemischen gereinigt. Man erhält 5,2 g (27mmol = 73 %) der Titelverbindung als gelbes Öl.
¹H-NMR (CDCl₃; δ in ppm):
7,25 (m, 4H, Phenyl); 3,8 (s, 3H, OCH₃); 3,75 (s, 3H, OCH₃); 2,3 (s, 3H, CH₃)

### Beispiel 3

### N-(2-Brommethylphenyl)-N-methoxy-carbaminsäuremethylester (Tabelle 2, Nr. 2)

2,5 g (12,8 mmol) des N-Methoxycarbamats aus Beispiel 2b, 2,5 g (14,1 mmol) N-Bromsuccinimid und eine Spatelspitze (1 g) Azoisobutyrodinitril in 20 ml Tetrachlorkohlenstoff werden mit einer 300 W UV-Lampe bestrahlt, wobei sich die Reaktionsmischung auf 30-40°C erwärmt. Nach drei Stunden wird die Reaktionsmischung zweimal mit Wasser extrahiert. Die organische Phase wird über MgSO₄ getrocknet und eingeengt. Der Rückstand wird säulenchromatographisch mit Hexan/ Essigester-Gemischen gereinigt. Man erhält 1,4 g (5,1 mmol = 40 %) der Titelverbindung als gelbes Öl.
¹H-NMR (CDCl₃; δ in ppm):
7,5 (m, 1H, Phenyl); 7,35 (m, 3H, Phenyl); 4,55 (s, 2H, CH₂-Br)1 3,8 (2s, 6H, 2x OCH₃)

### Beispiel 4

### N-[2-(2'-Methylphenoxymethyl)-phenyl]-N-methoxy-carbaminsäure-methylester (Tabelle 2, Nr. 3)

1,2 g (4,4 mmol) des Methylbromids aus Beispiel 3, 0,45 g (4,2mmol) o-Kresol und 0,7 g (4,8 mmol) K₂CO₃ in 30 ml Dimethylformamid werden über Nacht bei Raumtemperatur gerührt. Anschließend verdünnt man die Reaktionsmischung mit Wasser und extrahiert die wäßrige Phase dreimal mit Methyl-t-butylether. Die vereinigten organischen Phasen werden über MgSO₄ getrocknet und eingeengt. Der Rückstand wird säulenchromatographisch mit Hexan/Essigester-Gemischen gereinigt. Man erhält 1,2 g der Titelverbindung, verunreinigt mit o-Kresol. Das Gemisch wird im Kugelrohrofen bei ca. 1 mbar ca. 1 Stunde auf 125°C erhitzt. Als Rückstand erhält man 0,9 g (3 mmol = 68 %) der Titelverbindung als gelbes Öl.
¹H-NMR (CDCl₃; δ in ppm):
7,7 (m, 1H, Phenyl); 7,4 (m, 3H, Phenyl); 7,15 (m, 2H, Phenyl); 6,9 (t, breit, 2H, Phenyl); 5,15 (s, 2H, O-CH₂); 3,8 (s, 3H, OCH₃), 3,75 (s, 3H, OCH₃); 2,3 (s, 3H, CH₃)

### Beispiel 5

### O-Methyl-N-(2-Methylphenyl)-N-propionyl-hydroxylamin (Tabelle 6, Nr.1)

### a) N- (2 -Methylphenyl)-N-propionyl-hydroxylamin

30 g N-(2-Methylphenyl)-hydroxylamin (Rohprodukt, hergestellt laut Bamberger et al., Anm. Chem. 316 (1901), 278; Gehalt ca. 80 % ≙ 0,2 mol) in 500 ml Methylenchlorid wird bei 25 bis 30°C nacheinander tropfenweise mit 12,5 g (0,135 mol) Propionsäurechlorid und 10,7 g (0,135 mol) Pyridin versetzt. Man rührt 30 min bei Raumtemperatur und extrahiert die Reaktionsmischung anschließend mit verdünnter Salzsäure und Wasser. Die organische Phase wird über MgSO₄ getrocknet und eingeengt. Der Rückstand wird säulenchromatographisch mit Cyclohexan/Essigester-Gemischen gereinigt. Man erhält 22,7 g (0,127 mol ≙ 63 %) der Titelverbindung als gelbes Öl.
¹H-NMR (CDCl₃; δ in ppm):
9,4 (s, breit, 1H, OH); 7,2 (m, 4H, Phenyl); 2,4 (s, 3H, CH₃); 2,1 (q, breit, 2H, CH₂); 1,1 (t, 3H, J=7 Hz, CH₃)

### b) O-Methyl-N- (2-methylphenyl) -N-propionyl-hydroxylamin (Tabelle 6, Nr.1)

Zu einer gerührten Mischung von 3,4 g (0,14 mol) NaH in 150 ml Dimethylformamid wird bei 25 bis 300C eine Lösung von 22,7 g (0,127 mol) N-(2-Methylphenyl)-N-propionyl-hydroxylamin (Beispiel 5a) in 50 ml Dimethylformamid hinzugetropft. Nach beendeter Gasentwicklung (15 min) tropft man 18,4 g (0,13 mol) Methyliodid hinzu und rührt über Nacht bei Raumtemperatur. Anschließend verdünnt man die Reaktionsmischung mit Wasser und extrahiert die wäßrige Phase dreimal mit Methyl-t-butylether. Die vereinigten organischen Phasen werden mit Wasser extrahiert, über MgSO₄ getrocknet und eingeengt. Der Rückstand wird säulenchromatographisch mit Cyclohexan/ Essigester-Gemischen gereinigt. Man erhält 18 g (0,081 mol ≙ 64 %) der Titelverbindung als gelbes Öl.
¹H-NMR (CDCl₃; δ in ppm):
7,2 (m, 4H, Phenyl); 3,7 (s, breit, 3H, OCH₃); 2,6 (s, sehr breit, 2H, CH₂); 2,3 (s, 3H, CH₃); 1,2 (s, breit, 3H, CH₃)

### Beispiel 6

### O-Methyl-N-(2-brommethylphenyl)-N-propionyl-hydroxylamin (Tabelle 6, Nr. 2)

Eine Mischung von 10 g (51,8 mmol) des Hydroxylaminderivats aus Beispiel 5b, 11 g (61 mmol) N-Bromsuccinimid und 0,1 g Azoisobutyrodinitril in 100 ml CCl₄ wird zum Rückfluß erhitzt. Dann setzt man einen Tropfen Brom zu und erhitzt weitere 2,5 Stunden zum Rückfluß. Anschließend wird die Reaktionsmischung auf Raumtemperatur abgekühlt, mit Wasser gewaschen, über MgSO₄ getrocknet und eingeengt. Der Rückstand wird säulenchromatographisch mit Cyclohexan/Essigester-Gemischen gereinigt. Man erhält in dieser Reihenfolge 3,4 g (7,9 mmol ≙ 15 %) O-Methyl-N-(2-brommethylphenyl)-d-(α,α-dibrompropionyl)-hydroxylamin, 3,8 g (10,8 mmol ≙ 21 %) O-Methyl-N-(2-brommethylphenyl)-N-(α-brompropionyl)-hydroxylamin, 2,3 g (8,5 g (8,5 mmol ≙ 16 %) der Titelverbindung und 3,5 g Ausgangsmaterial, jeweils als braune Öle.

### a) O-Methyl-N-(2-brommethylphenyl)-N-(α,α-dibrompropio-nyl)-hydroxylamin

7,55 (m, 1H, Phenyl); 7,4 (m, 3H, Phenyl); 4,5 (s, 2H, CH₂, Br); 3,8 (s, 3H, OCH₃); 2,75 (s, 3H, CH₃)

### b) O-Methyl-N-(2-brommethylphenyl)-N- (α-brompropio- nyl) -hydroxylamin

7,5 (s, breit, 1H, Phenyl); 7,4 (s, breit, 3H, Phenyl); 5,15 (s, breit, 1H, CH-Br); 4,5 (dd, breit, 2H, CH₂-Br); 3,8 (s, 3H, OCH₃); 1,85 (s, breit, 3H, CH₃)

### c) O-Methyl-N- (2-brommethylphenyl) -N-propionyl-hydroxylamin

7,5 (m, 1H, Phenyl); 7,35 (m, 3H, Phenyl); 4,5 (s, breit, 2H, CH₂-Br); 3,75 (s, 3H, OCH₃); 2,55 (s, sehr breit, CH₂); 1,2 (t, 3H, J=7 Hz, CH₃)

### Beispiel 7

### O-Methyl-N-(2-(2'-methylphenyloxymethyl)-phenyl)-N-propionyl-hydroxylamin (Tabelle 6, Nr. 3)

Eine Lösung von 0,4 g (3,7 mmol) o-Kresol in 5 ml Dimethylformamid wird mit 0,12 g (5 mmol) Natriumhydrid versetzt. Wenn die Gasentwicklung beendet ist gibt man 1 g (3,6 mmol) des Benzylbromids aus Beispiel 6c hinzu und rührt 2 Stunden bei Raumtemperatur. Dann wird die Reaktionsmischung mit Wasser verdünnt und dreimal mit Methyl-t-butylether extrahiert. Die vereinigten organischen Phasen werden mit Wasser gewaschen, über MgSO₄ getrocknet und eingeengt. Der Rückstand wird mit Cyclohexan/Essigester-Gemischen einmal über Al₂O₃ und einmal über Kieselgel chromatographiert. Man erhält 0,4 g (1,33 mmol ≙ 37 %) der Titelverbindung als gelbes Öl.
¹H-NMR (CDCl₃; δ in ppm):
7,7 (d, breit, 1H, Phenyl); 7,35 (m, 3H, Phenyl); 7,1 (m, 2H, Phenyl); 6,85 (t, breit, 2H, Phenyl); 5,05 (s, 2H, OCH₃); 3,7 (s, 3H, OCH₃); 2,55 (s, sehr breit, 2H, CH₂); 2,3 (s, 3H, CH₃); 1,2 (t, breit, 3H, CH₃)

### Beispiel 8

### N-Methyl-N'-methoxy-N'-2-methylphenylharnstoff (Tabelle 6, Nr. 5)

### a) N-Hydroxy-N-(2-methylphenyl)-carbaminsäurephenylester

Eine Mischung von 2,5 g (20 mmol) N-(2-Methylphenyl)- hydroxylamin (Rohprodukt, erhalten nach Bamberger et al., Anm. Chem. 316 (1901), 278), 3,5 g (25 mmol) K₂CO₃ und 3,5 g (22 mmol) Phenylchlorformiat in 20 ml CH₂Cl₂ wird 2 Stunden bei Raumtemperatur gerührt. Anschließend wird die Reaktionsmischung mit Wasser extrahiert, über MgSO₄ getrocknet und eingeengt. Der Rückstand wird säulenchromatographisch mit Cyclohexan/Essigester-Gemischen gereinigt. Man erhält 2,0 g (8,2 mmol = 42 %) der Titelverbindung als farblosen Festkörper (Fp = 98°C).
¹H-NMR (CDCl₃; δ in ppm):
7-7,6 (m, 10H, Phenyl, OH); 2,35 (s, 3H, CH₃)

### b) N-Methoxy-N-(2-methylphenyl)-carbaminsäurephenylester

Eine Mischung von 2,0 g (8,2 mmol) des Carbaminsäurephenylesters aus Beispiel 8a, 2 g (15 mmol) K₂CO₃ und 1,3 g (10 mmol) Dimethylsulfat in 20 ml Aceton wird 3 Stunden bei Raumtemperatur gerührt. Dann wird die Reaktionsmischung filtriert, eingeengt und der Rückstand wird säulenchromatographisch mit Cyclohexan/Essigester-Gemischen gereingt. Man erhält 1,5 g (5,8 mmol ≙ 71 %) der Titelverbindung als farbloses Öl, das langsam durchkristallisiert (Fp = 60°C).
¹H-NMR (CDCl₃; δ in ppm):
7,1-7,5 (m, 9H, Phenyl); 3,8 (s, 3H, OCH₃); 2,4 (s, 3H, CH₃)

### c) N-Methyl-N'-methoxy-N'-2-methylphenyl-harnstoff (Tabelle 6, Nr. 5)

1,5 g (5,8 mmol) des Carbaminsäurephenylesters aus Beispiel 8b in 20 ml 40 %iger wäßriger Methylaminlösung wird 1 Stunde bei 50°C gerührt. Anschließend wird die Reaktionsmischung abgekühlt und mit CH₂Cl₂ extrahiert. Die vereinigten organischen Phasen werden über MgCO₄ getrocknet und eingeengt. Der Rückstand wird säulenchromatographisch mit Cyclohexan/Essigester-Gemischen gereinigt. Man erhält 0,6 g (3,1 mmol ≙ 53 %) der Titelverbindung als farblosen Festkörper (Fp = 99°C).
¹H-NMR (CDCl₃; δ in ppm):
7,2 (m, 4H, Phenyl); 5,9 (s, breit, 1H, NH); 3,6 (s, 3H, OCH₃); 2,9 (d, 3H, J=ca. 2 Hz, N-CH₃); 2,3 (s, 3H, CH₃)

### Beispiel 9

### N-(2,6-Dimethylphenyl)-N-methoxycarbonyl-O-methyl-hydroxylamin (Tabelle 8, Nr. 1)

### a) N- (2,6-Dimethylphenyl)-N-methoxycarbonyl-hydroxylamin

11,3 g (80 mmol) N-2,6-Dimethylphenyl-hydroxylamin (hergestellt analog Bamberger et al., Ann. Chem. 316 (1901), 278) und 12,5 g (90 mmol) K₂CO₃ in 30 ml Methylenchlorid werden bei 0 - 5°C tropfenweise mit 7,0 g (70 mmol) Chlorameisensäuremethylester versetzt. Man rührt 30 min bei 0-5°C, filtriert den unlöslichen Festkörper ab und dampft das Filtrat i. Vak. ein. Der Rückstand wird säulenchromatographisch mit Cyclohexan/Essigester-Gemischen gereinigt. Man erhält 1,4 g (7,2 mmol = 9 %) der Titelverbindung als dunkles Öl.
¹H-NMR (CDCl₃; δ in ppm):
8,85 (s, breit, 1H, OH); 7,1 (m, 3H, Phenyl); 3,75 (s, 3H, OCH₃); 2,3 (s, 6H, 2xCH₃)

### b) N-(2,6-Dimethylphenyl)-N-methoxycarbonyl-O-methylhydroxylamin

Eine Mischung von 1,4 g (7,2 mmol) N-(2,6-Dimethylphenyl)-N-methoxycarbonyl-hydroxylamin (Beispiel 9a), 1,3 g (9 mmol) K₂CO₃ und 10 g (8 mmol) Dimethylsulfat in 10 ml Aceton wird über Nacht bei Raumteperatur gerührt. Danach wird die Reaktionsmischung mit CH₂Cl₂ verdünnt und mit verdünnter NH₃-Lösung gerührt. Anschließend trennt man die Phasen und extrahiert die organische Phase noch zweimal mit Wasser. Die organische Phase wird über NgSO₄ getrocknet und eingeengt und der Rückstand wird säulenchromatographisch mit Cyclohexan/Essigester-Gemischen gereinigt. Man erhält 1,2 g (6 mmol=83 %) der Titelverbindung als farblosen Festkörper (Fp = 81°C).
¹H-NMR (CDCl₃; δ in ppm):
7,1 (m, 3H, Phenyl); 3,75 (s, breit, 6H, 2xOCH₃); 2,3 (s, 6H, 2 X CH₃)

### Beispiel 10

### N-Methyl-N'-methoxy-N'-(2-2',5'-dimethylphenoxymethyl)phenyl)-hamstoff

### a) N-Methoxy-N- (2-brommethylphenyl)-carbaminsäure-phenylester

Eine Mischung von 125 g (0,486 mol) N-Methoxy-N-(2-methylphenyl)-carbaminsäurephenylester (Beispiel 8b), 88 g (0,494 mol) N-Bromsuccinimid und 1 g Azoisobutyrodinitril (AIBN) in 800 ml CCl₄ wird ca. 12 Stunden zum Rückfluß erhitzt. Dann gibt man zusätzlich 10 g N-Bromsuccinimid hinzu und erhitzt anschließend ca. 4 Stunden zum Rückfluß. Dann extrahiert man die Reaktionsmischung mit Wasser und Natriumthiosulfat-Lösung, trocknet die organische Phase über MgSO₄ und engt im Vakuum ein. Der Rückstand kristallisiert, wird mit Hexan/Methyl-t-butylether ausgerührt und trockengesaugt. Man erhält 107 g (63 %) der Titelverbindung als farblosen Festkörper.
¹H-NMR (CDCl₃; δ in ppm):
7,1-7,6 (m, 9H, Phenyl); 4,65 (s, 2H, CH₂Br); 3,9 (s, 3H, OCH₃)

### b) N-Methoxy-N- (2-(2',5'-dimethylphenoxymethyl)-phenyl)carbaminsäurephenylester

Eine Mischung von 7 g (20 mmol) des Bromids aus Beispiel 10a und 3,3 g (22 mmol) Natriumjodid in 50 ml Aceton wird 30 Minuten zum Rückfluß erhitzt. Anschließend filtriert man den ausgefallenen Festkörper ab und engt die organische Phase im Vakuum ein. Als Rohprodukt erhält man das Beispiel 10a entsprechende Jodid, das ohne weitere Reinigung in die nächste Reaktion eingesetzt wird.

Man löst das oben erhaltene Rohprodukt in 100 ml

Dimethylformamid, gibt 3 g (21,6 mmol) K₂CO₃ und 7,3 g (60 mmol) 2,5-Dimethylphenol hinzu und rührt über Nacht bei Raumtemperatur. Anschließend verdünnt man die Reaktionsmischung mit Wasser und extrahiert die wäßrige Phase dreimal mit Methyl-t-butylether. Die vereinigten organischen Phasen werden mit Wasser extrahiert, über MgSO₄ getrocknet und eingeengt. Der Rückstand wird mit Methylenchlorid/Cyclohexan 1:2 über Al₂O₃ säulenchromatographisch gereinigt. Man erhält 7,3 g (94 %) der Titelverbindung als gelbes Öl.
¹H-NMR (CDCl₃; δ in PPM):
7,7 (d, breit, 1H, Phenyl); 7-7,6 (m, 9H, Phenyl); 6,7 (m, 2H, Phenyl); 5,2 (s, 2H, OCH₂); 3,85 (s, 3H, OCH₃); 2,3 (s, 6H, 2xCH₃).

### c) N-Methyl-N'-methoxy-N'-(2- (2',5'-dimethylphenoxymethyl)phenyl)-harnstoff (Tabelle 1, Nr. V.71)

Eine Mischung von 3,4 g (8,8 mmol) des Carbaminsäurephenylesters aus Beispiel 10b und 20 ml 40 %iger wäßriger Methylamin-Lösung wird 2 Stunden bei 50°C gerührt. Dann läßt man abkühlen und extrahiert die Reaktionsmischung mit Methylenchlorid. Die organische Phase wird eingeengt und der erhaltene Rückstand wird säulenchromatographisch mit Cyclohexan-Essigester-Gemischen gereinigt. Man erhält 1 g (36 %) der Titelverbindung als farblosen Festkörper (Fp = 101°C).
¹H-NMR (CDCl₃; δ in PPM):
7,75 (m, 1H, Phenyl); 6,6-7,4 (m, 6H, Phenyl); 6,0 (s, breit, NH); 5,15 (s, 2H, OCH₂); 3,65 (s, 3H, OCH₃); 2,9 (d, 3H, N-CH₃); 2,3 (s, 3H, CH₃); 2,25 (s, 3H, (H₃).

Entsprechend lassen sich die in den folgenden Tabellen aufgeführten Verbindungen herstellen.

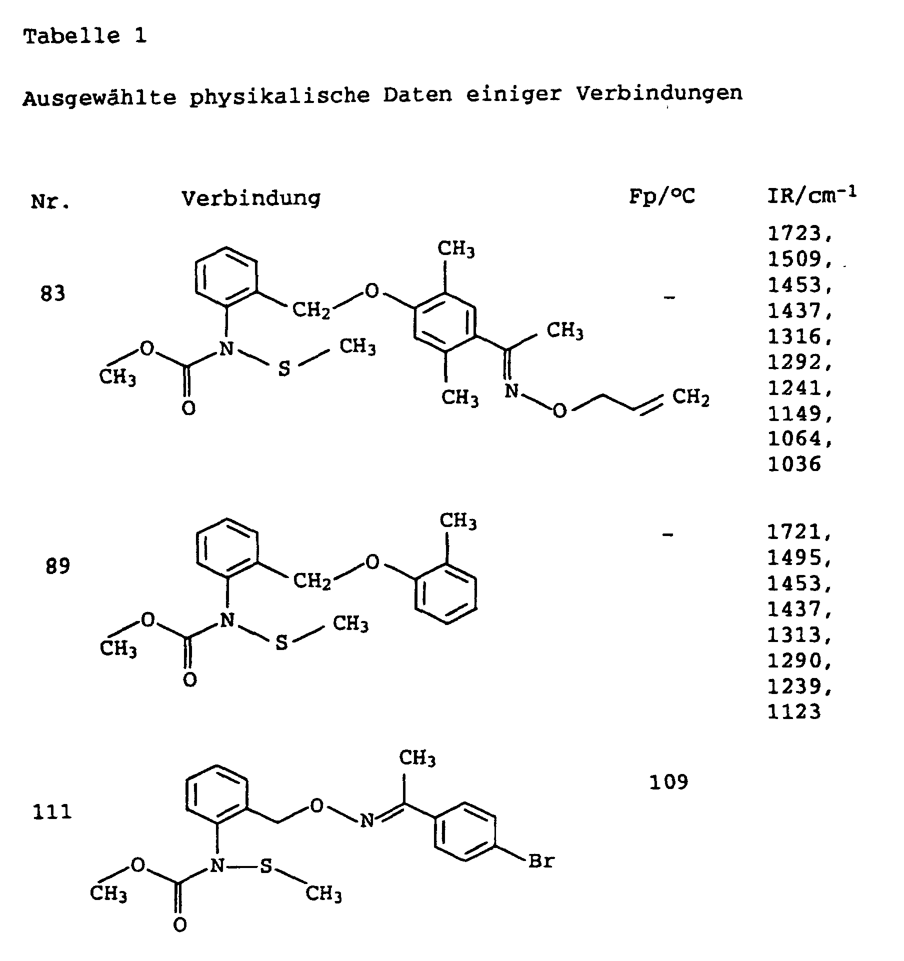

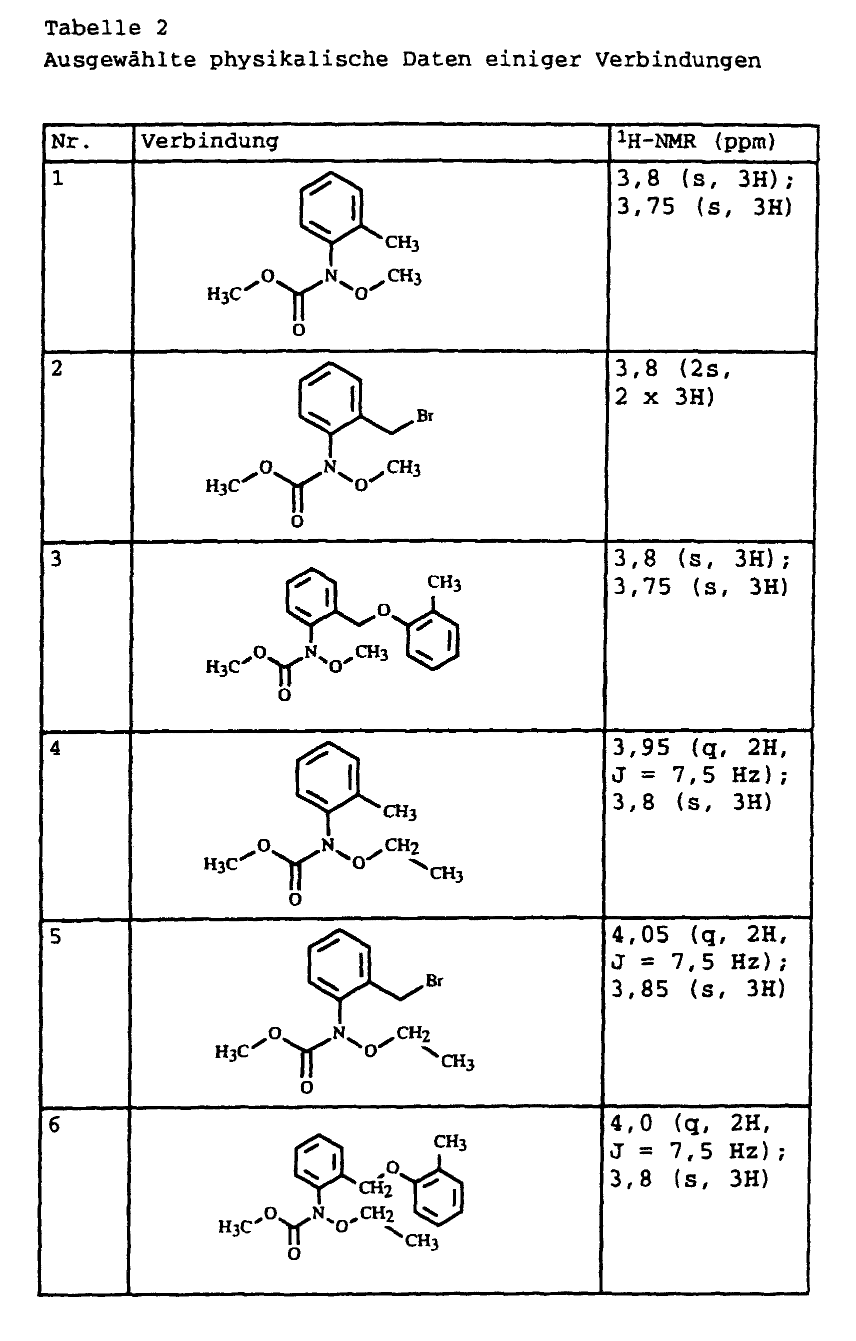

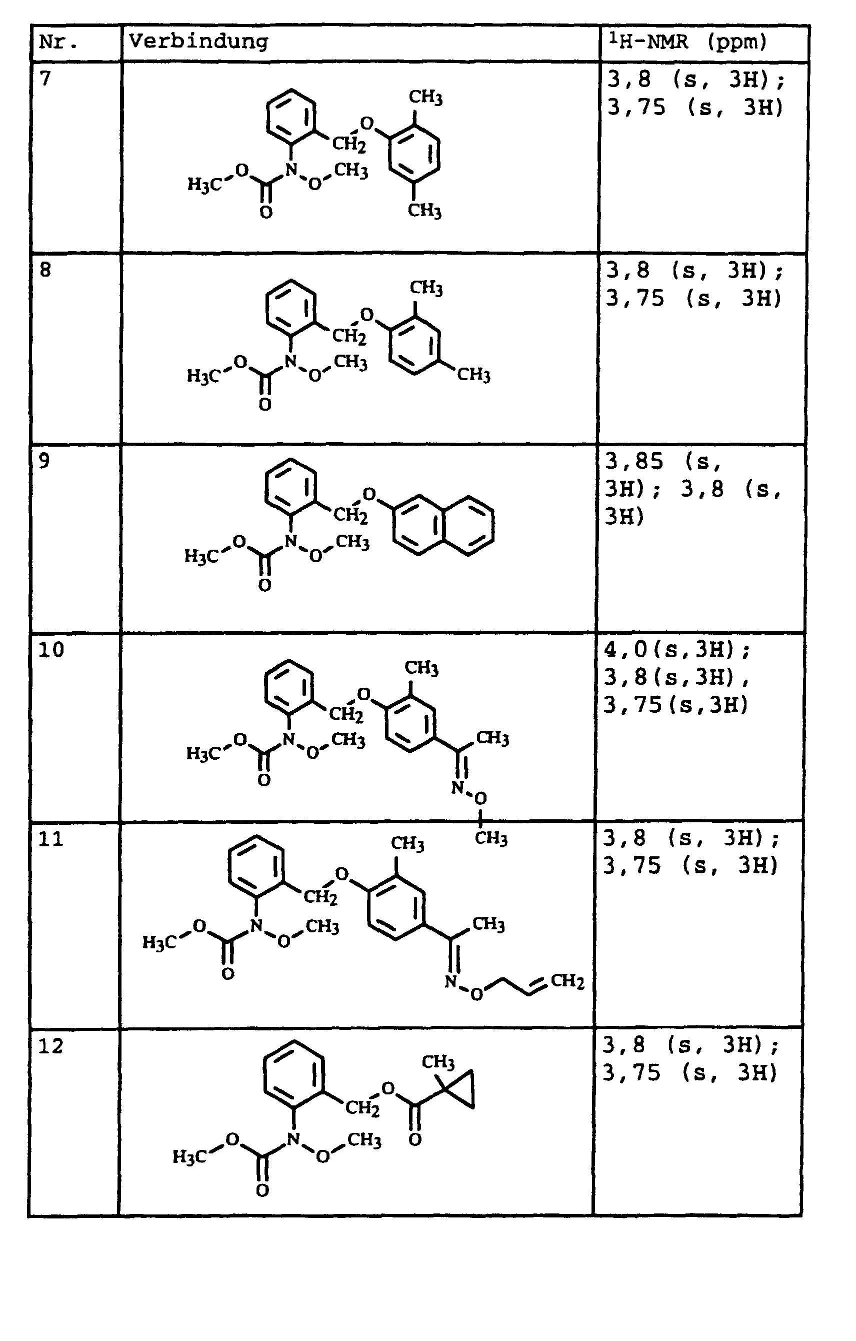

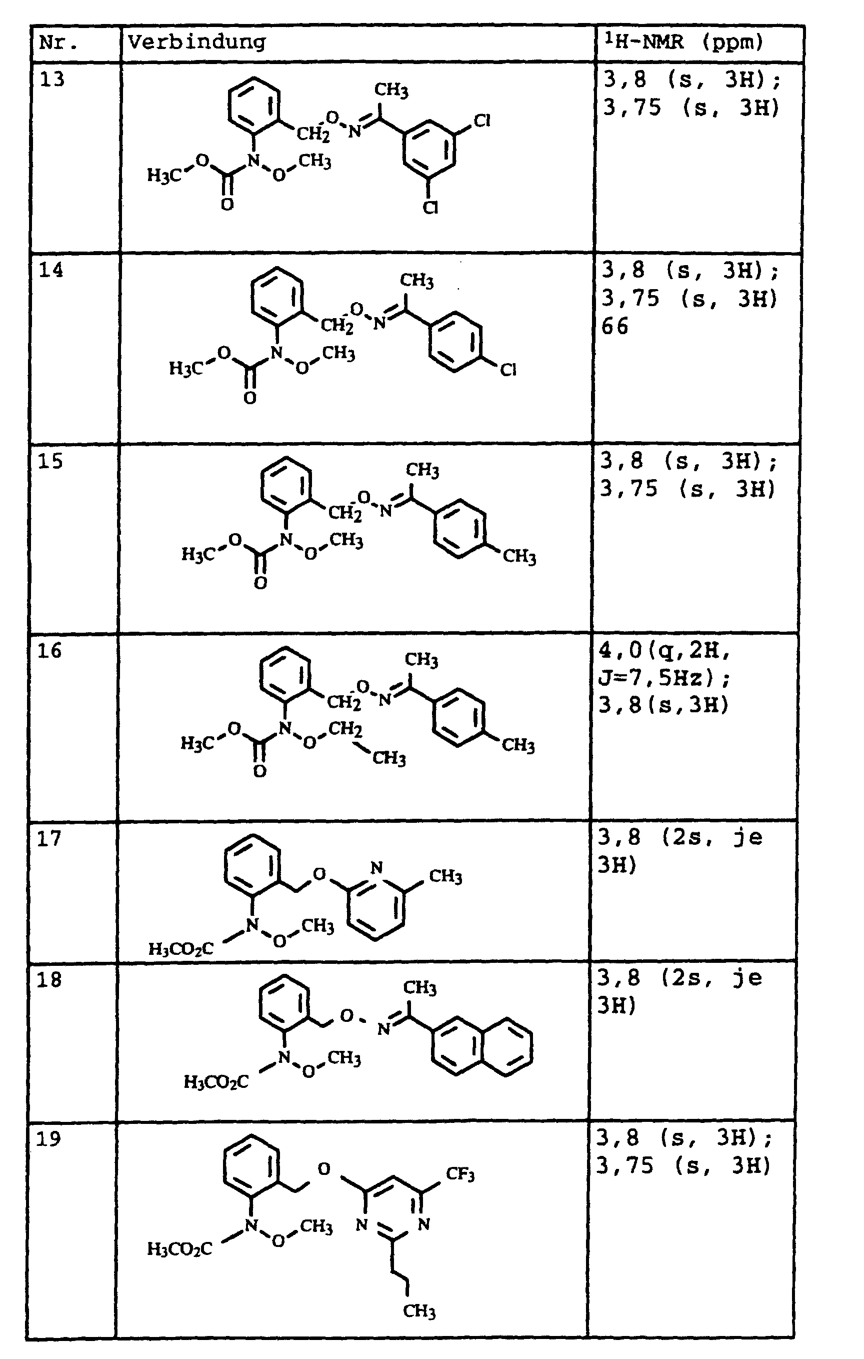

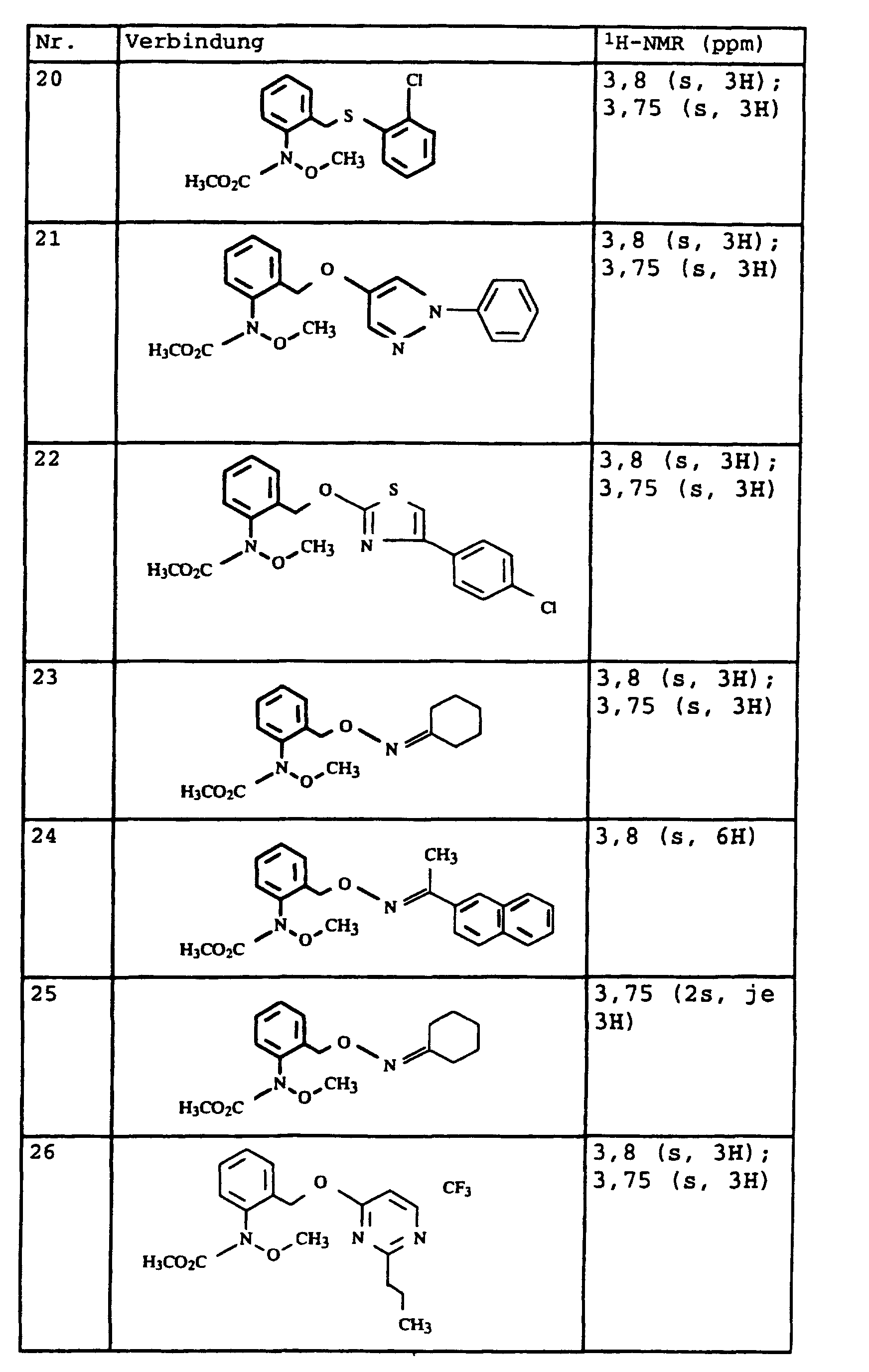

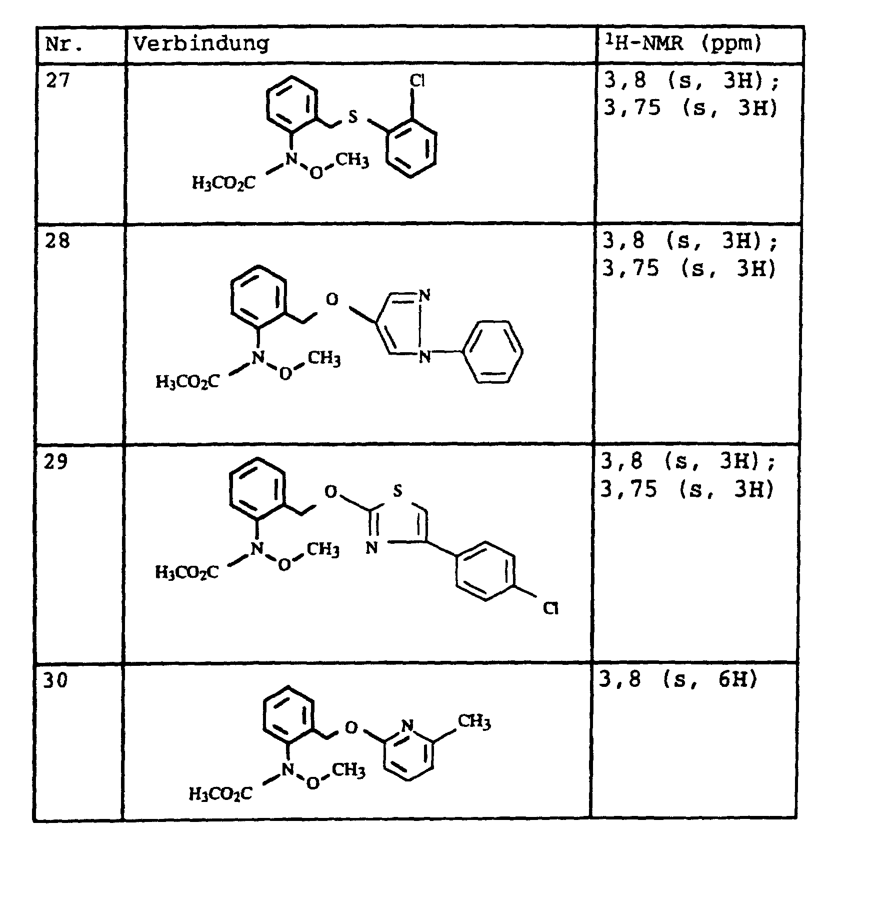

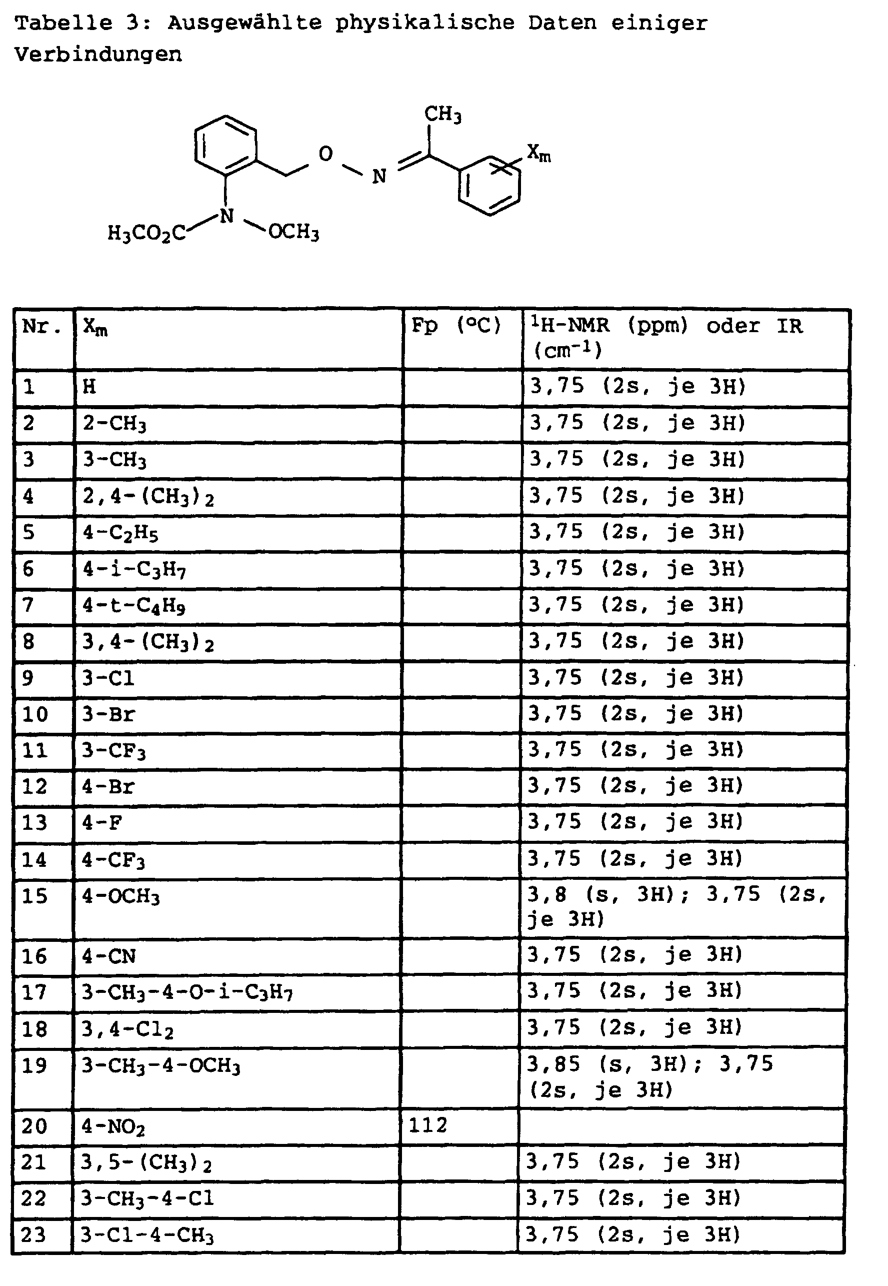

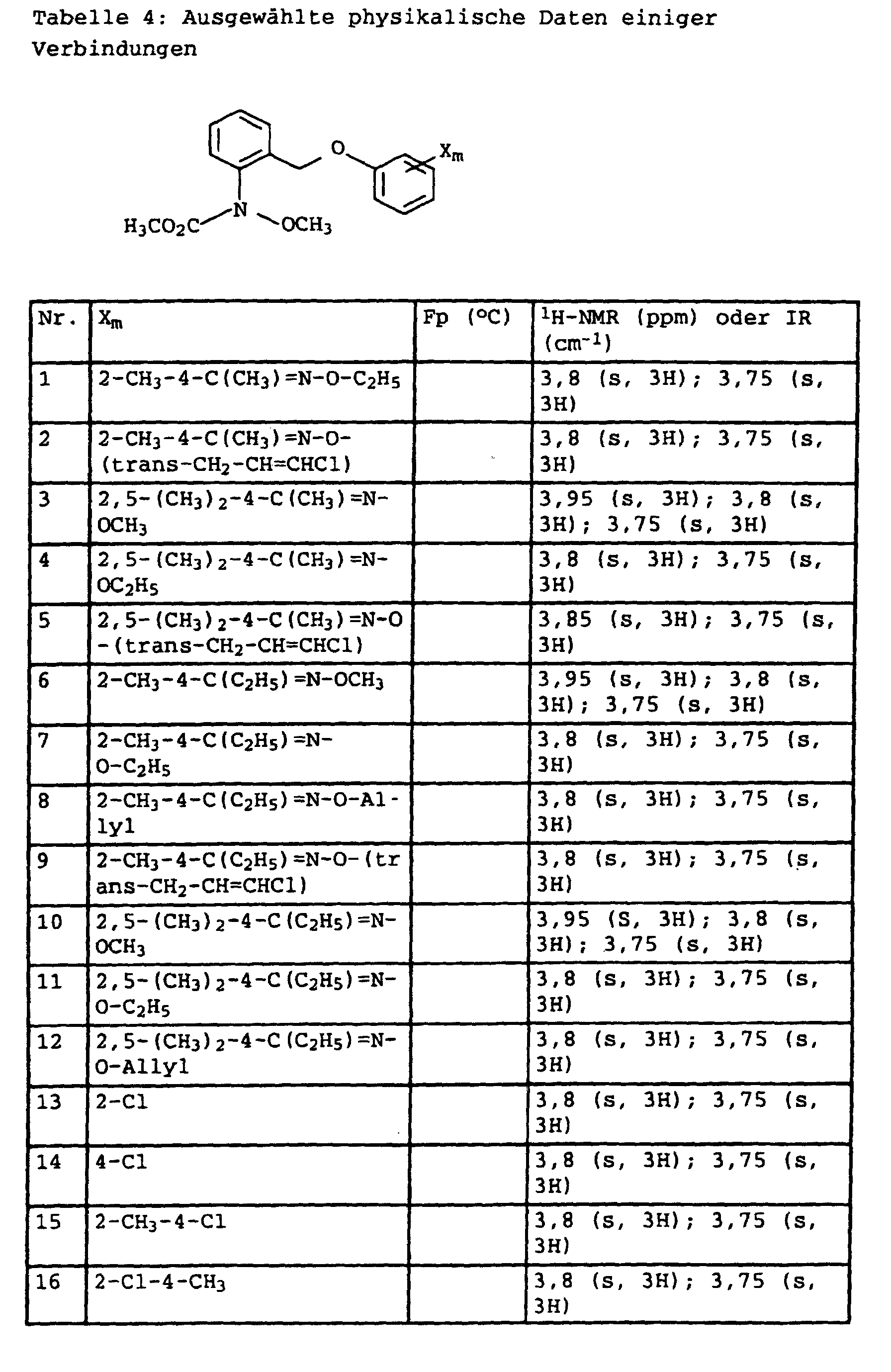

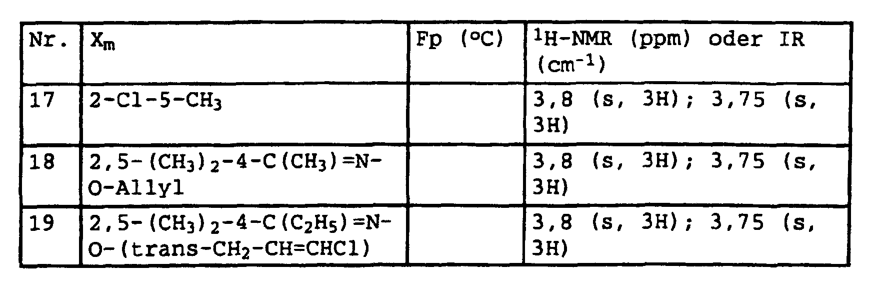

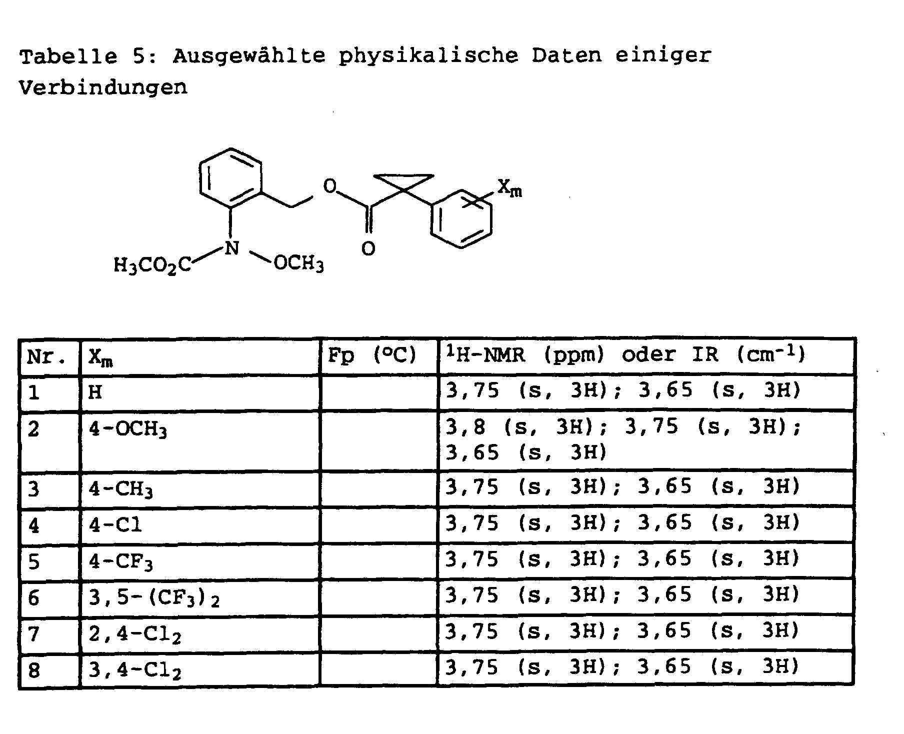

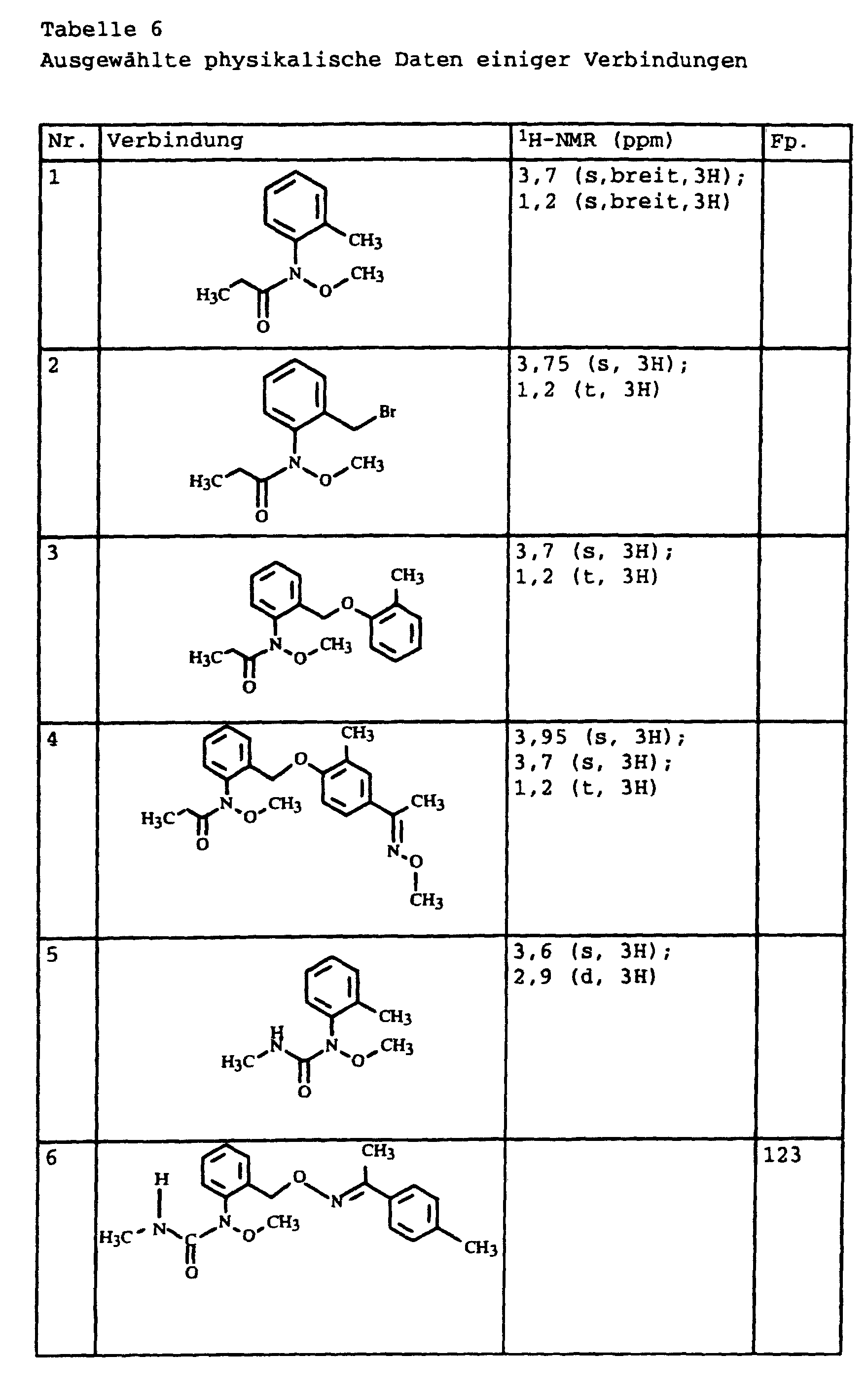

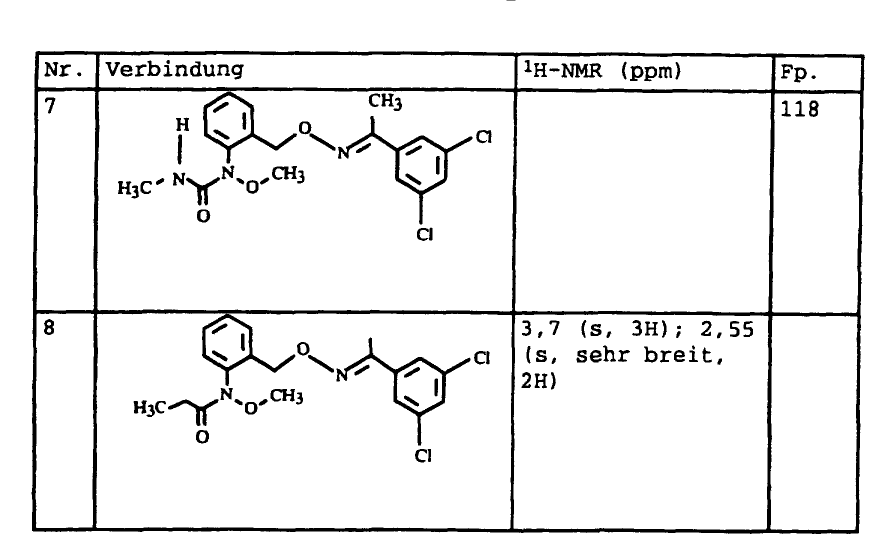

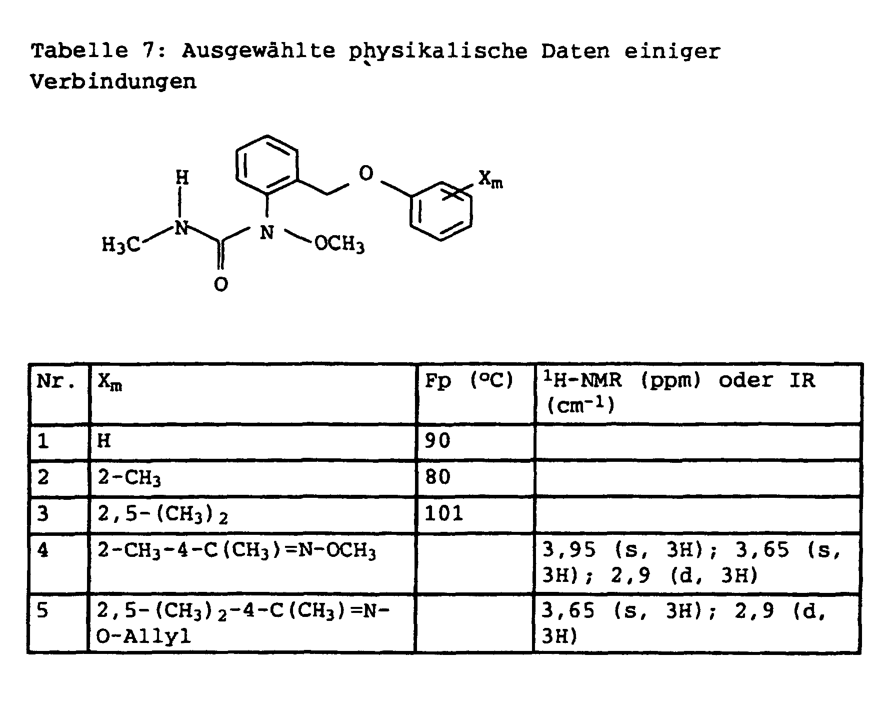

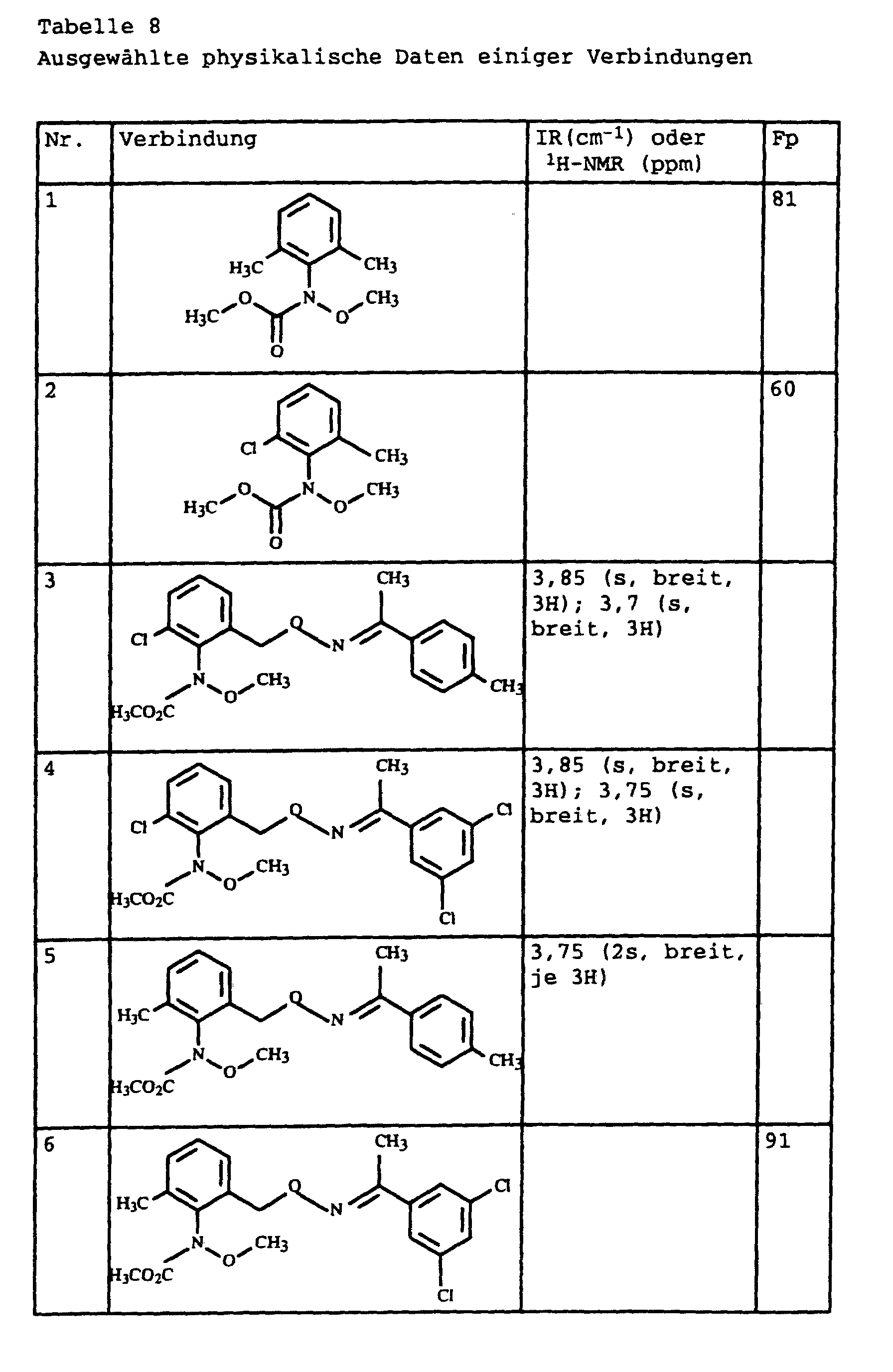

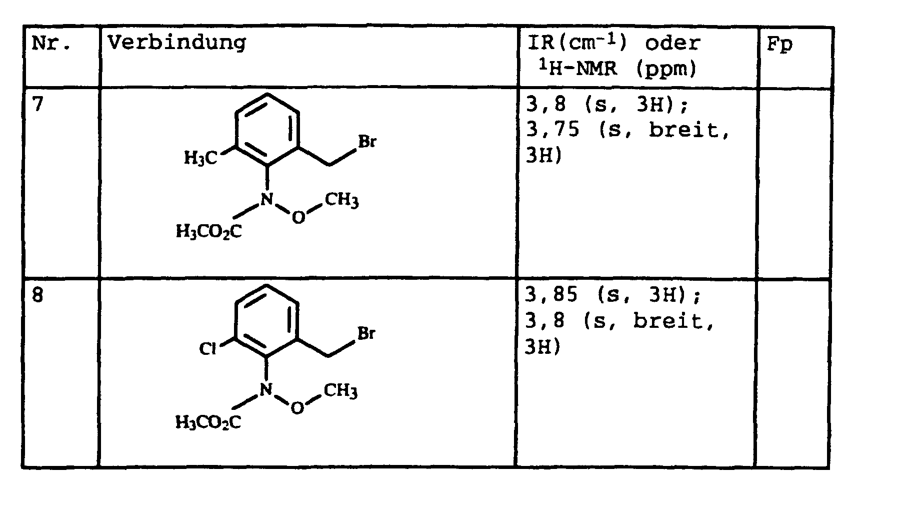

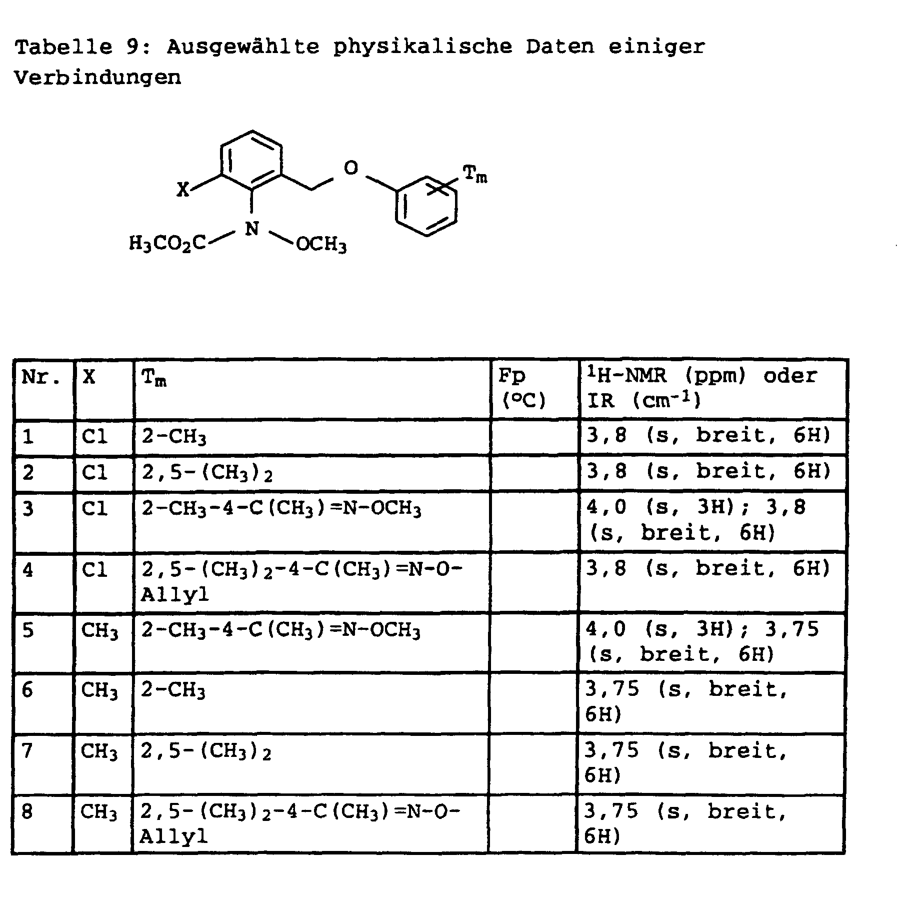

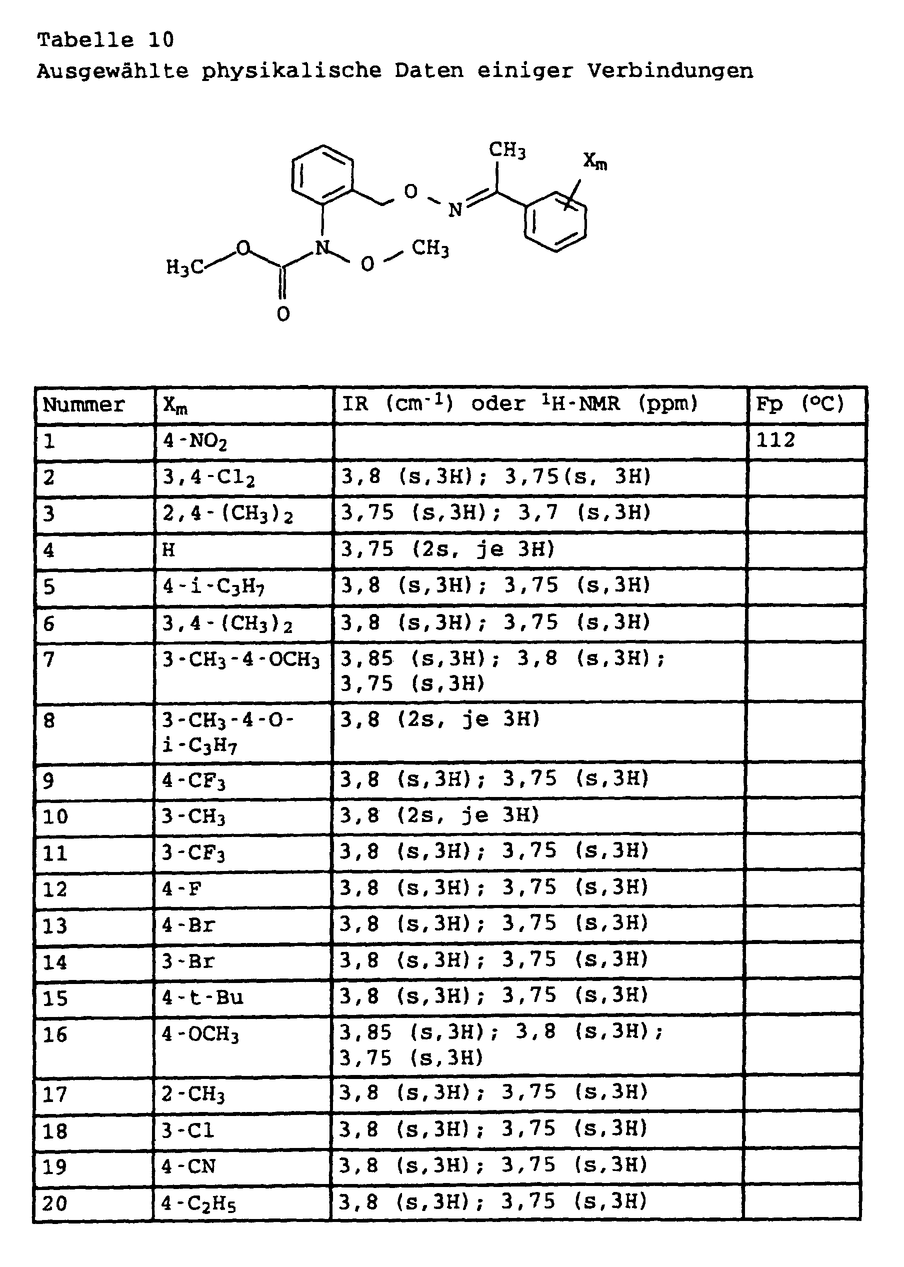

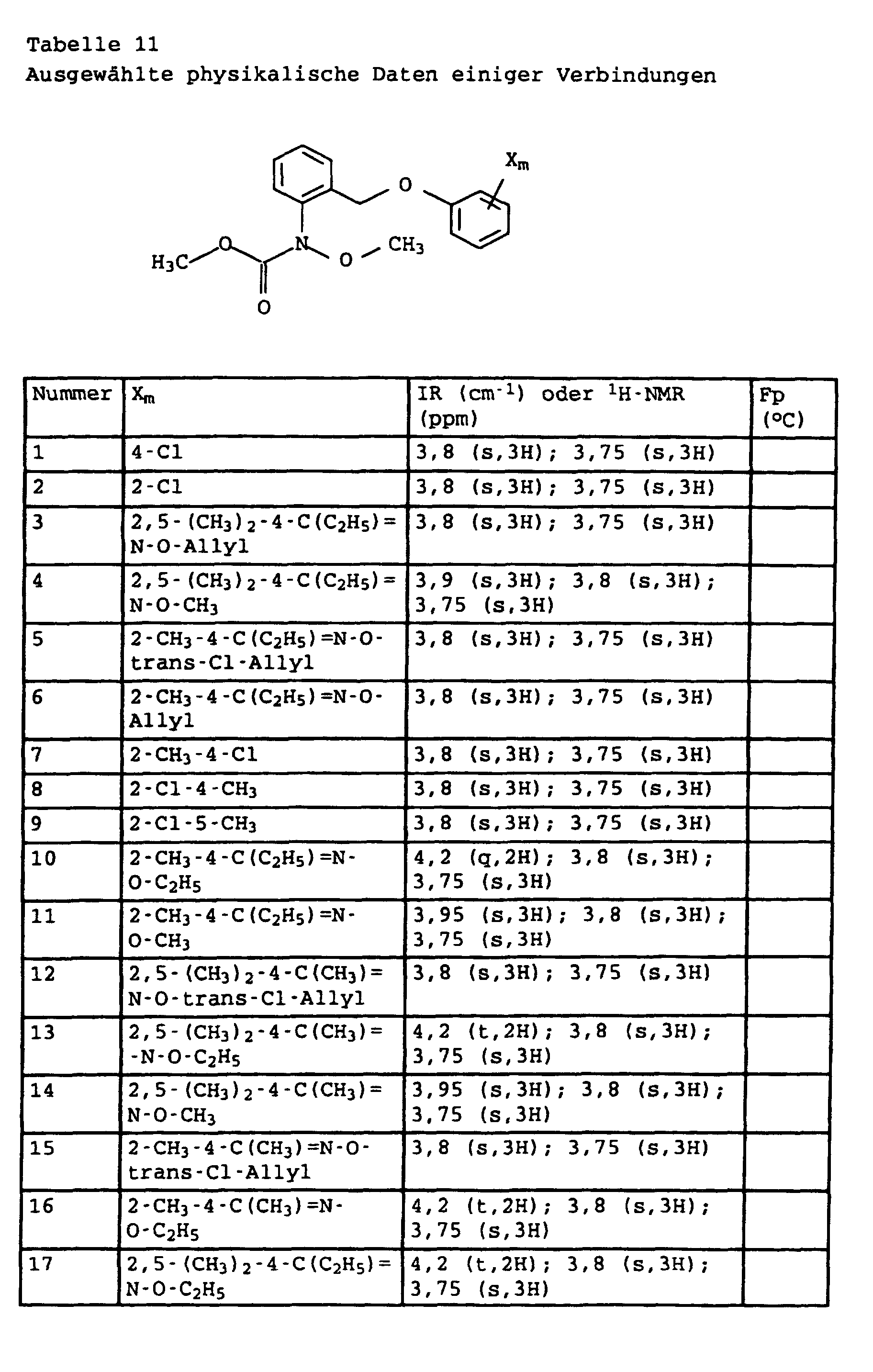

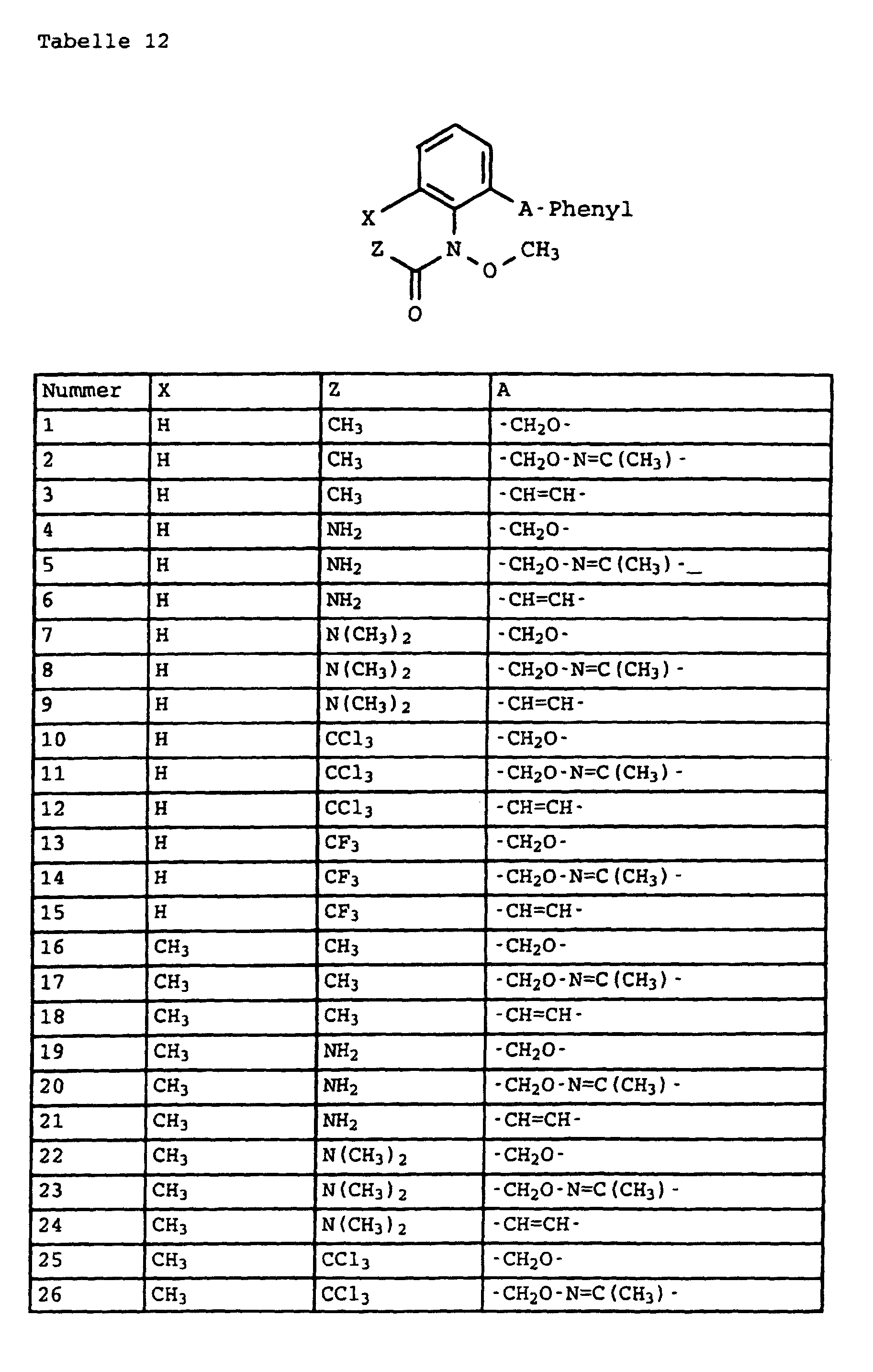

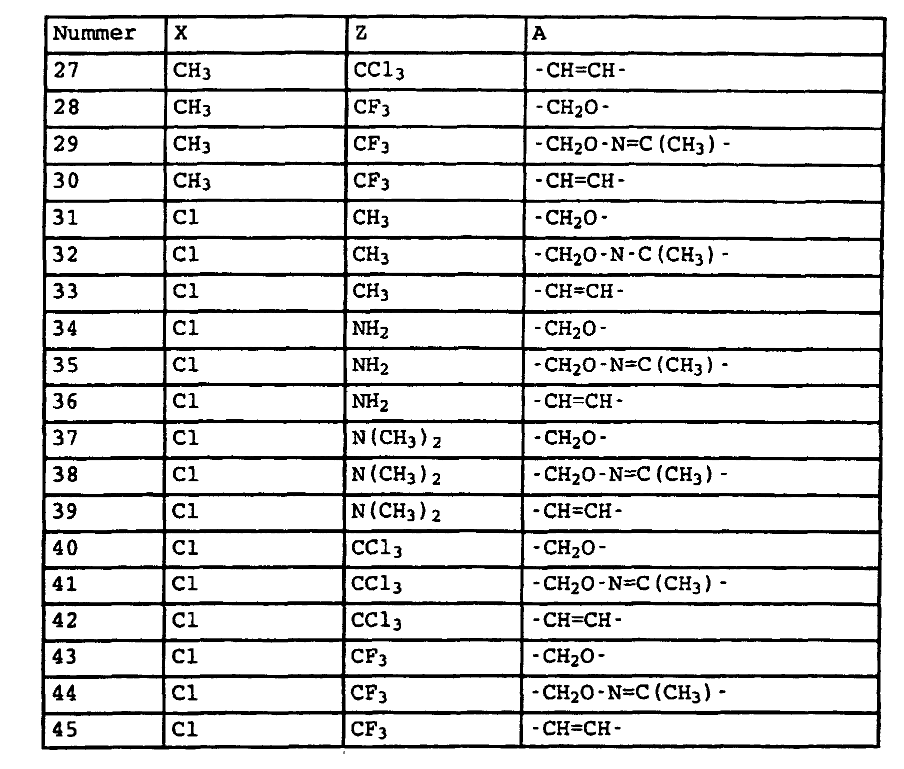

### Tabelle 13

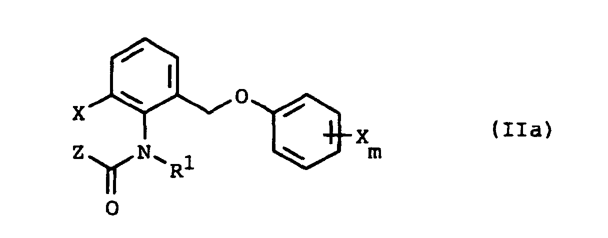

Verbindungen der Formel lla, in denen Z für OCH₃, X für H, R¹ für SCH₃ steht und Xₘ für eine Verbindung einer Zeile der Tabelle A entspricht

### Tabelle 14

Verbindungen der Formel lla, in denen Z für OCH₃, X für H, R¹ für OCH₃ steht und Xₘ für eine Verbindung einer Zeile der Tabelle A entspricht

### Tabelle 15

Verbindungen der Formel lla, in denen Z für OCH₃, X für H, R¹ für OCH₂CH₃ steht und Xₘ für eine Verbindung einer Zeile der Tabelle A entspricht

### Tabelle 16

Verbindungen der Formel lla, in denen Z für CH₃, X für H, R¹ für OCH₃ steht und Xₘ für eine Verbindung einer Zeile der Tabelle A entspricht

### Tabelle 17

Verbindungen der Formel lla, in denen Z für CH₃, X für H; R¹ für OCH₂CH₃ steht und Xₘ für eine Verbindung einer Zeile der Tabelle A entspricht

### Tabelle 18

Verbindungen der Formel lla, in denen Z für CH₂CH₃, X für H, R¹ für OCH₃ steht und Xₘ für eine Verbindung einer Zeile der Tabelle A entspricht

### Tabelle 19

Verbindungen der Formel IIa, in denen Z für CH₂CH₃, X für H, R¹ für OCH₂CH₃ steht und Xₘ für eine Verbindung einer Zeile der Tabelle A entspricht

### Tabelle 20

Verbindungen der Formel lla, in denen Z für NHCH₃, X für H, R¹ für OCH₃ steht und Xₘ für eine Verbindung einer Zeile der Tabelle A entspricht

### Tabelle 21

Verbindungen der Formel lla, in denen Z für NHCH₃, X für H, R¹ für OCH₂CH₃ steht und Xₘ für eine Verbindung einer Zeile der Tabelle A entspricht

### Tabelle 22

Verbindungen der Formel lla, in denen Z für OCH₃, X für CH₃, R¹ für OCH₃ steht und Xₘ für eine Verbindung einer Zeile der Tabelle A entspricht

### Tabelle 23

Verbindungen der Formel lla, in denen Z für OCH₃, X für CH₃, R¹ für OCH₂CH₃ steht und Xₘ für eine Verbindung einer Zeile der Tabelle A entspricht

### Tabelle 24

Verbindungen der Formel lla, in denen Z für OCH₃, X für Cl, R¹ für OCH₃ steht und Xₘ für eine Verbindung einer Zeile der Tabelle A entspricht

### Tabelle 25

Verbindungen der Formel lla, in denen Z für OCH₃, X für Cl, R¹ für OCH₂CH₃ steht und Xₘ für eine Verbindung einer Zeile der Tabelle A entspricht

### Tabelle 26

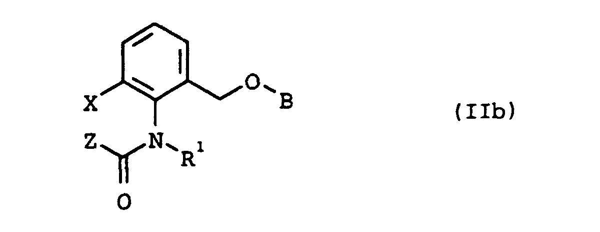

Verbindungen der Formel IIb, in denen Z für OCH₃, X für H, R¹ für SCH₃ steht und B für eine Verbindung einer Zeile der Nrn. 1 bis 383, 385 und 386 der Tabelle B entspricht

### Tabelle 27

Verbindungen der Formel IIb, in denen Z für OCH₃, X für H, R¹ für OCH₃ steht und B für eine Verbindung einer Zeile der Tabelle B entspricht

### Tabelle 28

Verbindungen der Formel IIb, in denen Z für OCH₃, X für H, R¹ für OCH₂CH₃ steht und B für eine Verbindung einer Zeile der Tabelle B entspricht

### Tabelle 29

Verbindungen der Formel IIb, in denen Z für CH₃, X für H, R¹ für OCH₃ steht und B für eine Verbindung einer Zeile der Tabelle B entspricht

### Tabelle 30

Verbindungen der Formel IIb, in denen Z für CH₃, X für H, R¹ für OCH₂CH₃ steht und B für eine Verbindung einer Zeile der Tabelle B entspricht

### Tabelle 31

Verbindungen der Formel IIb, in denen Z für CH₂CH₃, X für H, R¹ für OCH₃ steht und B für eine Verbindung einer Zeile der Tabelle B entspricht

### Tabelle 32

Verbindungen der Formel IIb, in denen Z für CH₂CH₃, X für H, R¹ für OCH₂CH₃ steht und B für eine Verbindung einer Zeile der Tabelle B entspricht

### Tabelle 33

Verbindungen der Formel IIb, in denen Z für NHCH₃, X für H, R¹ für OCH₃ steht und B für eine Verbindung einer Zeile der Tabelle B entspricht

### Tabelle 34

Verbindungen der Formel IIb, in denen Z für NHCH₃, X für H, R¹ für OCH₂CH₃ steht und B für eine Verbindung einer Zeile der Tabelle B entspricht

### Tabelle 35

Verbindungen der Formel IIb, in denen Z für OCH₃, X für CH₃, R¹ für OCH₃ steht und B für eine Verbindung einer Zeile der Tabelle B entspricht

### Tabelle 36

Verbindungen der Formel IIb, in denen Z für OCH₃, X für CH₃, R¹ für OCH₂CH₃ steht und B für eine Verbindung einer Zeile der Tabelle B entspricht

### Tabelle 37

Verbindungen der Formel IIb, in denen Z für OCH₃, X für Cl, R¹ für OCH₃ steht und B für eine Verbindung einer Zeile der Tabelle B entspricht

### Tabelle 38

Verbindungen der Formel IIb, in denen Z für OCH₃, X für Cl, R¹ für OCH₂CH₃ steht und B für eine Verbindung einer Zeile der Tabelle B entspricht

### Tabelle 39

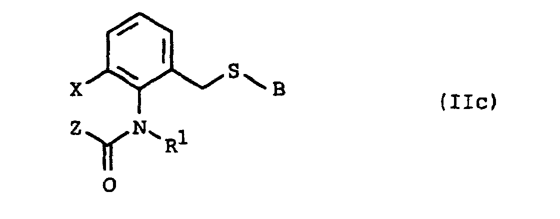

Verbindungen der Formel llc, in denen Z für CH₃, X für H, R¹ für OCH₃ steht und B für eine Verbindung einer Zeile der Tabelle C entspricht

### Tabelle 40

Verbindungen der Formel llc, in denen Z für CH₃, X für H, R¹ für OCH₂CH₃ steht und B für eine Verbindung einer Zeile der Tabelle C entspricht

### Tabelle 41

Verbindungen der Formel llc, in denen Z für CH₂CH₃, X für H, R¹ für OCH₃ steht und B für eine Verbindung einer Zeile der Tabelle C entspricht

### Tabelle 42

Verbindungen der Formel IIc, in denen Z für CH₂CH₃, X für H, R¹ für OCH₂CH₃ steht und B für eine Verbindung einer Zeile der Tabelle C entspricht

### Tabelle 43

Verbindungen der Formel llc, in denen Z für NHCH₃, X für H, R¹ für OCH₃ steht und B für eine Verbindung einer Zeile der Tabelle C entspricht

### Tabelle 44

Verbindungen der Formel llc, in denen Z für NHCH₃, X für H, R¹ für OCH₂CH₃ steht und B für eine Verbindung einer Zeile der Tabelle C entspricht

### Tabelle 45

Verbindungen der Formel llc, in denen Z für OCH₃, X für CH₃, R¹ für OCH₃ steht und B für eine Verbindung einer Zeile der Tabelle C entspricht

### Tabelle 46

Verbindungen der Formel llc, in denen Z für OCH₃, X für CH₃, R¹ für OCH₂CH₃ steht und B für eine Verbindung einer Zeile der Tabelle C entspricht

### Tabelle 47

Verbindungen der Formel llc, in denen Z für OCH₃, X für Cl, R¹ für OCH₃ steht und B für eine Verbindung einer Zeile der Tabelle C entspricht

### Tabelle 48

Verbindungen der Formel llc, in denen Z für OCH₃, X für Cl, R¹ für OCH₂CH₃ steht und B für eine Verbindung einer Zeile der Tabelle C entspricht

### Tabelle 49

Verbindungen der Formel llc, in denen Z für OCH₃, X für H, R¹ für OCH₃ steht und B für eine Verbindung einer Zeile der Tabelle C entspricht

### Tabelle 50

Verbindungen der Formel IIc, in denen Z für OCH₃, X für H, R¹ für OCH₂CH₃ steht und B für eine Verbindung einer Zeile der Tabelle C entspricht

### Tabelle 51

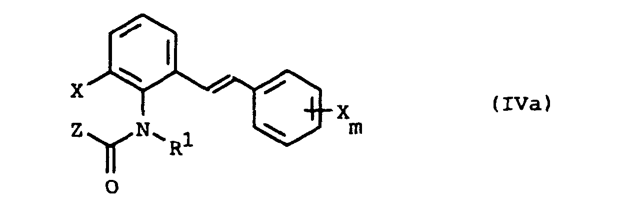

Verbindungen der Formel IVa, in denen Z für OCH₃, X für H, R¹ für SCH₃ steht und Xₘ für eine Verbindung einer Zeile der Nrn. 1 bis 640 der Tabelle A entspricht

### Tabelle 52

Verbindungen der Formel IVa, in denen Z für OCH₃, X für H, R¹ für OCH₃ steht und Xₘ für eine Verbindung einer Zeile der Nrn. 1 bis 640 der Tabelle A entspricht

### Tabelle 53

Verbindungen der Formel IVa, in denen Z für OCH₃, X für H, R¹ für OCH₂CH₃ steht und Xₘ für eine Verbindung einer Zeile der Nrn. 1 bis 640 der Tabelle A entspricht

### Tabelle 54

Verbindungen der Formel IVa, in denen Z für CH₃, X für H, R¹ für OCH₃ steht und Xₘ für eine Verbindung einer Zeile der Nrn. 1 bis 640 der Tabelle A entspricht

### Tabelle 55

Verbindungen der Formel IVa, in denen Z für CH₃ X für H, R¹ für OCH₂CH₃ steht und Xₘ für eine Verbindung einer Zeile der Nrn. 1 bis 640 der Tabelle A entspricht

### Tabelle 56

Verbindungen der Formel IVa, in denen Z für CH₂CH₃, X für H, R¹ für OCH₃ steht und Xₘ für eine Verbindung einer Zeile der Nrn. 1 bis 640 der Tabelle A entspricht

### Tabelle 57

Verbindungen der Formel IVa, in denen Z für CH₂CH₃ X für H, R¹ für OCH₂CH₃ steht und Xₘ für eine Verbindung einer Zeile der Nrn. 1 bis 640 der Tabelle A entspricht

### Tabelle 58

Verbindungen der Formel IVa, in denen Z für NHCH₃, X für H, R¹ für OCH₃ steht und Xₘ für eine Verbindung einer Zeile der Nrn. 1 bis 640 der Tabelle A entspricht

### Tabelle 59

Verbindungen der Formel IVa, in denen Z für NHCH₃, X für H, R¹ für OCH₂CH₃ steht und Xₘ für eine Verbindung einer Zeile der Nrn. 1 bis 640 der Tabelle A entspricht

### Tabelle 60

Verbindungen der Formel IVa, in denen Z für OCH₃, X für CH₃, R¹ für OCH₃ steht und Xₘ für eine Verbindung einer Zeile der Nrn. 1 bis 640 der Tabelle A entspricht

### Tabelle 61

Verbindungen der Formel IVa, in denen Z für OCH₃, X für CH₃, R¹ für OCH₂CH₃ steht und Xₘ für eine Verbindung einer Zeile der Nrn. 1 bis 640 der Tabelle A entspricht

### Tabelle 62

Verbindungen der Formel IVa, in denen Z für OCH₃, X für Cl, R¹ für OCH₃ steht und Xₘ für eine Verbindung einer Zeile der Nrn. 1 bis 640 der Tabelle A entspricht

### Tabelle 63

Verbindungen der Formel IVa, in denen Z für OCH₃, X für Cl, R¹ für OCH₂CH₃ steht und Xₘ für eine Verbindung einer Zeile der Nrn. 1 bis 640 der Tabelle A entspricht

### Tabelle 64

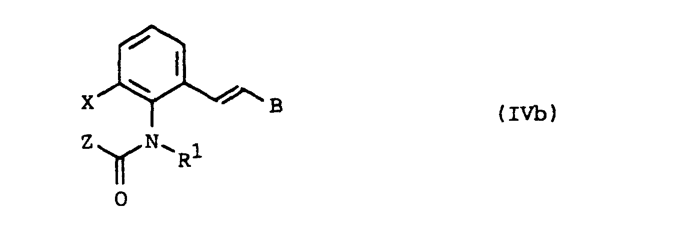

Verbindungen der Formel IVb, in denen Z für OCH₃, X für H, R¹ für SCH₃ steht und B für eine Verbindung einer Zeile der Nrn. 1 bis 383 der Tabelle B entspricht

### Tabelle 65

Verbindungen der Formel IVb, in denen Z für OCH₃, X für H, R¹ für OCH₃ steht und B für eine Verbindung einer Zeile der Nrn. 1 bis 384 der Tabelle B entspricht

### Tabelle 66

Verbindungen der Formel IVb, in denen Z für OCH₃, X für H, R¹ für OCH₂CH₃ steht und B für eine Verbindung einer Zeile der Nrn. 1 bis 384 der Tabelle B entspricht

### Tabelle 67

Verbindungen der Formel IVb, in denen Z für CH₃, X für H, R¹ für OCH₃ steht und B für eine Verbindung einer Zeile der Nrn. 1 bis 384 der Tabelle B entspricht

### Tabelle 68

Verbindungen der Formel IVb, in denen Z für CH₃, X für H, R¹ für OCH₂CH₃ steht und B für eine Verbindung einer Zeile der Nrn. 1 bis 384 der Tabelle B entspricht

### Tabelle 69

Verbindungen der Formel IVb, in denen Z für CH₂CH₃, X für H, R¹ für OCH₃ steht und B für eine Verbindung einer Zeile der Nrn. 1 bis 384 der Tabelle B entspricht

### Tabelle 70

Verbindungen der Formel IVb, in denen Z für CH₂CH₃, X für H, R¹ für OCH₂CH₃ steht und B für eine Verbindung einer Zeile der Nrn. 1 bis 384 der Tabelle B entspricht

### Tabelle 71

Verbindungen der Formel IVb, in denen Z für NHCH₃, X für H, R¹ für OCH₃ steht und B für eine Verbindung einer Zeile der Nrn. 1 bis 384 der Tabelle B entspricht

### Tabelle 72

Verbindungen der Formel IVb, in denen Z für NHCH₃, X für H, R¹ für OCH₂CH₃ steht und B für eine Verbindung einer Zeile der Nrn. 1 bis 384 der Tabelle B entspricht

### Tabelle 73

Verbindungen der Formel IVb, in denen Z für OCH₃, X für CH₃, R¹ für OCH₃ steht und B für eine Verbindung einer Zeile der Nrn. 1 bis 384 der Tabelle B entspricht

### Tabelle 74

Verbindungen der Formel IVb, in denen Z für OCH₃, X für CH₃, R¹ für OCH₂CH₃ steht und B für eine Verbindung einer Zeile der Nrn. 1 bis 384 der Tabelle B entspricht

### Tabelle 75

Verbindungen der Formel IVb, in denen Z für OCH₃, X für Cl, R¹ für OCH₃ steht und B für eine Verbindung einer Zeile der Nrn. 1 bis 384 der Tabelle B entspricht

### Tabelle 76

Verbindungen der Formel IVb, in denen Z für OCH₃, X für Cl, R¹ für OCH₂CH₃ steht und B für eine Verbindung einer Zeile der Nrn. 1 bis 384 der Tabelle B entspricht

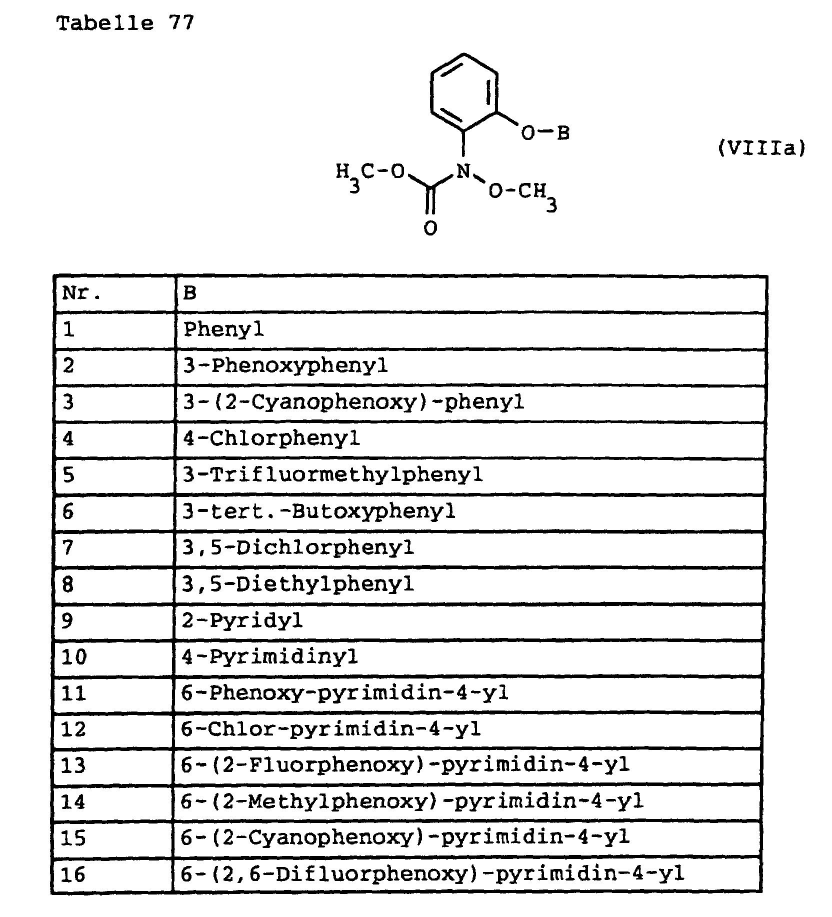

### Tabelle 78

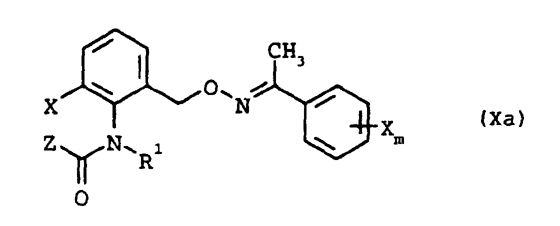

Verbindungen der Formel Xa, in denen Z für OCH₃, X für H, R¹ für SCH₃ steht und Xₘ für eine Verbindung einer Zeile der Tabelle A entspricht

### Tabelle 79

Verbindungen der Formel Xa, in denen Z für OCH₃, X für H, R¹ für OCH₃ steht und Xₘ für eine Verbindung einer Zeile der Tabelle A entspricht

### Tabelle 80

Verbindungen der Formel Xa, in denen Z für OCH₃, X für H, R¹ für OCH₂CH₃ steht und Xₘ für eine Verbindung einer Zeile der Tabelle A entspricht

### Tabelle 81

Verbindungen der Formel Xa, in denen Z für CH₃, X für H, R¹ für OCH₃ steht und Xₘ für eine Verbindung einer Zeile der Tabelle A entspricht

### Tabelle 82

Verbindungen der Formel Xa, in denen Z für CH₃, X für H, R¹ für OCH₂CH₃ steht und Xₘ für eine Verbindung einer Zeile der Tabelle A entspricht

### Tabelle 83

Verbindungen der Formel Xa, in denen Z für CH₂CH₃, X für H R¹ für OCH₃ steht und Xₘ für eine Verbindung einer Zeile der Tabelle A entspricht

### Tabelle 84

Verbindungen der Formel Xa, in denen Z für CH₂CH₃, X für H, R¹ für OCH₂CH₃ steht und Xₘ für eine Verbindung einer Zeile der Tabelle A entspricht

### Tabelle 85

Verbindungen der Formel Xa, in denen Z für NHCH₃, X für H, R¹ für OCH₃ steht und Xₘ für eine Verbindung einer Zeile der Tabelle A entspricht

### Tabelle 86

Verbindungen der Formel Xa, in denen Z für NHCH₃, X für H, R¹ für OCH₂CH₃ steht und Xₘ für eine Verbindung einer Zeile der Tabelle A entspricht

### Tabelle 87

Verbindungen der Formel Xa, in denen Z für OCH₃, X für CH₃, R¹ für OCH₃ steht und Xₘ für eine Verbindung einer Zeile der Tabelle A entspricht

### Tabelle 88

Verbindungen der Formel Xa, in denen Z für OCH₃ X für CH₃, R¹ für OCH₂CH₃ steht und Xₘ für eine Verbindung einer Zeile der Tabelle A entspricht

### Tabelle 89

Verbindungen der Formel Xa, in denen Z für OCH₃, X für Cl, R¹ für OCH₃ steht und Xₘ für eine Verbindung einer Zeile der Tabelle A entspricht

### Tabelle 90

Verbindungen der Formel Xa, in denen Z für OCH₃, X für Cl, R¹ für OCH₂CH₃ steht und Xₘ für eine Verbindung einer Zeile der Tabelle A entspricht

### Tabelle 91

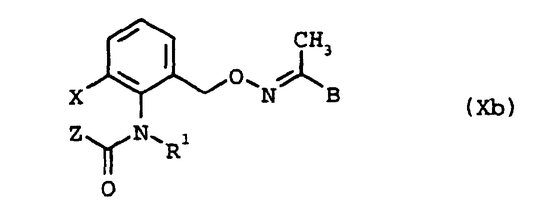

Verbindungen der Formel Xb, in denen Z für OCH₃, X für H, R¹ für SCH₃ steht und B für eine Verbindung einer Zeile der Nrn. 1 bis 383, 385 und 386 der Tabelle B entspricht

### Tabelle 92

Verbindungen der Formel Xb, in denen Z für OCH₃, X für H, R¹ für OCH₃ steht und B für eine Verbindung einer Zeile der Tabelle B entspricht

### Tabelle 93

Verbindungen der Formel Xb, in denen Z für OCH₃, X für H, R¹ für OCH₂CH₃ steht und B für eine Verbindung einer Zeile der Tabelle B entspricht

### Tabelle 94

Verbindungen der Formel Xb, in denen Z für CH₃, X für H, R¹ für OCH₃ steht und B für eine Verbindung einer Zeile der Tabelle B entspricht

### Tabelle 95

Verbindungen der Formel Xb, in denen Z für OCH₃, X für H, R¹ für OCH₂CH₃ steht und B für eine Verbindung einer Zeile der Tabelle B entspricht

### Tabelle 96

Verbindungen der Formel Xb, in denen Z für CH₂CH₃, X für H, R¹ für OCH₃ steht und B für eine Verbindung einer Zeile der Tabelle B entspricht

### Tabelle 97

Verbindungen der Formel Xb, in denen Z für CH₂CH₃, X für H, R¹ für OCH₂CH₃ steht und B für eine Verbindung einer Zeile der Tabelle B entspricht

### Tabelle 98

Verbindungen der Formel Xb, in denen Z für NHCH₃, X für H, R¹ für OCH₃ steht und B für eine Verbindung einer Zeile der Tabelle B entspricht

### Tabelle 99

Verbindungen der Formel Xb, in denen Z für NHCH₃, X für H, R¹ für OCH₂CH₃ steht und B für eine Verbindung einer Zeile der Tabelle B entspricht

### Tabelle 100

Verbindungen der Formel Xb, in denen Z für OCH₃, X für CH₃, R¹ für OCH₃ steht und B für eine Verbindung einer Zeile der Tabelle B entspricht

### Tabelle 101

Verbindungen der Formel Xb, in denen Z für OCH₃, X für CH₃, R¹ für OCH₂CH₃ steht und B für eine Verbindung einer Zeile der Tabelle B entspricht

### Tabelle 102

Verbindungen der Formel Xb, in denen Z für OCH₃, X für Cl, R¹ für OCH₃ steht und B für eine Verbindung einer Zeile der Tabelle B entspricht

### Tabelle 103

Verbindungen der Formel Xb, in denen Z für OCH₃, X für Cl, R¹ für OCH₂CH₃ steht und B für eine Verbindung einer Zeile der Tabelle B entspricht

**Tabelle A**

| Nummer | Xₘ |
|---|---|
| 1 | H |
| 2 | 2-F |
| 3 | 3-F |
| 4 | 4-F |
| 5 | 2,4-F₂ |
| 6 | 2,4,6-F₃ |
| 7 | 2,3,4,5,6-F₅ |
| 8 | 2,3-F₂ |
| 9 | 2-CI |
| 10 | 3-CI |
| 11 | 4-CI |
| 12 | 2,3-Cl₂ |
| 13 | 2,4-Cl₂ |
| 14 | 2,5-Cl₂ |
| 15 | 2,6-Cl₂ |
| 16 | 3,4-Cl₂ |
| 17 | 3,5-Cl₂ |
| 18 | 2,3,4-Cl₃ |
| 19 | 2,3,5-Cl₃ |
| 20 | 2,3,6-Cl₃ |
| 21 | 2,4,5-Cl₃ |
| 22 | 2,4,6-Cl₃ |
| 23 | 3,4,5-Cl₃ |
| 24 | 2,3,4,6-Cl₄ |
| 25 | 2,3,5,6-Cl₄ |
| 26 | 2,3,4,5,6-Cl₅ |
| 27 | 2-Br |
| 28 | 3-Br |
| 29 | 4-Br |
| 30 | 2,4-Br₂ |
| 31 | 2,5-Br₂ |
| 32 | 2,6-Br₂ |
| 33 | 2,4,6-Br₃ |
| 34 | 2,3,4,5,6-Br₅ |
| 35 | 2-J |
| 36 | 3-J |
| 37 | 4-J |
| 38 | 2,4-J₂ |
| 39 | 2-Cl, 3-F |
| 40 | 2-Cl, 4-F |
| 41 | 2-Cl, 5-F |
| 42 | 2-Cl, 6-F |
| 43 | 2-Cl, 3-Br |
| 44 | 2-Cl, 4-Br |
| 45 | 2-Cl, 5-Br |
| 46 | 2-Cl, 6-Br |
| 47 | 2-Br, 3-Cl |
| 48 | 2-Br, 4-Cl |
| 49 | 2-Br, 5-Cl |
| 50 | 2-Br, 3-F |
| 51 | 2-Br, 4-F |
| 52 | 2-Br, 5-F |
| 53 | 2-Br, 6-F |
| 54 | 2-F, 3-Cl |
| 55 | 2-F, 4-Cl |
| 56 | 2-F, 5-Cl |
| 57 | 3-Cl, 4-F |
| 58 | 3-Cl, 5-F |
| 59 | 3-Cl, 4-Br |
| 60 | 3-Cl, 5-Br |
| 61 | 3-F, 4-Cl |
| 62 | 3-F, 4-Br |
| 63 | 3-Br, 4-CI |
| 64 | 3-Br, 4-F |
| 65 | 2,6-Cl₂, 4-Br |
| 66 | 2-CH₃ |
| 67 | 3-CH₃ |
| 68 | 4-CH₃ |
| 69 | 2,3-(CH₃)₂ |
| 70 | 2,4-(CH₃)₂ |
| 71 | 2,5-(CH₃)₂ |
| 72 | 2,6-(CH₃)₂ |
| 73 | 3,4-(CH₃)₂ |
| 74 | 3,5-(CH₃)₂ |
| 75 | 2,3,5-(CH₃)₃ |
| 76 | 2,3,4-(CH₃)₃ |
| 77 | 2,3,6-(CH₃)₃ |
| 78 | 2,4,5-(CH₃)₃ |
| 79 | 2,4,6-(CH₃)₃ |
| 80 | 3,4,5-(CH₃)₃ |
| 81 | 2,3,4,6-(CH₃)₄ |
| 82 | 2,3,5,6-(CH₃)₄ |
| 83 | 2,3,4,5,6-(CH₃)₅ |
| 84 | 2-C₂H₅ |
| 85 | 3-C₂H₅ |
| 86 | 4-C₂H₅ |
| 87 | 2,4-(C₂H₅)₂ |
| 88 | 2,6-(C₂H₅)₂ |
| 89 | 3,5-(C₂H₅)₂ |
| 90 | 2,4,6-(C₂H₅)₃ |
| 91 | 2-n-C₃H₇ |
| 92 | 3-n-C₃H₇ |
| 93 | 4-n-C₃H₇ |
| 94 | 2-i-C₃H₇ |
| 95 | 3-i-C₃H₇ |
| 96 | 4-i-C₃H₇ |
| 97 | 2,4-(i-C₃H₇)₂ |
| 98 | 2,6-(i-C₃H₇)₂ |
| 99 | 3,5-(i-C₃H₇)₂ |
| 100 | 2,4,6-(i-C₃H₇)₃ |
| 101 | 2-s-C₄H₉ |
| 102 | 3-s-C₄H₉ |
| 103 | 4-s-C₄H₉ |
| 104 | 2-t-C₄H₉ |
| 105 | 3-t-C₄H₉ |
| 106 | 4-t-C₄H₉ |
| 107 | 2,3-(t-C₄H₉)₂ |
| 108 | 2,4-(t-C₄H₉)₂ |
| 109 | 2,5-(t-C₄H₉)₂ |
| 110 | 2,6-(t-C₄H₉)₂ |
| 111 | 3,4-(t-C₄H₉)₂ |
| 112 | 2,4,6-(t-C₄H₉)₃ |
| 113 | 4-n-C₉H₁₉ |
| 114 | 4-n-C₁₂H₂₅ |
| 115 | 4-n-C₁₅H₃₁ |
| 116 | 4-(1,1,3,3-Tetramethylbutyl) |
| 117 | 4-(2,4,4-Trimethylpropyl) |
| 118 | 2-t-C₄H₉, 4-CH₃ |
| 119 | 2-t-C₄H₉, 5-CH₃ |
| 120 | 2,6-(t-C₄H₉)₂, 4-CH₃ |
| 121 | 2-CH₃, 4-t-C₄H₉ |
| 122 | 2-CH₃, 6-t-C₄H₉ |
| 123 | 2-CH₃, 4-i-C₃H₇ |
| 124 | 2-CH₃, 5-i-C₃H₇ |
| 125 | 3-CH₃ 4-i-C₃H₇ |
| 126 | 2-i-C₃H₇, 5-CH₃ |
| 127 | 2,4-(t-C₄H₉)₂, 6-i-C₃H₇ |
| 128 | 2-Allyl |
| 129 | 3-Allyl |
| 130 | 4-Allyl |
| 131 | 2-Allyl, 6-CH₃ |
| 132 | 2-cyclo-C₆H₁₁ |
| 133 | 3-cyclo-C₆H₁₁ |
| 134 | 4-cyclo-C₆H₁₁ |
| 135 | 2,4-(cyclo-C₆H₁₁)₂, 6-CH₃ |
| 136 | 2-CH₃ 4-cyclo-C₆H₁₁ |
| 137 | 2-CH₂-C₆H₅ |
| 138 | 3-CH₂-C₆H₅ |
| 139 | 4-CH₂-C₆H₅ |
| 140 | 2-CH₂-C₆H₅, 4-CH₃ |
| 141 | 2-CH₃, 4-CH₂-C₆H₅ |
| 142 | 2-C₆H₅ |
| 143 | 3-C₆H₅ |
| 144 | 4-C₆H₅ |
| 145 | 4-(2-i-C₃H₇-C₆H₄) |
| 146 | 4-C₆H₅, 2,6-(CH₃)₂ |
| 147 | 2-Cl, 4-C₆H₅ |
| 148 | 2-Br, 4-C₆H₅ |
| 149 | 2-C₆H₅, 4-Cl |
| 150 | 2-C₆H₅, 4-Br |
| 151 | 2-CH₂C₆H₅, 4-CI |
| 152 | 2-CH₂C₆H₅, 4-Br |
| 153 | 2-Cl, 4-CH₂C₆H₅ |
| 154 | 2-Br, 4-CH₂C₆H₅ |
| 155 | 2-cyclo-C₆H₁₁, 4-Cl |
| 156 | 2-cyclo-C₆H₁₁, 4-Br |
| 157 | 2-Cl, 4-cyclo-C₆H₁₁ |
| 158 | 2-Br, 4-cyclo-C₆H₁₁ |
| 159 | 2-OCH₃ |
| 160 | 3-OCH₃ |
| 161 | 4-OCH₃ |
| 162 | 2-OC₂H₅ |
| 163 | 3-O-C₂H₅ |
| 164 | 4-O-C₂H₅ |
| 165 | 2-O-n-C₃H₇ |
| 166 | 3-O-n-C₃H₇ |
| 167 | 4-O-n-C₃H₇ |
| 168 | 2-O-i-C₃H₇ |
| 169 | 3-O-i-C₃H₇ |
| Nummer | Xₘ |
| 170 | 4-O-i-C₃H₇ |
| 171 | 2-O-n-C₆H₁₃ |
| 172 | 3-O-n-C₆H₁₃ |
| 173 | 4-O-n-C₆H₁₃ |
| 174 | 2-O-n-C₈H₁₇ |
| 175 | 3-O-n-C₈H₁₇ |
| 176 | 4-O-n-C₈H₁₇ |
| 177 | 2-O-CH₂C₆H₅ |
| 178 | 3-O-CH₂C₆H₅ |
| 179 | 4-O-CH₂C₆H₅ |
| 180 | 2-O-(CH₂)₃C₆H₅ |
| 181 | 3-O-(CH₂)₃C₆H₅ |
| 182 | 4-O-(CH₂)₃C₆H₅ |
| 183 | 2,4-(OCH₃)₂ |
| 184 | 2-CF₃ |
| 185 | 3-CF₃ |
| 186 | 4-CF₃ |
| 187 | 2-OCF₃ |
| 188 | 3-OCF₃ |
| 189 | 4-OCF₃ |
| 190 | 3-OCH₂CHF₂ |
| 191 | 2-NO₂ |
| 192 | 3-NO₂ |
| 193 | 4-NO₂ |
| 194 | 2-CN |
| 195 | 3-CN |
| 196 | 4-CN |
| 197 | 2-CH₃, 3-CI |
| 198 | 2-CH₃, 4-CI |
| 199 | 2-CH₃, 5-CI |
| 200 | 2-CH₃, 6-CI |
| 201 | 2-CH₃, 3-F |
| 202 | 2-CH₃, 4-F |
| 203 | 2-CH₃, 5-F |
| 204 | 2-CH₃, 6-F |
| 205 | 2-CH₃, 3-Br |
| 206 | 2-CH₃, 4-Br |
| 207 | 2-CH₃, 5-Br |
| 208 | 2-CH₃, 6-Br |
| 209 | 2-Cl, 3-CH₃ |
| 210 | 2-Cl, 4-CH₃ |
| 211 | 2-Cl, 5-CH₃ |
| 212 | 2-F, 3-CH₃ |
| 213 | 2-F, 4-CH₃ |
| 214 | 2-F, 5-CH₃ |
| 215 | 2-Br, 3-CH₃ |
| 216 | 2-Br, 4-CH₃ |
| 217 | 2-Br, 5-CH₃ |
| 218 | 3-CH₃, 4-CI |
| 219 | 3-CH₃, 5-CI |
| 220 | 3-CH₃, 4-F |
| 221 | 3-CH₃, 5-F |
| 222 | 3-CH₃, 4-Br |
| 223 | 3-CH₃, 5-Br |
| 224 | 3-F, 4-CH₃ |
| 225 | 3-Cl, 4-CH₃ |
| 226 | 3-Br, 4-CH₃ |
| 227 | 2-Cl, 4,5-(CH₃)₂ |
| 228 | 2-Br, 4,5-(CH₃)₂ |
| 229 | 2-Cl, 3,5-(CH₃)₂ |
| 230 | 2- Br, 3,5-(CH₃)₂ |
| 231 | 2,6-Cl₂, 4-CH₃ |
| 232 | 2,6-F₂, 4-CH₃ |
| 233 | 2,6-Br₂, 4-CH₃ |
| 234 | 24-Br₂, 6-CH₃ |
| 235 | 2,4-F₂, 6-CH₃ |
| 236 | 2,4-Br₂, 6-CH₃ |
| 237 | 2,6-(CH₃)₂, 4-F |
| 238 | 2,6-(CH₃)₂, 4-Cl |
| 239 | 2,6-(CH₃)₂, 4-Br |
| 240 | 3,5-(CH₃)₂, 4-F |
| 241 | 3,5-(CH₃)₂, 4-Cl |
| 242 | 3,5-(CH₃)₂, 4-Br |
| 243 | 2,3,6-(CH₃)₃, 4-F |
| 244 | 2,3,6-(CH₃)₃, 4-Cl |
| 245 | 2,3,6-(CH₃)₃, 4-Br |
| 246 | 2,4-(CH₃)₂, 6-F |
| 247 | 2,4-(CH₃)₂, 6-Cl |
| 248 | 2,4-(CH₃)₂, 6-Br |
| 249 | 2-i-C₃H₇, 4-Cl, 5-CH₃ |
| 250 | 2-Cl, 4-NO₂ |
| 251 | 2-NO₂, 4-Cl |
| 252 | 2-OCH₃, 5-NO₂ |
| 253 | 2,4-Cl₂, 5-NO₂ |
| 254 | 2,4-Cl₂, 6-NO₂ |
| 255 | 2,6-Cl₂, 4-NO₂ |
| 256 | 2,6-Br₂, 4-NO₂ |
| 257 | 2,6-J₂, 4-NO₂ |
| 258 | 2-CH₃, 5-i-C₃H₇, 4-Cl |
| 259 | 2-CO₂CH₃ |
| 260 | 3-CO₂CH₃ |
| 261 | 4-CO₂CH₃ |
| 262 | 2-CO₂(C₂H₅) |
| 263 | 3-CO₂(C₂H₅) |
| 264 | 4-CO₂(C₂H₅) |
| 265 | 2-CO₂(n-C₃H₇) |
| 266 | 3-CO₂(n-C₃H₇) |
| 267 | 4-CO₂(n-C₃H₇) |
| 268 | 2-CO₂(i-C₃H₇) |
| 269 | 3-CO₂(i-C₃H₇) |
| 270 | 4-CO₂(i-C₃H₇) |
| 271 | 2-CO₂(n-C₆H₁₃) |
| 272 | 3-CO₂(n-C₆H₁₃) |
| 273 | 4-CO₂(n-C₆H₁₃) |
| 274 | 2-CH₂-OCH₃ |
| 275 | 3-CH₂-OCH₃ |
| 276 | 4-CH₂-OCH₃ |
| 277 | 2-CH₂O(C₂H₅) |
| 278 | 3-CH₂O(C₂H₅) |
| 279 | 4-CH₂O(C₂H₅) |
| 280 | 2-CH₂O(n-C₃H₇) |
| 281 | 3-CH₂O(n-C₃H₇) |
| 282 | 4-CH₂O(n-C₃H₇) |
| 283 | 2-CH₂O(i-C₃H₇) |
| 284 | 3-CH₂O(i-C₃H₇) |
| 285 | 4-CH₂O(i-C₃H₇) |
| 286 | 2-CHO |
| 287 | 3-CHO |
| 288 | 4-CHO |
| 289 | 2-CO-CH₃ |
| 290 | 3-CO-CH₃ |
| 291 | 4-CO-CH3 |
| 292 | 2-CO-CH₂-CH₃ |
| 293 | 3-CO-CH₂-CH₃ |
| 294 | 4-CO-CH₂-CH₃ |
| 295 | 2-CO-CH₂-CH₂-CH₃ |
| 296 | 3-CO-CH₂-CH₂-CH₃ |
| 297 | 4-CO-CH₂-CH₂-CH₃ |
| 298 | 2-CO-CH(CH₃)-CH₃ |
| 299 | 3-CO-CH(CH₃))-CH₃ |
| 300 | 4-CO-CH(CH₃)-CH₃ |
| 301 | 2-Me-4-CHO |
| 302 | 2-Me-4-CH₃-CO |
| 303 | 2-Me-4-CH₃-CH₂-CO |
| 304 | 2-Me-4-CH₃-CH₂-CH₂-CO |
| 305 | 2-Me-4-CH₃-CH(CH₃)-CO |
| 306 | 2,5-Me₂-4-CHO |
| 307 | 2,5-Me₂-4-CH₃-CO |
| 308 | 2,5-Me₂-4-CH₃-CH₂-CO |
| 309 | 2,5-Me₂-4-CH₃-CH₂-CH₂-CO |
| 310 | 2,5-Me₂-4-CH₃-CH(CH₃)-CO |
| 311 | 2-Cl-4-CHO |
| 312 | 2-Cl-4-CH₃-CO |
| 313 | 2-Cl-4-CH₃-CH₂-CO |
| 314 | 2-Cl-4-CH₃-CH(CH₃)-CO |
| 315 | 2,5-Cl₂-4-CHO |
| 316 | 2,5-Cl₂-4-CH₃-CO |
| 317 | 2,5-Cl₂-4-CH₃-CH₂-CO |
| 318 | 2,5-Cl₂-4-CH₃-CH₂-CH₂-CO |
| 319 | 2,5-Cl₂-4-CH₃--CH(CH₃)-CO |
| 320 | 2-C(=NOCH₃)-CH₃ |
| 321 | 3-C(=NOCH₃)-CH₃ |
| 322 | 4-C(=NOCH₃)-CH₃ |
| 323 | 2-C(=NOC₂H₅)-CH₃ |
| 324 | 3-C(=NOC₂H₅)-CH₃ |
| 325 | 4-C(=NOC₂H₅)-CH₃ |
| 326 | 2-C(=NO-n-C₃H₇)-CH₃ |
| 327 | 3-C(=NO-n-C₃H₇)-CH₃ |
| 328 | 4-C(=NO-n-C₃H₇)-CH₃ |
| 329 | 2-C(=NO-i-C₃H₇)-CH₃ |
| 330 | 3-C(=NO-i-C₃H₇)-CH₃ |
| 331 | 4-C(=NO-i-C₃H₇)-CH₃ |
| 332 | 2-C(=NO-Allyl)-CH₃ |
| 333 | 3-C(=NO-Allyl)-CH₃ |
| 334 | 4-C(=NO-Allyl)-CH₃ |
| 335 | 2-C(=NO-trans-Chlorallyl)-CH₃ |
| 336 | 3-C(=NO-trans-Chlorallyl)-CH₃ |
| 337 | 4-C(=NO-trans-Chlorallyl)-CH₃ |
| 338 | 2-C(=NO-Propargyl)-CH₃ |
| 339 | 3-C(=NO-Propargyl)-CH₃ |
| 340 | 4-C(=NO-Propargyl)-CH₃ |
| 341 | 2-C(=NO-n-C₄H₉)-CH₃ |
| 342 | 3-C(=NO-n-C₄H₉)-CH₃ |
| 343 | 4-C(=NO-n-C₄H₉)-CH₃ |
| 344 | 2-C(=NO-CH₂-C₆H₅)-CH₃ |
| 345 | 3-C(=NO-CH₂-C₆H₅)-CH₃ |
| 346 | 4-C(=NO-CH₂-C₆H₅)-CH₃ |
| 347 | 2-CH₃-4-CH=NOCH₃ |
| 348 | 2-CH₃-4-CH=NOC₂H₅ |
| 349 | 2-CH₃-4-CH=NO-n-C₃H₇ |
| 350 | 2-CH₃-4-CH=NO-i-C₃H₇ |
| 351 | 2-CH₃-4-CH=NO-Allyl |
| 352 | 2-CH₃-4-CH=NO-(trans-Chlorallyl) |
| 353 | 2-CH₃-4-CH=NO-Propargyl |
| 354 | 2-CH₃-4-CH=NO-n-C₄H₉ |
| 355 | 2-CH₃-4-CH=NO-CH₂-C₆H₅ |
| 356 | 2-CH₃-4-(CH₃-C=NOCH₃) |
| 357 | 2-CH₃-4-(CH₃-C=NOC₂H₅) |
| 358 | 2-CH₃-4-(CH₃-C=NO-n-C₃H₇) |
| 359 | 2-CH₃-4-(CH₃-C=NO-i-C₃H₇) |
| 360 | 2-CH₃-4-(CH₃-C=NO-Allyl) |
| 361 | 2-CH₃-4-(CH₃-C=NO-trans-Chlorallyl) |
| 362 | 2-CH₃-4-(CH₃-C=NO-Propargyl) |
| 363 | 2-CH₃-4-(CH₃-C=NO-n-C₄H₉) |
| 364 | 2-CH₃-4-(CH₃-C=NO-CH₂-C₆H₅) |
| 365 | 2-CH₃-4-(C₂H₅-C=NO-CH₃) |
| 366 | 2-CH₃-4-(C₂H₅-C=NO-C₂H₅) |
| 367 | 2-CH₃-4-(C₂H₅-C=NO-n-C₃H₇) |
| 368 | 2-CH₃-4-(C₂H₅-C=NO-i-C₃H₇ |
| 369 | 2-CH₃-4-(C₂H₅-C=NO-Allyl) |
| 370 | 2-CH₃-4-(C₂H₅-C=NO-trans-Chlorallyl) |
| 371 | 2-CH₃-4-(C₂H₅-C=NO-Propargyl) |
| 372 | 2-CH₃-4-(C₂H₅-C=NO-n-C₄H₉) |
| 373 | 2-CH₃-4-(C₂H₅-C=NO-CH₂-C₆H₅) |
| 374 | 2,5-(CH₃)₂-4-(CH₃-C=NOCH₃) |
| 375 | 2,5-(CH₃)₂-4-(CH₃-C=NoC₂H₅) |
| 376 | 2,5-(CH₃)₂-4-(CH₃-C=NO-n-C₃H₇) |
| 377 | 2,5-(CH₃)₂-4-(CH₃-C=NO-i-C₃H₇) |
| 378 | 2,5-(CH₃)₂-4-(CH₃-C=NO-Allyl) |
| 379 | 2,5-(CH₃)₂-4-(CH₃-C=NO-trans-Chlorallyl) |
| 380 | 2,5-(CH₃)₂-4- (CH₃-C=NO-Proparyl) |
| 381 | 2,5-(CH₃)₂-4-(CH₃-C=NO-n-C₄H₉) |
| 382 | 2,5-(CH₃)₂-4-(CH₃-C=NO-CH₂-C₆H₅) |
| 383 | 2-C₆H₅ |
| 384 | 3-C₆H₅ |
| 385 | 4-C₆H₅ |
| 386 | 2-(2'-F-C₆H₄) |
| 387 | 2-(3'-F-C₆H₄) |
| 388 | 2- (4'-F-C6H4) |
| 389 | 3- (2'-F-C6H4) |
| 390 | 3-(3'-F-C₆H₄) |
| 391 | 3-(4'-F-C₆H₄) |
| 392 | 4-(2'-F-C₆H₄) |
| 393 | 4-(3'-F-C₆H₄) |
| 394 | 4-(4'-F-C₆H₄) |
| 395 | 2-(2'-Cl-C₆H₄) |
| 396 | 2-(3'-Cl-C₆H₄) |
| 397 | 2-(4'-Cl-C₆H₄) |
| 398 | 3-(2'-Cl-C₆H₄) |
| 399 | 3-(3'-Cl-C₆H₄) |
| 400 | 3-(4'-Cl-C₆H₄) |
| 401 | 4-(2'-Cl-C₆H₄) |
| 402 | 4-(3'-Cl-C₆H₄) |
| 403 | 4-(4'-Cl-C₆H₄) |
| 405 | 2-(2'-CH₃-C₆H₄) |
| 406 | 2-(3'-CH₃-C₆H₄) |
| 407 | 2-(4'-CH₃-C₆H₄) |
| 408 | 3-(2'-CH₃-C₆H₄) |
| 409 | 3-(3'-CH₃-C₆H₄) |
| 410 | 3-(4'-CH₃-C₆H₄) |
| 411 | 4-(2'-CH₃-C₆H₄) |
| 412 | 4-(3'-CH₃-C₆H₄) |
| 413 | 4-(4'-CH₃-C₆H₄) |
| 414 | 2-(2'-CH₃-CO-C₆H₄) |
| 415 | 2-(3'-CH₃-CO-C₆H₄) |
| 416 | 2-(4'-CH₃-CO-C₆H₄) |
| 417 | 3-(2'-CH₃-CO-C₆H₄) |
| 418 | 3-(3'-CH₃-CO-C₆H₄) |
| 419 | 3-(4'-CH₃-CO-C₆H₄) |
| 420 | 4-(2'-CH₃-CO-C₆H₄) |
| 421 | 4-(3'-CH₃-CO-C₆H₄) |
| 422 | 4-(4'-CH₃-CO-C₆H₄) |
| 423 | 2-(2'-(CH₃-C(=NOAllyl))-C₆H₄) |
| 424 | 2-(3'-(CH₃-C(=NOAllyl))-C₆H₄) |
| 425 | 2-(4'-(CH₃-C(=NoAllyl))-C₆H₄) |
| 426 | 3-(2'-(CH₃-C(=NOAllyl))-C₆H₄) |
| 427 | 3-(3'-(CH₃-C(=NOAllyl))-C₆H₄) |
| 428 | 3-(4'-(CH₃-C(=NOAllyl))-C₆H₄) |
| 429 | 4-(2'-(CH₃-C(=NOAllyl))-C₆H₄) |
| 430 | 4-(3'-(CH₃-C(=NOAllyl))-C₆H₄) |
| 431 | 4-(4'-(CH₃-C(=NOAllyl))-C₆H₄) |
| 432 | 2-(2'-CH₃O₂C-C₆H₄) |
| 433 | 2-(3'-CH₃O₂C-C₆H₄) |
| 434 | 2-(4'-CH₃O₂C-C₆H₄) |
| 435 | 3-(2'-CH₃O₂C-C₆H₄) |
| 436 | 3-(3'-CH₃O₂C-C₆H₄) |
| 437 | 3-(4'-CH₃O₂C-C₆H₄) |
| 438 | 4-(2'-CH₃O₂C-C₆H₄) |
| 439 | 4-(3'-CH₃O₂C-C₆H₄) |
| 440 | 4-(4'-CH₃O₂C-C₆H₄) |
| 441 | 2-(2'-CH₃O-C₆H₄) |
| 442 | 2-(3'-CH₃O-C₆H₄) |
| 443 | 2-(4'-CH₃O-C₆H₄) |
| 444 | 3-(2'-CH₃O-C₆H₄) |
| 445 | 3-(3'-CH₃O-C₆H₄) |
| 446 | 3-(4'-CH₃O-C₆H₄) |
| 447 | 4-(2'-CH₃O-C₆H₄) |
| 448 | 4-(3'-CH₃O-C₆H₄) |
| 449 | 4-(4'-CH₃O-C₆H₄) |
| 450 | 2-(2'-O₂N-C₆H₄) |
| 451 | 2-(3'-O₂N-C₆H₄) |
| 452 | 2-(4'-O₂N-C₆H₄) |
| 453 | 3-(2'-O₂N-C₆H₄) |
| 454 | 3-(3'-O₂N-C₆H₄) |
| 455 | 3-(4'-O₂N-C₆H₄) |
| 456 | 4-(2'-O₂N-C₆H₄) |
| 457 | 4-(3'-O₂N-C₆H₄) |
| 458 | 4-(4'-O₂N-C₆H₄) |
| 459 | 2-(2'-NC-C₆H₄) |
| 460 | 2-(3'-NC-C₆H₄) |
| 461 | 2-(4'-NC-C₆H₄) |
| 462 | 3-(2'-NC-C₆H₄) |
| 463 | 3-(3'-NC-C₆H₄) |
| 464 | 3-(4'-NC-C₆H₄) |
| 465 | 4-(2'-NC-C₆H₄) |
| 466 | 4-(3'-NC-C₆H₄) |
| 467 | 4-(4'-NC-C₆H₄) |
| 468 | 2-(2'-CF₃-C₆H₄) |
| 469 | 2-(3'-CF₃-C₆H₄) |
| 470 | 2-(4'-CF₃-C₆H₄) |
| 471 | 3-(2'-CF₃-C₆H₄) |
| 472 | 3-(3'-CF₃-C₆H₄) |
| 473 | 3-(4'-CF₃-C₆H₄) |
| 474 | 4-(2'-CF₃-C₆H₄) |
| 475 | 4-(3'-CF₃-C₆H₄) |
| 476 | 4-(4'-CF₃-C₆H₄) |
| 477 | 2-O-C₆H₅ |
| 475 | 3-O-C₆H₅ |
| 476 | 4-O-C₆H₅ |
| 478 | 2-O-(2'-F-C₆H₄) |
| 479 | 2-O-(3'-F-C₆H₄) |
| 480 | 2-O-(4'-F-C₆H₄) |
| 481 | 3-O-(2'-F-C₆H₄) |
| 482 | 3-O-(3'-F-C₆H₄) |
| 483 | 3-O-(4'-F-C₆H₄) |
| 484 | 4-O-(2'-F-C₆H₄) |
| 485 | 4-O-(3'-F-C₆H₄) |
| 486 | 4-O-(4'-F-C₆H₄) |
| 487 | 2-O-(2'-Cl-C₆H₄) |
| 488 | 2-O-(3'-Cl-C₆H₄) |
| 489 | 2-O-(4'-Cl-C₆H₄) |
| 490 | 3-O-(2'-Cl-C₆H₄) |
| 491 | 3-O-(3'-Cl-C₆H₄) |
| 492 | 3-O-(4'-Cl-C₆H₄) |
| 493 | 3-O-(4,-Cl-C₆H₄) |
| 494 | 4-O-(2'-Cl-C₆H₄) |
| 495 | 4-O-(3'-Cl-C₆H₄) |
| 496 | 4-O-(4'-Cl-C₆H₄) |
| 497 | 2-O-(2'-CH₃-C₆H₄) |
| 498 | 2-O-(3,-CH₃-C₆H₄) |
| 499 | 2-O-(4'-CH₃-C₆H₄) |
| 500 | 3-O-(2'-CH₃-C₆H₄) |
| 501 | 3-O-(3'-CH₃-C₆H₄) |
| 502 | 3-O-(4'-CH₃-C₆H₄) |
| 503 | 4-O-(2'-CH₃-C₆H₄) |
| 504 | 4-O-(3'-CH₃-C₆H₄) |
| 505 | 4-O-(4'-CH₃-C₆H₄) |
| 506 | 2-O-(2'-CH₃-CO-C₆H₄) |
| 507 | 2-O-(3'-CH₃-CO-C₆H₄) |
| 508 | 2-O-(4'-CH₃-CO-C₆H₄) |
| 509 | 3-O-(2'-CH₃-CO-C₆H₄) |
| 510 | 3-O-(3'-CH₃-CO-C₆H₄) |
| 511 | 3-O-(4'-CH₃-CO-C₆H₄) |
| 512 | 4-O-(2'-CH₃-CO-C₆H₄) |
| 513 | 4-O-(3'-CH₃-CO-C₆H₄) |
| 514 | 4-O-(4'-CH₃-CO-C₆H₄) |
| 515 | 2-O-(2'-(CH₃-C(=NOAllyl))-C₆H₄) |
| 516 | 2-O-(3'-(CH₃-C(=NOAllyl))-C₆H₄) |
| 517 | 2-O-(4'-(CH₃-C(=NOAllyl))-C₆H₄) |
| 518 | 3-O-(2'-(CH₃-C (=NOAllyl))-C₆H₄) |
| 519 | 3-O-(3'-(CH₃-C(=NOAllyl))-C₆H₄) |
| 520 | 3-O-(4'-(CH₃-C(=NOAllyl))-C₆H₄) |
| 521 | 4-O-(2'-(CH₃-C(=NOAllyl))-C₆H₄) |
| 522 | 4-O-(3'-(CH₃-C(=NOAllyl))-C₆H₄) |
| 523 | 4-O-(4'-(CH₃-C(=NOAllyl))-C₆H₄) |
| 524 | 2-O-(2'-CH₃O₂C-C₆H₄) |
| 525 | 2-O-(3'-CH₃O₂C-C₆H₄) |
| 526 | 2-O-(4'-CH₃O₂C-C₆H₄) |
| 527 | 3-O-(2'-CH₃O₂C-C₆H₄) |
| 528 | 3-O-(3'-CH₃O₂C-C₆H₄) |
| 529 | 3-O-(4'-CH₃O₂C-C₆H₄) |
| 530 | 4-O-(2'-CH₃O₂C-C₆H₄) |
| 531 | 4-O-(3'-CH₃O₂C-C₆H₄) |
| 532 | 4-O-(4'-CH₃O₂C-C₆H₄) |
| 533 | 2-O-(2'-CH₃O-C₆H₄) |
| 534 | 2-O-(3'-CH₃O-C₆H₄) |
| 535 | 2-O-(4'-CH₃O-C₆H₄) |
| 536 | 3-O-(2'-CH₃O-C₆H₄) |
| 537 | 3-O-(3'-CH₃O-C₆H₄) |
| 538 | 3-O-(4'-CH₃O-C₆H₄) |
| 539 | 4-O-(2'-CH₃O-C₆H₄) |
| 540 | 4-O-(3'-CH₃O-C₆H₄) |
| 541 | 4-O-(4'-CH₃O-C₆H₄) |
| 542 | 2-O-(2'-O₂N-C₆H₄) |
| 543 | 2-O-(3'-O₂N-C₆H₄) |
| 544 | 2-O-(4'-O₂N-C₆H₄) |
| 545 | 3-O-(2'-O₂N-C₆H₄) |
| 546 | 3-O-(3'-O₂N-C₆H₄) |
| 547 | 3-O-(4'-O₂N-C₆H₄) |
| 548 | 4-O-(2'-O₂N-C₆H₄) |
| 549 | 4-O-(3'-O₂N-C₆H₄) |
| 550 | 4-O-(4'-O₂N-C₆H₄) |
| 551 | 2-O-(2'-NC-C₆H₄) |
| 552 | 2-O-(3'-NC-C₆H₄) |
| 553 | 2-O-(4'-NC-C₆H₄) |
| 554 | 3-O-(2'-NC-C₆H₄) |
| 555 | 3-O-(3'-NC-C₆H₄) |
| 556 | 3-O-(4'-NC-C₆H₄) |
| 557 | 4-O-(2'-NC-C₆H₄) |
| 558 | 4-O-(3'-NC-C₆H₄) |
| 559 | 4-O-(4'-NC-C₆H₄) |
| 560 | 2-O-(2'-CF₃-C₆H₄) |
| 561 | 2-O-(3'-CF₃-C₆H₄) |
| 562 | 2-O-(4'-CF₃-C₆H₄) |
| 563 | 3-O-(2'-CF₃-C₆H₄) |
| 564 | 3-O-(3'-CF₃-C₆H₄) |
| 565 | 3-O-(4'-CF₃-C₆H₄) |
| 566 | 4-O-(2'-CF₃-C₆H₄) |
| 567 | 4-O-(3'-CF₃-C₆H₄) |
| 568 | 4-O-(4'-CF₃-C₆H₄) |
| 569 | 2-Pyridinyl-2' |
| 570 | 2-Pyridinyl-3' |
| 571 | 2-Pyridinyl-4' |
| 572 | 3-Pyridinyl-2' |
| 573 | 3-Pyridinyl-3' |
| 574 | 3-Pyridinyl-4' |
| 575 | 4-Pyridinyl-2' |
| 576 | 4-Pyridinyl-3' |
| 577 | 4-Pyridinyl-4' |
| 578 | 2-Pyrimidinyl-2' |
| 579 | 2-Pyrimidinyl-3' |
| 580 | 2-Pyrimidinyl-4' |
| 581 | 3-Pyrimidinyl-2' |
| 582 | 3-Pyrimidinyl-3' |
| 583 | 3-Pyrimidinyl-4' |
| 584 | 4-Pyrimidinyl-2' |
| 585 | 4-Pyrimidinyl-3' |
| 586 | 4-Pyrimidinyl-4' |
| 587 | 2-Pyrazolyl-1' |
| 588 | 2-Pyrazolyl-3' |
| 589 | 2-Pyrazolyl-4' |
| 590 | 3-Pyrazolyl-1' |
| 591 | 3-Pyrazolyl-3' |
| 592 | 3-Pyrazolyl-4' |
| 593 | 4-Pyrazolyl-1' |
| 594 | 4-Pyrazolyl-3' |
| 595 | 4-Pyrazolyl-4' |
| 596 | 2-Isoxazolyl-3' |
| 597 | 2-Isoxazolyl-4' |
| 598 | 2-Isoxazolyl-5' |
| 599 | 3-Isoxazolyl-3' |
| 600 | 3-Isoxazolyl-4' |
| 601 | 3-Isoxazolyl-5' |
| 602 | 4-Isoxazolyl-3' |
| 603 | 4-Isoxazolyl-4' |
| 604 | 4-Isoxazolyl-5' |
| 605 | 2-Isothiazolyl-3' |
| 606 | 2-Isothiazolyl-4' |
| 607 | 2-Isothiazolyl-5' |
| 608 | 3-Isothiazolyl-3' |
| 609 | 3-Isothiazolyl-4' |
| 610 | 3-lsothiazolyl-5' |
| 611 | 4-Isothiazolyl-3' |
| 612 | 4-lsothiazolyl-4' |
| 613 | 4-Isothiazolyl-5' |
| 614 | 2-Imidazolyl-1' |
| 615 | 2-Imidazolyl-2' |
| 616 | 2-Imidazolyl-4' |
| 617 | 3-Imidazolyl-1' |
| 618 | 3-Imidazolyl-2' |
| 619 | 3-Imidazolyl-4' |
| 620 | 4-Imidazolyl-1' |
| 621 | 4-Imidazolyl-2' |
| 622 | 4-Imidazolyl-4' |
| 623 | 2-Oxazolyl-2' |
| 624 | 2-Oxazolyl-4' |
| 625 | 2-Oxazolyl-5' |
| 626 | 3-Oxazolyl-2' |
| 627 | 3-Oxazolyl-4' |
| 628 | 3-Oxazolyl-5' |
| 629 | 4-Oxazolyl-2' |
| 630 | 4-Oxazolyl-4' |
| 631 | 4-Oxazolyl-5' |
| 632 | 2-Thiazolyl-2' |
| 633 | 2-Thiazolyl-4' |
| 634 | 2-Thiazolyl-5' |
| 635 | 3-Thiazolyl-2' |
| 636 | 3-Thiazolyl-4' |
| 637 | 3-Thiazolyl-5' |
| 638 | 4-Thiazolyl-2' |
| 639 | 4-Thiazolyl-4' |
| 640 | 4-Thiazolyl-5' |
| 641 | 2-CH₃-4-(CH₃-C=N-O-CH₂-CH₂-OCH₃) |
| 642 | 2-CH₃-4-(C₂H₅-C=N-O-CH₂-CH₂-OCH₃) |
| 643 | 2,5-(CH₃)₂-4-(CH₃-C=N-O-CH₂-CH₂-OCH₃) |
| 644 | 2-CH₃-4-(n-C₃H₇-C=N-OCH₃) |
| 645 | 2-CH₃-4-(n-C₃H₇-C=N-OC₂H₅) |
| 646 | 2-CH₃-4-(n-C₃H₇-C=N-O-n-C₃H₇) |
| 647 | 2-CH₃-4-(n-C₃H₇-C=N-O-i-C₃H₇) |
| 648 | 2-CH₃-4-(n-C₃H₇-C=N-O-Allyl) |
| 649 | 2-CH₃-4-(n-C₃H₇-C=N-O-trans-Chlorallyl) |
| 650 | 2-CH₃-4-(n-C₃H₇-C=N-O-Propargyl) |
| 651 | 2-CH₃-4-(n-C₃H₇-C=N-O-n-C₄H₉) |
| 652 | 2-CH₃-4-(n-C₃H₇-C=N-O-CH₂-C₆H₅) |
| 653 | 2-CH₃-4-(i-C₃H₇-C=N-OCH₃) |
| 654 | 2-CH₃-4-(i-C₃H₇-C=N-OC₂H₅) |
| 655 | 2-CH₃-4-(i-C₃H₇-C=N-O-n-C₃H₇) |
| 656 | 2-CH₃-4-(i-C₃H₇-C=N-O-i-C₃H₇) |
| 657 | 2-CH₃-4-(i-C₃H₇-C=N-O-Allyl) |
| 658 | 2-CH₃-4-(i-C₃H₇-C=N-O-trans-Chlorallyl) |
| 659 | 2-CH₃-4-(i-C₃H₇-C=N-O-Propargyl) |
| 660 | 2-CH₃-4-(i-C₃H₇-C=N-O-n-C₄H₉) |
| 661 | 2-CH₃-4-(i-C₃H₇-C=N-O-CH₂-C₆H₅) |
| 662 | 2-O-n-C₄H₉ |
| 663 | 2-O-i-C₄H₉ |
| 664 | 2-O-s-C₄H₉ |
| 665 | 2-O-t-C₄H₉ |
| 666 | 2-Neopentyloxy |
| 667 | 3-O-n-C₄H₉ |
| 668 | 3-O-i-C₄H₉ |
| 669 | 3-O-s-C₄H₉ |
| 670 | 3-O-t-C₄H₉ |
| 671 | 3-Neopentyloxy |
| 672 | 4-O-n-C₄H₉ |
| 673 | 4-O-i-C₄H₉ |
| 674 | 4-O-s-C₄H₉ |
| 675 | 4-O-t-C₄H₉ |
| 676 | 4-Neopentyloxy |
| 677 | 3-CH₃-4-OCH₃ |
| 678 | 3-CH₃-4-OC₂H₅ |
| 679 | 3-CH₃-4-O-n-C₃H₇ |
| 680 | 3-CH₃-4-O-n-C₄H₉ |
| 681 | 3-CH₃-4-O-i-C₄H₉ |
| 682 | 3-CH₃-4-O-s-C₄H₉ |
| 683 | 3-CH₃-4-O-t-C₄H₉ |
| 684 | 3-CH₃-4-Neopentyloxy |
| 685 | 2-CH₃-3-OCH₃ |
| 686 | 2-CH₃-4-OCH₃ |
| 687 | 2-CH₃-5-OCH₃ |
| 688 | 2-CH₃-6-OCH₃ |
| 689 | 3-CH₃-4-OCH₃ |
| 690 | 3-CH₃-5-OCH₃ |
| 691 | 3-CH₃-6-OCH₃ |
| 692 | 4-CH₃-5-O-CH₃ |
| 693 | 4-CH₃-6-O-CH₃ |
| 694 | 4-CH₃-6-OCH₃ |
| 695 | 2-CH₃-3-O-i-C₃H₇ |
| 696 | 2-CH₃-4-O-i-C₃H₇ |
| 697 | 2-CH₃-5-O-i-C₃H₇ |
| 698 | 2-CH₃-6-O-i-C₃H₇ |
| 699 | 3-CH₃-4-O-i-C₃H₇ |
| 700 | 3-CH₃-5-O-i-C₃H₇ |
| 701 | 3-CH₃-6-O-i-C₃H₇ |
| 702 | 4-CH₃-5-O-i-C₃H₇ |
| 703 | 4-CH₃-6-O-i-C₃H₇ |
| 704 | 5-CH₃-6-O-i-C₃H₇ |
| 705 | 2-Cl-3-OCH₃ |
| 706 | 2-Cl-4-OCH₃ |
| 707 | 2-Cl-5-OCH₃ |
| 708 | 2-Cl-6-OCH₃ |
| 709 | 3-Cl-4-OCH₃ |
| 710 | 3-Cl-5-OCH₃ |
| 711 | 3-Cl-6-OCH₃ |
| 712 | 4-Cl-5-OCH₃ |
| 713 | 4-Cl-6-OCH₃ |
| 714 | 5-Cl-6-OCH₃ |

**Tabelle B**

| Nummer | B |
|---|---|
| 1 | Pyrrolyl-3 |
| 2 | N-CH₃-Pyrrolyl-3 |
| 3 | N-C₆H₅-Pyrrolyl-3 |
| 4 | N-(4'-CH₃-C₆H₄)-Pyrrolyl-3 |
| 5 | N-(3'-CH₃-C₆H₄)-Pyrrolyl-3 |
| 6 | N-(2'-CH₃-C₆H₄)-Pyrrolyl-3 |
| 7 | N-(4'-CH₃O-C₆H₄)-Pyrrolyl-3 |
| 8 | N-(3'-CH₃O-C₆H₄)-Pyrrolyl-3 |
| 9 | N-(2'-CH₃O-C₆H₄)-Pyrrolyl-3 |
| 10 | N-(4'-NO₂-C₆H₄)-Pyrrolyl-3 |
| 11 | N-(3'-NO₂-C₆H₄)-Pyrrolyl-3 |
| 12 | N-(2'-NO₂-C₆H₄)-Pyrrolyl-3 |
| 13 | N-(4'-CN-C₆H₄)-Pyrrolyl-3 |
| 14 | N-(3'-CN-C₆H₄)-Pyrrolyl-3 |
| 15 | N-(2'-CN-C₆H₄)-Pyrrolyl-3 |
| 16 | N-(4'-Cl-C₆H₄)-Pyrrolyl-3 |
| 17 | N-(3'-Cl-C₆H₄)-Pyrrolyl-3 |
| 18 | N-(2'-Cl-C₆H₄)-Pyrrolyl-3 |
| 19 | Pyrrolyl-2 |
| 20 | N-CH₃-Pyrrolyl-2 |
| 21 | N-C₆H₅-Pyrrolyl-2 |
| 22 | N-(4'-CH₃-C₆H₄)-Pyrrolyl-2 |
| 23 | N-(3'-CH₃-C₆H₄)-Pyrrolyl-2 |
| 24 | N-(2'-CH₃-C₆H₄)-Pyrrolyl-2 |
| 25 | N-(4'-CH₃O-C₆H₄)-Pyrrolyl-2 |
| 26 | N-(3'-CH₃O-C₆H₄)-Pyrrolyl-2 |
| 27 | N-(2'-CH₃O-C₆H₄)-Pyrrolyl-2 |
| 28 | N-(4'-NO₂-C₆H₄)-Pyrrolyl-2 |
| 29 | N-(3'-NO₂-C₆H₄)-Pyrrolyl-2 |
| 30 | N-(2'-NO₂-C₆H₄)-Pyrrolyl-2 |
| 31 | N-(4'-CN-C₆H₄)-Pyrrolyl-2 |
| 32 | N-(3'-CN-C₆H₄)-Pyrrolyl-2 |
| 33 | N-(2'-CN-C₆H₄)-Pyrrolyl-2 |
| 34 | N-(4'-Cl-C₆H₄)-Pyrrolyl-2 |
| 35 | N-(3'-Cl-C₆H₄)-Pyrrolyl-2 |
| 36 | N-(2'-Cl-C₆H₄)-Pyrrolyl-2 |
| 37 | Furyl-2 |
| 38 | 5-CH₃-Furyl-2 |
| 39 | 5-C₆H₅-Furyl-2 |
| 40 | 5-(4'-CH₃-C₆H₄)-Furyl-2 |
| 41 | 5-(3'-CH₃-C₆H₄)-Furyl-2 |
| 42 | 5-(2'-CH₃-C₆H₄)-Furyl-2 |
| 43 | 5-(4'-CH₃O-C₆H₄)-Furyl-2 |
| 44 | 5-(3'-CH₃O-C₆H₄)-Furyl-2 |
| 45 | 5-(2'-CH₃O-C₆H₄)-Furyl-2 |
| 46 | 5-(4'-NO₂-C₆H₄)-Furyl-2 |
| 47 | 5-(3'-NO₂-C₆H₄)-Furyl-2 |
| 48 | 5-(2'-NO₂-C₆H₄)-Furyl-2 |
| 49 | 5-(4'-CN-C₆H₄)-Furyl-2 |
| 50 | 5-(3'-CN-C₆H₄)-Furyl-2 |
| 51 | 5-(2'-CN-C₆H₄)-Furyl-2 |
| 52 | 5-(4'-Cl-C₆H₄)-Furyl-2 |
| 53 | 5-(3'-Cl-C₆H₄)-Furyl-2 |
| 54 | 5-(2'-Cl-C₆H₄)-Furyl-2 |
| 55 | 4-CH₃-Furyl-2 |
| 56 | 4-C₆H₅-Furyl-2 |
| 57 | 4-(4'-CH₃-C₆H₄)-Furyl-2 |
| 58 | 4-(3'-CH₃-C₆H₄)-Furyl-2 |
| 59 | 4-(2'-CH₃-C₆H₄)-Furyl-2 |
| 60 | 4-(4'-CH₃O-C₆H₄)-Furyl-2 |
| 61 | 4-(3'-CH₃O-C₆H₄)-Furyl-2 |
| 62 | 4-(2'-CH₃O-C₆H₄)-Furyl-2 |
| 63 | 4-(4'-NO₂-C₆H₄)-Furyl-2 |
| 64 | 4-(3'-NO₂-C₆H₄)-Furyl-2 |
| 65 | 4-(2'-NO₂-C₆H₄)-Furyl-2 |
| 66 | 4-(4'-CN-C₆H₄)-Furyl-2 |
| 67 | 4-(3'-CN-C₆H₄)-Furyl-2 |
| 68 | 4-(2'-CN-C₆H₄)-Furyl-2 |
| 69 | 4-(4'-Cl-C₆H₄)-Furyl-2 |
| 70 | 4-(3'-Cl-C₆H₄)-Furyl-2 |
| 71 | 4-(2'-Cl-C₆H₄)-Furyl-2 |
| 72 | Thienyl-2 |
| 73 | 5-CH₃-Thienyl-2 |
| 74 | 5-C₆H₅-Thienyl-2 |
| 70 | 5-(4'-CH₃-C₆H₄)-Thienyl-2 |
| 76 | 5-(3'-CH₃-C₆H₄)-Thienyl-2 |
| 77 | 5-(2'-CH₃-C₆H₄)-Thienyl-2 |
| 78 | 5-(4'-CH₃O-C₆H₄)-Thienyl-2 |
| 79 | 5-(3'-CH₃O-C₆H₄)-Thienyl-2 |
| 80 | 5-(2'-CH₃O-C₆H₄)-Thienyl-2 |
| 81 | 5-(4'-NO₂-C₆H₄)-Thienyl-2 |
| 82 | 5-(3'-NO₂-C₆H₄)-Thienyl-2 |
| 83 | 5-(2'-NO₂-C₆H₄)-Thienyl-2 |
| 84 | 5-(4'-CN-C₆H₄)-Thienyl-2 |
| 85 | 5-(3'-CN-C₆H₄)-Thienyl-2 |
| 86 | 5-(2'-CN-C₆H₄)-Thienyl-2 |
| 87 | 5-(4'-Cl-C₆H₄)-Thienyl-2 |
| 88 | 5-(3'-Cl-C₆H₄)-Thienyl-2 |
| 89 | 5-(2'-Cl-C₆H₄)-Thienyl-2 |
| 90 | 4-CH₃-Thienyl-2 |
| 91 | 4-C₆H₅-Thienyl-2 |
| 92 | 4-(4'-CH₃-C₆H₄)-Thienyl-2 |
| 93 | 4-(3'-CH₃-C₆H₄)-Thienyl-2 |
| 94 | 4-(2'-CH3-C₆H₄)-Thienyl-2 |
| 95 | 4-(4'-CH₃O-C₆H₄)-Thienyl-2 |
| 96 | 4-(3'-CH₃O-C₆H₄)-Thienyl-2 |
| 97 | 4-(2'-CH₃O-C₆H₄)-Thienyl-2 |
| 98 | 4-(4'-NO₂-C₆H₄)-Thienyl-2 |
| 99 | 4-(3'-NO₂-C₆H₄)-Thienyl-2 |
| 100 | 4-(2'-NO₂-C₆H₄)-Thienyl-2 |
| 101 | 4-(4'-CN-C₆H₄)-Thienyl-2 |
| 102 | 4-(3'-CN-C₆H₄)-Thienyl-2 |
| 103 | 4-(2'-CN-C₆H₄)-Thienyl-2 |
| 104 | 4-(4'-Cl-C₆H₄)-Thienyl-2 |
| 105 | 4-(3'-Cl-C₆H₄)-Thienyl-2 |
| 106 | 4-(2'-Cl-C₆H₄)-Thienyl-2 |
| 107 | Thienyl-3 |
| 108 | 5-CH₃-Thienyl-3 |
| 109 | 5-C₆H₅-Thienyl-3 |
| 110 | 5-(4'-CH₃-C₆H₄)-Thienyl-3 |
| 111 | 5-(3'-CH₃-C₆H₄)-Thienyl-3 |
| 112 | 5-(2'-CH₃-C₆H₄)-Thienyl-3 |
| 113 | 5-(4'-CH₃O-C₆H₄)-Thienyl-3 |
| 114 | 5-(3'-CH₃O-C₆H₄)-Thienyl-3 |
| 115 | 5-(2'-CH₃O-C₆H₄)-Thienyl-3 |
| 116 | 5-(4'-NO₂-C₆H₄)-Thienyl-3 |
| 117 | 5-(3'-NO₂-C₆H₄)-Thienyl-3 |
| 118 | 5-(2'-NO₂-C₆H₄)-Thienyl-3 |
| 119 | 5-(4'-CN-C₆H₄)-Thienyl-3 |
| 120 | 5-(3'-CN-C₆H₄)-Thienyl-3 |
| 121 | 5-(2'-CN-C₆H₄)-Thienyl-3 |
| 122 | 5-(4'-Cl-C₆H₄)-Thienyl-3 |
| 123 | 5-(3'-Cl-C₆H₄)-Thienyl-3 |
| 124 | 5-(2'-Cl-C₆H₄)-Thienyl-3 |
| 125 | Pyrazolyl-4 |
| 126 | N-CH₃-Pyrazolyl-4 |
| 127 | N-C₆H₅-Pyrazolyl-4 |
| 128 | N-(4'-CH₃-C₆H₄)-Pyrazolyl-4 |
| 129 | N-(3'-CH₃-C₆H₄)-Pyrazolyl-4 |
| 130 | N-(2'-CH₃-C₆H₄)-Pyrazolyl-4 |
| 131 | N-(4'-CH₃O-C₆H₄)-Pyrazolyl-4 |
| 132 | N-(3'-CH₃O-C₆H₄)-Pyrazolyl-4 |
| 133 | N-(2'-CH₃O-C₆H₄)-Pyrazolyl-4 |
| 134 | N-(4'-NO₂-C₆H₄)-Pyrazolyl-4 |
| 135 | N-(3'-NO₂-C₆H₄)-Pyrazolyl-4 |
| 136 | N-(2'-NO₂-C₆H₄)-Pyrazolyl-4 |
| 137 | N-(4'-CN-C₆H₄)-Pyrazolyl-4 |
| 138 | N-(3'-CN-C₆H₄)-Pyrazolyl-4 |
| 139 | N-(2'-CN-C₆H₄)-Pyrazolyl-4 |
| 140 | N-(4'-Cl-C₆H₄)-Pyrazolyl-4 |
| 141 | N-(3'-Cl-C₆H₄)-Pyrazolyl-4 |
| 142 | N-(2'-Cl-C₆H₄)-Pyrazolyl-4 |
| 143 | 3-CH₃-N-Methylpyrazolyl-4 |
| 144 | 3-C₆H₅-N-Methylpyrazolyl-4 |
| 145 | 3-(4'-CH₃-C₆H₄)-N-Methylpyrazolyl-4 |
| 146 | 3-(3'-CH₃-C₆H₄)-N-Methylpyrazolyl-4 |
| 147 | 3-(2'-CH₃-C₆H₄)-N-Methylpyrazolyl-4 |
| 148 | 3-(4'-CH₃O-C₆H₄)-N-Methylpyrazolyl-4 |
| 149 | 3-(3'-CH₃O-C₆H₄)-N-Methylpyrazolyl-4 |
| 150 | 3-(2'-CH₃O-C₆H₄)-N-Methylpyrazolyl-4 |
| 151 | 3-(4'-NO₂-C₆H₄)-N-Methylpyrazolyl-4 |
| 152 | 3-(3'-NO₂-C₆H₄)-N-Methylpyrazolyl-4 |
| 153 | 3-(2'-NO₂-C₆H₄)-N-Methylpyrazolyl-4 |
| 154 | 3-(4'-CN-C₆H₄)-N-Methylpyrazolyl-4 |
| 155 | 3-(3'-CN-C₆H₄)-N-Methylpyrazolyl-4 |
| 156 | 3-(2'-CN-C₆H₄)-N-Methylpyrazolyl-4 |
| 157 | 3-(4'-Cl-C₆H₄)-N-Methylpyrazolyl-4 |
| 158 | 3-(3'-Cl-C₆H₄)-N-Methylpyrazolyl-4 |
| 159 | 3-(2'-Cl-C₆H₄)-N-Methylpyrazolyl-4 |
| 160 | Isoxazolyl-5 |
| 161 | 3-CH₃-Isoxazolyl-5 |
| 162 | 3-C₆H₅-Isoxazolyl-5 |
| 163 | 3-(4'-CH₃-C₆H₄)-Isoxazolyl-5 |
| 164 | 3-(3'-CH₃-C₆H₄)-Isoxazolyl-5 |
| 165 | 3-(2'-CH₃-C₆H₄)-Isoxazolyl-5 |
| 166 | 3-(4'-CH₃O-C₆H₄)-Isoxazolyl-5 |
| 167 | 3-(3'-CH₃O-C₆H₄)-Isoxazolyl-5 |
| 168 | 3-(2'-CH₃O-C₆H₄)-Isoxazolyl-5 |
| 169 | 3-(4'-NO₂-C₆H₄)-Isoxazolyl-5 |
| 170 | 3-(3'-NO₂-C₆H₄)-Isoxazolyl-5 |
| 171 | 3-(2'-NO₂-C₆H₄)-Isoxazolyl-5 |
| 172 | 3-(4'-CN-C₆H₄)-Isoxazolyl-5 |
| 173 | 3-(3'-CN-C₆H₄)-Isoxazolyl-5 |
| 174 | 3-(2'-CN-C₆H₄)-Isoxazolyl-5 |
| 175 | 3-(4'-Cl-C₆H₄)-Isoxazolyl-5 |
| 176 | 3-(3'-Cl-C₆H₄)-Isoxazolyl-5 |
| 177 | 3-(2'-Cl-C₆H₄)-Isoxazolyl-5 |
| 178 | 4-Chlorisoxazolyl-5 |
| 179 | 3-CH₃-4-Chlorisoxazolyl-5 |
| 180 | 3-C₆H₅-4-Chlorisoxazolyl-5 |
| 181 | 3-(4'-CH₃-C₆H₄)-4-Chlorisoxazolyl-5 |
| 182 | 3-(3'-CH₃-C₆H₄)-4-Chlorisoxazolyl-5 |
| 183 | 3-(2'-CH₃-C₆H₄)-4-Chlorisoxazolyl-5 |
| 184 | 3-(4'-CH₃O-C₆H₄)-4-Chlorisoxazolyl-5 |
| 185 | 3-(3'-CH₃O-C₆H₄)-4-Chlorisoxazolyl-5 |
| 186 | 3-(2'-CH₃O-C₆H₄)-4-Chlorisoxazolyl-5 |
| 187 | 3-(4'-NO₂-C₆H₄)-4-Chlorisoxazolyl-5 |
| 188 | 3-(3'-NO₂-C₆H₄)-4-Chlorisoxazolyl-5 |
| 189 | 3-(2'-NO₂-C₆H₄)-4-Chlorisoxazolyl-5 |
| 190 | 3-(4'-CN-C₆H₄)-4-Chlorisoxazolyl-5 |
| 191 | 3-(3'-CN-C₆H₄)-4-Chlorisoxazolyl-5 |
| 192 | 3-(2-CN-C₆H₄)-4-Chlorisoxazolyl-5 |
| 193 | 3-(4'-Cl-C₆H₄)-4-Chlorisoxazolyl-5 |
| 194 | 3-(3'-Cl-C₆H₄)-4-Chlorisoxazolyl-5 |
| 195 | 3-(2'-Cl-C₆H₄)-4-Chlorisoxazolyl-5 |
| 196 | Isoxazolyl-3 |
| 197 | 5-CH₃-Isoxazolyl-3 |
| 198 | 5-C₆H₅-Isoxazolyl-3 |
| 199 | 5-(4'-CH₃-C₆H₄)-Isoxazolyl-3 |
| 200 | 5-(3'-CH₃-C₆H₄)-Isoxazolyl-3 |
| 201 | 5-(2'-CH₃-C₆H₄)-Isoxazolyl-3 |
| 202 | 5-(4'-CH₃O-C₆H₄)-Isoxazolyl-3 |
| 203 | 5-(3'-CH₃O-C₆H₄)-Isoxazolyl-3 |
| 204 | 5-(2'-CH₃O-C₆H₄)-Isoxazolyl-3 |
| 205 | 5-(4'-NO₂-C₆H₄)-Isoxazolyl-3 |
| 206 | 5-(3'-NO₂-C₆H₄)-Isoxazolyl-3 |
| 207 | 5-(2'-NO₂-C₆H₄)-Isoxazolyl-3 |
| 208 | 5-(4'-CN-C₆H₄)-Isoxazolyl-3 |
| 209 | 5-(3'-CN-C₆H₄)-Isoxazolyl-3 |
| 210 | 5-(2'-CN-C₆H₄)-Isoxazolyl-3 |
| 211 | 5-(4'-Cl-C₆H₄)-Isoxazolyl-3 |
| 212 | 5-(3'-Cl-C₆H₄)-Isoxazolyl-3 |
| 213 | 5-(2'-Cl-C₆H₄)-Isoxazolyl-3 |
| 214 | Isothiazolyl-5 |
| 215 | 3-CH₃-Isothiazolyl-5 |
| 216 | 3-C₆H₅-Isothiazolyl-5 |
| 217 | 3-(4'-CH₃-C₆H₄)-Isothiazolyl-5 |
| 218 | 3-(3'-CH₃-C₆H₄)-Isothiazolyl-5 |
| 219 | 3-(2'-CH₃-C₆H₄)-Isothiazolyl-5 |
| 220 | 3-(4'-CH₃O-C₆H₄)-Isothiazolyl-5 |
| 221 | 3-(3'-CH₃O-C₆H₄)-Isothiazolyl-5 |
| 222 | 3-(2'-CH₃O-C₆H₄)-Isothiazolyl-5 |
| 223 | 3-(4'-NO₂-C₆H₄)-Isothiazolyl-5 |
| 224 | 3-(3'-NO₂-C₆H₄)-Isothiazolyl-5 |
| 225 | 3-(2'-NO₂-C₆H₄)-Isothiazolyl-5 |
| 226 | 3-(4'-CN-C₆H₄)-Isothiazolyl-5 |
| 227 | 3-(3'-CN-C₆H₄)-Isothiazolyl-5 |
| 228 | 3-(2'-CN-C₆H₄)-Isothiazolyl-5 |
| 229 | 3-(4'-Cl-C₆H₄)-Isothiazolyl-5 |
| 230 | 3-(3'-Cl-C₆H₄)-Isothiazolyl-5 |
| 231 | 3-(2'-Cl-C₆H₄)-Isothiazolyl-5 |
| 232 | Oxazolyl-4 |
| 233 | 2-CH₃-Oxazolyl-4 |
| 234 | 2-C₆H₅-Oxazolyl-4 |
| 235 | 2-(4'-CH₃-C₆H₄-Oxazolyl-4 |
| 236 | 2-(3'-CH₃-C₆H₄)-Oxazolyl-4 |
| 237 | 2-(2'-CH₃-C₆H₄)-Oxazolyl-4 |
| 238 | 2-(4'-CH₃O-C₆H₄)-Oxazolyl-4 |
| 239 | 2-(3'-CH₃O-C₆H₄)-Oxazolyl-4 |
| 240 | 2-(2'-CH₃O-C₆H₄)-Oxazolyl-4 |
| 241 | 2-(4'-NO₂-C₆H₄)-Oxazolyl-4 |
| 242 | 2-(3'-NO₂-C₆H₄)-Oxazolyl-4 |
| 243 | 2-(2'-NO₂-C₆H₄)-Oxazolyl-4 |
| 244 | 2-(4'-CN-C₆H₄)-Oxazolyl-4 |
| 245 | 2-(3'-CN-C₆H₄)-Oxazolyl-4 |
| 246 | 2-(2'-CN-C₆H₄)-Oxazolyl-4 |
| 247 | 2-(4'-Cl-C₆H₄)-Oxazolyl-4 |
| 248 | 2-(3'-Cl-C₆H₄)-Oxazolyl-4 |
| 249 | 2-(2'-Cl-C₆H₄)-Oxazolyl-4 |
| 250 | Thiazolyl-4 |
| 251 | 2-CH₃-Thiazolyl-4 |
| 252 | 2-C₆H₅-Thiazolyl-4 |
| 253 | 2-(4'-CH₃-C₆H₄)-Thiazolyl-4 |
| 254 | 2-(3'-CH₃-C₆H₄)-Thiazolyl-4 |
| 255 | 2-(2'-CH₃-C₆H₄)-Thiazolyl-4 |
| 256 | 2-(4'-CH₃O-C₆H₄)-Thiazolyl-4 |
| 267 | 2-(3'-CH₃O-C₆H₄)-Thiazolyl-4 |
| 258 | 2-(2'-CH₃O-C₆H₄)-Thiazolyl-4 |
| 259 | 2-(4'-NO₂-C₆H₄)-Thiazolyl-4 |
| 260 | 2-(3'-NO₂-C₆H₄)-Thiazolyl-4 |
| 261 | 2-(2'-NO₂-C₆H₄)-Thiazolyl-4 |
| 262 | 2-(4'-CN-C₆H₄)-Thiazolyl-4 |
| 263 | 2-(3'-CN-C₆H₄)-Thiazolyl-4 |
| 264 | 2-(2'-CN-C₆H₄)-Thiazolyl-4 |
| 265 | 2-(4'-Cl-C₆H₄)-Thiazolyl-4 |
| 266 | 2-(3'-Cl-C₆H₄)-Thiazolyl-4 |
| 267 | 2-(2'-Cl-C₆H₄)-Thiazolyl-4 |
| 268 | N-CH₃-1,2,4-Triazolyl-5 |
| 269 | 3-CH₃-N-CH₃-1,2,4-Triazolyl-5 |
| 270 | 3-C₆H₅-N-CH₃-1,2,4-Triazolyl-5 |
| 271 | 3-(4'-CH₃-C₆H₄)-N-CH₃-1,2,4-Triazolyl-5 |
| 272 | 3-(3'-CH₃-C₆H₄)-N-CH₃-1,2,4-Triazolyl-5 |
| 273 | 3-(2'-CH₃-C₆H₄)-N-CH₃-1,2,4-Triazolyl-5 |
| 274 | 3-(4'-CH₃O-C₆H₄)-N-CH₃-1,2,4-Triazolyl-5 |
| 275 | 3-(3'-CH₃O-C₆H₄)-N-CH₃-1,2,4-Triazolyl-5 |
| 276 | 3-(2'-CH₃O-C₆H₄)-N-CH₃-1,2,4-Triazolyl-5 |
| 277 | 3-(4'-NO₂-C₆H₄)-N-CH₃-12,4-Triazolyl-5 |
| 278 | 3-(3'-NO₂-C₆H₄)-N-CH₃-1,2,4-Triazolyl-5 |
| 279 | 3-(2'-NO₂-C₆H₄)-N-CH₃-1,2,4-Triazolyl-5 |
| 280 | 3-(4'-CN-C₆H₄)-N-CH₃-1,2,4-Triazolyl-5 |
| 281 | 3-(3'-CN-C₆H₄)-N-CH₃-1,2,4-Triazolyl-5 |
| 282 | 3-(2'-CN-C₆H₄)-N-CH₃-1,2,4-Triazolyl-5 |
| 283 | 3-(4'-Cl-C₆H₄)-N-CH₃-1,2,4-Triazolyl-5 |
| 284 | 3-(3'-Cl-C₆H₄)-N-CH₃-1,2,4-Triazolyl-5 |
| 285 | 3-(2'-Cl-C₆H₄)-N-CH₃-1,2,4-Triazolyl-5 |
| 286 | 1,3,4-Oxadiazolyl-2 |
| 287 | 5-CH₃-1,3,4-Oxadiazolyl-2 |
| 288 | 5-C₆H₅-1,3,4-Oxadiazolyl-2 |
| 289 | 5-(4'-CH₃-C₆H₄)-1,3,4-Oxadiazotyl-2 |
| 290 | 5-(3'-CH₃-C₆H₄)-1,3,4-Oxadiazolyl-2 |
| 291 | 5-(2'-CH₃-C₆H₄)-1,3,4-Oxadiazolyl-2 |
| 292 | 5-(4'-CH₃O-C₆H₄)-1,3,4-Oxadiazolyl-2 |
| 293 | 5-(3'-CH₃O-C₆H₄)-1,3,4-Oxadiazolyl-2 |
| 294 | 5-(2'-CH₃O-C₆H₄)-1,3,4-Oxadiazolyl-2 |
| 295 | 5-(4'-NO₂-C₆H₄)-1,3,4-Oxadiazolyl-2 |
| 296 | 5-(3'-NO₂-C₆H₄)-1,3,4-Oxadiazolyl-2 |
| 297 | 5-(2'-NO₂-C₆H₄)-1,3,4-Oxadiazolyl-2 |
| 298 | 5-(4'-CN-C₆H₄)-1,3,4-Oxadiazolyl-2 |
| 299 | 5-(3'-CN-C₆H₄)-1,3,4-Oxadiazotyl-2 |
| 300 | 5-(2'-CN-C₆H₄)-1,3,4-Oxadiazolyl-2 |
| 301 | 5-(4'-Cl-C₆H₄)-1,3,4-Oxadiazolyl-2 |
| 302 | 5-(3'-Cl-C₆H₄)-1,3,4-Oxadiazolyl-2 |
| 303 | 5-(2'-Cl-C₆H₄)-1,3,4-Oxadiazolyl-2 |
| 304 | 1,2,4-Oxadiazolyl-3 |
| 305 | 5-CH₃-1,2,4-Oxadiazolyl-3 |
| 306 | 5-C₆H₅-1,2,4-Oxadiazolyl-3 |
| 307 | 5-(4'-CH₃-C₆H₄)-1,2,4-Oxadiazolyl-3 |
| 308 | 5-(3'-CH₃-C₆H₄)-1,2,4-Oxadiazolyl-3 |
| 309 | 5-(2'-CH₃-C₆H₄)-1,2,4-Oxadiazolyl-3 |
| 310 | 5-(4'-CH₃O-C₆H₄)-1,2,4-Oxadiazolyl-3 |
| 311 | 5-(3'-CH₃O-C₆H₄)-1,2,4-Oxadiazolyl-3 |
| 312 | 5-(2'-CH₃O-C₆H₄)-1,2,4-Oxadiazolyl-3 |
| 313 | 5-(4'-NO₂-C₆H₄)-1,2,4-Oxadiazolyl-3 |
| 314 | 5-(3'-NO₂-C₆H₄)-1,2,4-Oxadiazolyl-3 |
| 315 | 5-(2'-NO₂-C₆H₄)-1,2,4-Oxadiazolyl-3 |
| 316 | 5-(4'-CN-C₆H₄)-1,2,4-Cxadiazolyl-3 |
| 317 | 5-(3'-CN-C₆H₄)-1,2,4-Oxadiazolyl-3 |
| 318 | 5-(2'-CN-C₆H₄)-1,2,4-Oxadiazolyl-3 |
| 319 | 5-(4'-Cl-C₆H₄)-1,2,4-Oxadiazolyl-3 |
| 320 | 5-(3'-Cl-C₆H₄)-1,2,4-Oxadiazolyl-3 |
| 321 | 5-(2'-Cl-C₆H₄)-1,2,4-Oxadiazolyl-3 |
| 322 | 1,2,4-Oxadiazolyl-5 |
| 323 | 3-CH₃-12,4-Oxadiazolyl-5 |
| 324 | 3-C₆H₅-1,2,4-Oxadiazolyl-5 |
| 325 | 3-(4'-CH₃-C₆H₄)-1,2,4-Oxadiazolyl-5 |
| 326 | 3-(3'-CH₃-C₆H₄)-1,2,4-Oxadiazolyl-5 |
| 327 | 3-(2'-CH₃-C₆H₄)-1,2,4-Oxadiazolyl-5 |
| 328 | 3-(4'-CH₃O-C₆H₄)-1,2,4-Oxadiazolyl-5 |
| 329 | 3-(3'-CH₃O-C₆H₄)-1,2,4-Oxadiazolyl-5 |
| 330 | 3-(2'-CH₃O-C₆H₄)-1,2,4-Oxadiazolyl-5 |
| 331 | 3-(4'-NO₂-C₆H₄)-1,2,4-Oxadiazolyl-5 |
| 332 | 3-(3'-NO₂-C₆H₄)-1,2,4-Oxadiazolyl-5 |
| 333 | 3-(2'-NO₂-C₆H₄)-1,2,4-Oxadiazolyl-5 |
| 334 | 3-(4'-CN-C₆H₄)-1,2,4-Oxadiazolyl-5 |
| 335 | 3-(3'-CN-C₆H₄)-1,2,4-Oxadiazolyl-5 |
| 336 | 3-(2'-CN-C₆H₄)-1,2,4-Oxadiazolyl-5 |
| 337 | 3-(4'-Cl-C₆H₄)-1,2,4-Oxadiazolyl-5 |
| 338 | 3-(3'-Cl-C₆H₄)-1,2,4-Oxadiazolyl-5 |
| 339 | 3-(2'-Cl-C₆H₄)-1,2,4-Oxadiazolyl-5 |
| 340 | 1,2,4-Thiadiazolyl-3 |
| 341 | 5-CH₃-1,2,4-Thiadiazolyl-3 |
| 342 | 5-C₆H₅-1,2,4-Thiadiazolyl-3 |
| 343 | 5-(4'-CH₃-C₆H₄)-1,2,4-Thiadiazolyl-3 |
| 344 | 5-(3'-CH₃-C₆H₄)-1,2,4-Thiadiazolyl-3 |
| 345 | 5-(2'-CH₃-C₆H₄)-1,2,4-Thiadiazolyl-3 |
| 346 | 5-(4'-CH₃O-C₆H₄)-1,2,4-Thiadiazolyl-3 |
| 347 | 5-(3'-CH₃O-C₆H₄)-1,2,4-Thiadiazolyl-3 |
| 348 | 5-(2'-CH₃O-C₆H₄)-1,2,4-Thiadiazolyl-3 |
| 349 | 5-(4'-NO₂-C₆H₄)-1,2,4-Thiadiazolyl-3 |
| 350 | 5-(3'-NO₂-C₆H₄)-1,2,4-Thiadiazolyl-3 |
| 351 | 5-(2'-NO₂-C₆H₄)-1,2,4-Thiadiazolyl-3 |
| 352 | 5-(4'-CN-C₆H₄)-1,2,4-Thiadiazolyl-3 |
| 353 | 5-(3'-CN-C₆H₄)-1,2,4-Thiadiazolyl-3 |
| 354 | 5-(2'-CN-C₆H₄)-1,2,4-Thiadiazolyl-3 |
| 355 | 5-(4'-Cl-C₆H₄)-1,2,4-Thiadiazolyl-3 |
| 356 | 5-(3'-Cl-C₆H₄)-1,2,4-Thiadiazolyl-3 |
| 357 | 5-(2'-Cl-C₆H₄)-1,2,4-Thiadiazolyl-3 |
| 358 | 1,3,4-Thiadiazolyl-2 |
| 359 | 5-CH₃-1,3,4-Thiadiazolyl-2 |
| 360 | 5-C₆H₅-1,3,4-Thiadiazolyl-2 |
| 361 | 5-(4'-CH₃-C₆H₄)-1,3,4-Thiadiazolyl-2 |
| 362 | 5-(3'-CH₃-C₆H₄)-1,3,4-Thiadiazolyl-2 |
| 363 | 5-(2'-CH₃-C₆H₄)-1,3,4-Thiadiazolyl-2 |
| 364 | 5-(4'-CH₃O-C₆H₄)-1,3,4-Thiadiazolyl-2 |
| 365 | 5-(3'-CH₃O-C₆H₄)-1,3,4-Thiadiazolyl-2 |
| 366 | 5-(2'-CH₃O-C₆H₄)-1,3,4-Thiadiazolyl-2 |
| 367 | 5-(4'-NO₂-C₆H₄)-1,3,4-Thiadiazolyl-2 |
| 368 | 5-(3'-NO₂-C₆H₄)-1,3,4-Thiadiazolyl-2 |
| 369 | 5-(2'-NO₂-C₆H₄)-1,3,4-Thiadiazolyl-2 |
| 370 | 5-(4'-CN-C₆H₄)-1,3,4-Thiadiazolyl-2 |
| 371 | 5-(3'-CN-C₆H₄)-1,3,4-Thiadiazolyl-2 |
| 372 | 5-(2'-CN-C₆H₄)-1,3,4-Thiadiazolyl-2 |
| 373 | 5-(4'-Cl-C₆H₄)-1,3,4-Thiadiazolyl-2 |
| 374 | 5-(3'-Cl-C₆H₄)-1,3,4-Thiadiazolyl-2 |
| 375 | 5-(2'-Cl-C₆H₄)-1,3,4-Thiadiazolyl-2 |
| 376 | Pyridinyl-2 |
| 377 | Pyridinyl-4 |
| 378 | Pyridazinyl-3 |
| 379 | Pyridazinyl-4 |
| 380 | Pyridazinyl-2 |
| 381 | Pyrimidinyl-4 |
| 382 | Pyrimidinyl-5 |
| 383 | Pyrimidinyl-2 |
| 384 | Pyridinyl-3 |
| 385 | 1-Naphthyl |
| 386 | 2-Naphthyl |

**Tabelle C**

| Nummer | B |
|---|---|
| 1 | 2-Pyridyl |
| 2 | 3-Trifluormethyl-2-pyridyl |
| 3 | 5-Trifluormethyl-2-pyridyl |
| 4 | 3,5-Bis-(trifluormethyl)-2-pyridyl |
| 5 | 3,5-Dichlor-2-pyridyl |
| 6 | 3-Chlor-5-trifluormethyl-2-pyridyl |
| 7 | 3,5-Dichlor-2-pyridyl |
| 8 | 2-Chloro-4-trifluormethylphenyl |
| 9 | 2-Benzothiazolyl |
| 10 | 5-Chloro-4-methyl-2-benzimidazolyl |
| 11 | 2-Benzoxazolyl |
| 12 | 1-Methyl-5-trifluoromethylimidazo[5,4-a]-pyridin-2-yl |
| 13 | 5-Chloro-2-pyrimidinyl |
| 14 | 4-Methyl-5-phenyl-2-thiazolin-2-yl |
| 15 | 4-Methyl-5-phenyl-2-oxazolin-2-yl |
| 16 | 7-Trifluoromethyl-4-chinolinyl |

Die neuen Verbindungen eignen sich als Fungizide.

Die erfindungsgemäßen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwekken; sie sollten in jedem Fall möglichst die feinste Verteilung der erfindungsgemäßen Wirkstoffe gewährleisten. Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z. B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Iso- tridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Li- gnin-Sulfitablaugen oder Methylcellulose in Betracht.

Pulver-, Streu- und Stäubemittel können durch Mischen oder gemeinsames Vermahlen der wirksamen Substanzen. mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holzund Nußschalenmehl, Cellulosepulver oder andere feste Trägerstoffe.

Beispiele für solche Zubereitungen sind:
I. eine Lösung aus 90 Gew.-Teilen einer erfindungsgemäßen Verbindung und 10 Gew.-Teilen N-Methyl-a-pyrrolidon, die zur Anwendung in Form kleinster Tropfen geeignet ist;
II. eine Mischung aus 20 Gew.-Teilen einer erfindungsgemäßen Verbindung, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 20 Gew.-Teilen einer erfindungsgemäßen Verbindung, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen einer erfindungsgemäßen Verbindung, 25 Gew.-Teilen Cyclohexanol, 65 Gew.-Teilen einer Mineralölfraktion vom Siedepunkt 210 bis 280'C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen einer erfindungsgemäßen Verbindung, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung, 10 Gew.-Teilen des Natriumsalzes eines Phenolsulfonsäüre-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten, Fusarium- und Verticillium-Arten an verschiedenen Pflanzen,
Plasmopara viticola an Reben,
Altemaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

### Anwendungsbeispiele

Als Vergleichswirkstoffe wurden N-Phenylcarbaminsäure-isopropylester (A) - bekannt aus GB 574 995 -, N-3-Chlorphenylcarbaminsäure-isopropylester (B) - bekannt aus GB 574 995-und N-3,4-Dichlorphenylcarbaminsäuremethylester (C) - bekannt aus BE 612 550 - benutzt.

### Anwendungsbeispiele

### Anwendunggsbeispiel 1

### Wirksamkeit gegen Weizenmehltau

Blätter von in Töpfen gewachsenen Weizenkeimlingen der Sorte "Frühgold" wurden mit wäßriger Spritzbrühe, die 80 % Wirkstoff und 20 % Emulgiermittel in der Trockensubstanz enthielten, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung ermittelt.

Das Ergebnis zeigt, daß der Wirkstoff aus Tabelle 1, Nr. 89 bei der Anwendung als 250 ppm Wirkstoff enthaltenden Spritzbrühe eine bessere fungizide Wirkung zeigen (95 %) als die bekannten Vergleichswirkstoffe A (45 %), B (45 %) und C (45 %).

### Anwendungsbeispiel 2

### Wirksamkeit gegen Pyricularia oryzae (protektiv)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 bis 24°C und 95 bis 99 % relativer Luftfeuchtigkeit aufgestellt. Nach 6 Tagen wurde das Ausmaß des Krankheitsbefalls ermittelt.

Das Ergebnis zeigt, daß die Wirkstoffe aus Tabelle 1, Nr. 83 und 89 bei der Anwendung als 250 ppm Wirkstoff enthaltenden Spritzbrühe eine bessere fungizide Wirkung zeigen (95 %) als die bekannten Vergleichswirkstoffe A (30 %), B (30 %) und C (30 %).

### Anwendungsbeispiel 3

### Wirksamkeit gegen Weizenmehltau

Blätter von in Töpfen gewachsenen Weizenkeimlingen der Sorte "Frühgold" wurden mit wäßriger Spritzbrühe, die 80 % Wirkstoff und 20 % Emulgiermittel in der Trockensubstanz enthielten, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung ermittelt.

Das Ergebnis zeigt, daß der Wirkstoff aus Tabelle 6, Nr. 3 und 4, Tabelle 2, Nr. 3, 7, 8, 9, 10, 11, 13, 14, 15, 16, 17, 18, 20, 23, aus Tabelle 3, Nr. 1, 2, 3, 4, 5, 6, 7, 8, 9, 10, 11, 12, 13, 14, 15, 16, 17, 17, 19, aus Tabelle 4, Nr. 1, 2, 3, 4, 5, 6, 7, 8, 9, 10, 11, 12, 13, 14, 15, 16, 17 bei der Anwendung als 250 ppm Wirkstoff enthaltende Spritzbrühe eine bessere fungizide Wirkung zeigt (100 %) als die bekannten Vergleichswirkstoffe A, B und C (15 %).

### Anwendungsbeispiel 4

### Wirksamkeit gegen Pyricularia oryzae (protektiv)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 bis 24°C und 95 bis 99 % relativer Luftfeuchtigkeit aufgestellt. Nach 6 Tagen wurde das Ausmaß des Krankheitsbefalls ermittelt.

Das Ergebnis zeigt, daß der Wirkstoff aus Tabelle 2 Nr. 3, 7, 8, 9, 10, 11, 13, 14, 15, 16, 17, 18, 20, 21, 22, 24, aus Tabelle 3, Nr. 1, 2, 3, 4, 5, 6, 7, 8, 9, 10, 11, 12, 13, 14, 15, 16, 17, 18, 19, 20, aus Tabelle 4, Nr. 1, 2, 3, 4, 5, 6, 7, 8, 9, 10, 11, 12, 13, 14, 15, 16, 17, aus Tabelle 6, Nr. 3 und 4, bei der Anwendung als 250 ppm Wirkstoff enthaltende Spritzbrühe eine bessere fungizide Wirkung zeigt (100 %) als die bekannten Vergleichswirkstoffe A, B und C (0 %).

### Anwendungsbeispiel 5

### Wirksamkeit gegen Botrytis cinerea

Paprikasämlinge der Sorte "Neusiedler Ideal Elite" wurden, nachdem sich 4 - 5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 - 24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedecken.

| Wirkstoff | %-Befall der Blätter nach Applikation von 500 ppm-haltiger wäßriger Wirkstoffaufbereitung |
|---|---|
| Tabelle 8, Wirkstoff Nr. 2 | 5 |
| Vergleichssubstanz A | 100 |
| Vergleichssubstanz B | 100 |
| Vergleichssubstanz C | 100 |
| Unbehandelt | 100 |

### Anwendungsbeispiel 6

### Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruchs auf den Blattunterseiten.

| Wirkstoff | %-Befall der Blätter nach Applikation von 500 ppm-haltiger wäßriger Wirkstoffaufbereitung |
|---|---|
| Tabelle 6, Wirkstoff Nr. 3 | 5 |
| Tabelle 6, Wirstoff Nr. 4 | 0 |
| Tabelle 8, Wirkstoff Nr. 1 | 15 |
| Vergleichssubstanz A | 65 |
| Vergleichssubstanz B | 40 |
| Vergleichssubstanz C | 25 |
| Unbehandelt | 65 |

## Patentansprüche

1. Carbamate der Formel I 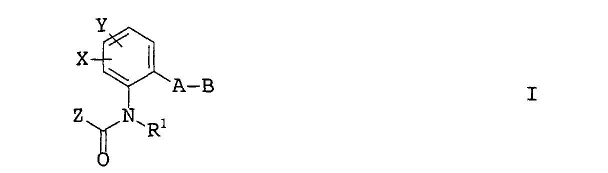 in der die Substituenten die folgende Bedeutung haben:
Z Methoxy, Amino, Methylamino, Dimethylamino, Methyl, Ethyl, Trifluormethyl oder Trichlormethyl;
X, Y Wasserstoff, Fluor, Chlor, Brom, Trifluormethyl, Cyano, Nitro, C₁-C₆-Alkyl, C₂-C₆-Alkenyl, C₂-C₆-Alkinyl, C₁-C₆-Alkoxy, C₂-C₆-Alkenyloxy, C₂-C₆-Alkinyloxy, oder
X,Y sind gemeinsam zu einem ggf. substituierten aromatischen oder heteroaromatischen, alicyclischen oder heterocyclischen, partiell oder vollständig hydrierten Ring kondensiert;
A eine direkte Bindung, O, S, CR²=CR³, CHR²O, CHR²S, CHR²-ON=CR⁴, CR²=NO, ON=CR⁴, C≡C, CHR²-CHR³, CHR²O-CO oder OCHR²;
R²,R³ Wasserstoff, C₁-C₆-Alkyl, C₂-C₆-Alkenyl, C₂-C₆-Alkinyl oder C₃-C₁₀-Cycloalkyl; und
R⁴ Wasserstoff, Cyano, C₁-C₆-Alkyl, C₂-C₆-Alkenyl, C₂-C₆-Alkinyl, C₁-C₆-Alkoxy oder C₃-C₁₀-Cycloalkyl;
B Wasserstoff,
ggf. subst. C₁-C₆-Alkyl, C₂-C₆-Alkenyl, C₂-C₆-Alkinyl, C₃-C₁₀-Cycloalkyl, C₅-C₁₄-Cycloalkenyl, Aryl, Hetaryl, Heterocyclyl, Aryl-C₁-C₆-alkyl, Hetaryl-C₁-C₆-alkyl, C₃-C₁₀-Cycloalkyl-C₁-C₆-alkyl oder C₅-C₁₄-Cycloalkenyl-C₁-C₆-alkyl;
R¹ SR⁵, C₁-C₆-Alkoxy, C₂-C₆-Alkenyloxy, C₂-C₆-Alkinyloxy, C₃-C₁₀-Cycloalkoxy, C₅-C₁₄-Cycloalkenyloxy oder C₁-C₆-Alkoxycarbonyloxy; und
R⁵ C₁-C₆-Alkyl, Cyclopropyl, Cyclopropylmethyl oder Cyclobutyl;
wobei B nicht Wasserstoff bedeutet, wenn A für eine direkte Bindung steht;
ausgenommen die Carbamate der Formel I, in der
a)
X,Y Wasserstoff;
Z Methyl;
R¹ Methoxy;
A-B Methoxy; und
b)
X,Y Wasserstoff;
Z Amino;
R¹ Ethoxycarbonyloxy;
A-B Methyl und
c)
X,Y 4-Methyl, 6-Methyl;
Z Methyl;
R¹ Methoxy;
A-B Methyl und
d)
X,Y Wasserstoff;
Z Methyl;
R¹ Methoxy;
A-B Phenyl
bedeuten;
und ihre pflanzenverträglichen Säureadditionsprodukte und Basenadditionsprodukte.

2. Carbamate der Formel I gemäß Anspruch 1, in der B die folgende Bedeutung hat:
Wasserstoff,
C₁-C₆-Alkyl, C₂-C₆-Alkenyl, C₂-C₆-Alkinyl, C₃-C₁₀-Cycloalkyl, C₅-C₁₄-Cycloalkenyl,
C₆-, C₁₀- oder C₁₄-Aryl,
Hetaryl mit 5 bis 14 Ringatomen, davon 1 bis 4 Heteroatome aus der Gruppe N, O oder S, Heterocyclyl mit 5 bis 14 Ringatomen, davon 1 bis 4 Heteroatome aus der Gruppe N, O oder S,
C₆-, C₁₀- oder C₁₄-Aryl-C₁-C₆-alkyl,
Fünf- bis 14-Ring-Hetaryl-C₁-C₆-alkyl, C₃-C₁₀-Cycloalkyl-C₁-C₆-alkyl oder C₅-C₁₄-Cycloalkenyl-C₁-C₆-alkyl,
wobei die Reste: Cycloalkyl, Cycloalkenyl, Aryl, Hetaryl und Heterocyclyl 1 bis 4 gleiche oder verschiedene Substituenten R⁶ tragen können;
R⁶ kann mit 1 bis 4 gleichen oder verschiedenen Substituenten R⁷ substituiert sein und R⁶ bedeutet Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Alkoxy, Alkenyloxy, Alkinyloxy, Cycloalkyloxy, Aryloxy, Hetaryloxy, Heterocyclyloxy, Cycloalkenyloxy, Alkoximino, Alkenyloximino, Alkinyloximino, Cycloalkyloximino, Cycloalkenyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Alkylaminocarbonyl, Dialkylaminocarbonyl, Alkenylaminocarbonyl, Dialkenylaminocarbonyl, Alkylthio, Alkenylthio, Alkinylthio, Cycloalkylthio, Arylthio, Hetarylthio, Heterocyclylthio, Cycloalkenylthio, Alkylamino, Alkenylamino, Alkinylamino, Cycloalkylamino, Arylamino, Hetarylamino, Heterocyclylamino, Cycloalkenylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl oder Cycloalkenylsulfinyl;
R⁷ bedeutet Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Alkoxy, Alkenyloxy, Alkinyloxy, Cycloalkyloxy, Aryloxy, Hetaryloxy, Heterocyclyloxy, Cycloalkenyloxy, Alkoximino, Alkenyloximino, Alkinyloximino, Cycloalkyloximino, Cycloalkenyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Alkylaminocarbonyl, Dialkylaminocarbonyl, Alkenylaminocarbonyl, Dialkenylaminocarbonyl, Alkylthio, Alkenylthio, Alkinylthio, Cycloalkylthio, Arylthio, Hetarylthio, Heterocyclylthio, Cycloalkenylthio, Alkylamino, Alkenylamino, Alkinylamino, Cycloalkylamino, Arylamino, Hetarylamino, Heterocyclylamino, Cycloalkenylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl oder Cycloalkenylsulfinyl.

3. Carbamate der Formel I gemäß Anspruch 1, in der Z für Methoxy und R¹ für OR^{y} steht, wobei
R^{y} für C₁-C₆-Alkyl, C₂-C₆-Alkenyl, C₂-C₆-Alkinyl, C₃-C₁₀-Cycloalkyl, C₅-C₁₄-Cycloalkenyl oder C₁-C₆-Alkoxycarbonyl; und
B für ggf. subst. C₁-C₆-Alkyl, C₂-C₆-Alkenyl, C₂-C₆-Alkinyl, C₃-C₁₀-Cycloalkyl, C₅-C₁₄-Cycloalkenyl, Aryl, Hetaryl oder Heterocyclyl steht.

4. Carbamate der Formel I gemäß Anspruch 3, in der X und Y für Wasserstoff stehen.

5. Carbamate der Formel I gemäß Anspruch 4, in der A für CH₂O steht.

6. Carbamate der Formel I gemäß Anspruch 4, in der A für CH₂O-N=C(CH₃) steht.

7. Fungizid, enthaltend einen inerten Trägerstoff und eine fungizid wirksame Menge eines Carbamats der Formel I gemäß Anspruch 1, oder dessen pflanzenverträgliches Säureadditionsprodukt und Basenadditionsprodukt.

8. Verfahren zur Bekämpfung von Pilzen, **dadurch gekennzeichnet, daß** man die Pilze oder die von Pilzbefall bedrohten Pflanzen, Saatgut, Materialien oder den Erdboden mit einer fungizid wirksamen Menge einer Verbindung I gemäß Anspruch 1, oder dessen pflanzenverträgliches Säureadditionsprodukt und Basenadditionsprodukt behandelt.

9. Verbindungen der Formel A 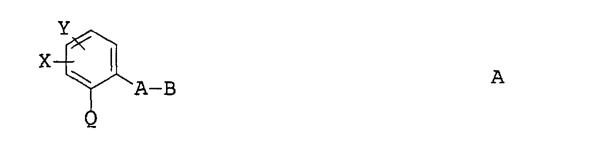 in der X und Y Wasserstoff bedeuten, A und B die in Anspruch 5 und 6 gegebene Bedeutung haben und Q für die folgenden Gruppen steht:
NHOH und N(OH)-CO-Z, wobei Z Methoxy bedeutet, ausgenommen die Verbindung der Formel A, in der
A CH₂O;
B Wasserstoff und
Q NHOH bedeuten.

10. Verbindungen der Formel B 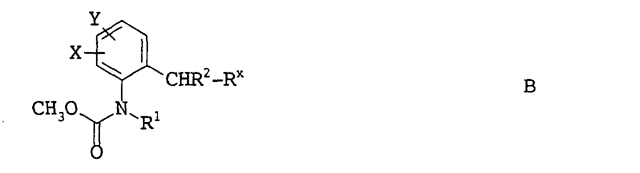 in der X und Y Wasserstoff bedeuten und R¹ und R² die in Anspruch 1 gegebene Bedeutung haben und R^{x} für Wasserstoff, ein Brom- oder Chloratom oder eine der folgenden Gruppen steht: [P(C₆H₅)₃⁺ Cl-], [P(C₆H₅)₃⁺ Br⁻] oder PO(OAlkyl)₂.

11. Verbindungen der Formel 31 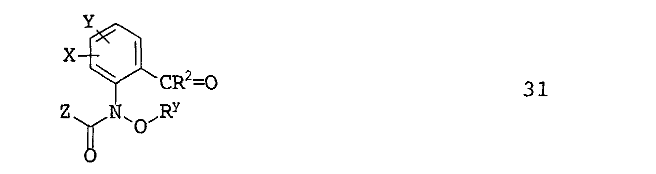 in der die Substituenten X, Y, Z und R² die in Anspruch 1 gegebene Bedeutung haben und RY wie in Anspruch 3 definiert ist.

12. Verbindungen der Formel C 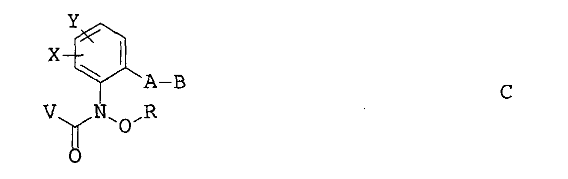 in der die Substituenten X, Y, A und B die in Anspruch 1 gegebene Bedeutung haben und die Reste R und V die folgende Bedeutung haben:
R Wasserstoff oder eine Gruppe R^{y}, wobei
R^{y} die in Anspruch 3 gegebene Bedeutung hat,
V Trichlormethoxy, Phenoxy oder p-Nitrophenoxy.

## Claims

1. A carbamate of the formula I 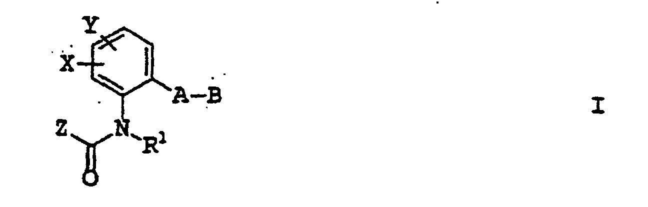 where
Z is methoxy, amino, methylamino, dimethylamino, methyl, ethyl, trifluoromethyl or trichloromethyl;
X and Y are each hydrogen, fluorine, chlorine, bromine, trifluoromethyl, cyano, nitro, C₁-C₆-alkyl, C₂-C₆-alkenyl, C₂-C₆-alkynyl, C₁-C₆-alkoxy, C₂-C₆-alkenyloxy or C₂-C₆-alkynyloxy or
X and Y together are condensed to form an unsubstituted or substituted aromatic or heteroaromatic, alicyclic or heterocyclic, partially or completely hydrogenated ring;
A is a direct bond, O, S, CR²=CR³, CHR²O, CHR²S, CHR²-ON=CR⁴, CR²=NO, ON=CR⁴, C≡C, CHR²-CHR³, CHR²O-CO or OCHR²;
R² and R³ are each hydrogen, C₁-C₆-alkyl, C₂-C₆-alkenyl, C₂-C₆-alkynyl or C₃-C₁₀-cycloalkyl; and
R₄ is hydrogen, cyano, C₁-C₆-alkyl, C₂-C₆-alkenyl, C₂-C₆-alkynyl, C₁-C₆-alkoxy or C₃-C₁₀-cycloalkyl;
B is hydrogen,
unsubstituted or substituted C₁-C₆-alkyl, C₂-C₆-alkenyl, C₂-C₆-alkynyl, C₃-C₁₀-cycloalkyl, C₅-C₁₄-cycloalkenyl, aryl, hetaryl, heterocyclyl, aryl-C₁-C₆-alkyl, hetaryl-C₁-C₆-alkyl, C₃-C₁₀-cycloalkyl-C₁-C₆-alkyl or C₅-C₁₄-cycloalkenyl-C₁-C₆-alkyl;
R₁ is SR⁵, C₁-C₆-alkoxy, C₂-C₆-alkenyloxy, C₂-C₆-alkynyloxy, C₃-C₁₀-cycloalkoxy, C₅-C₁₄-cycloalkenyloxy or C₁-C₆-alkoxycarbonyloxy; and
R⁵ is C₁-C₆-alkyl, cyclopropyl, cyclopropylmethyl or cyclobutyl;
B not being hydrogen when A is a direct bond;
with the exception of a carbamate of the formula I where
a)
X and Y are each hydrogen;
Z is methyl;
R¹ is methoxy;
A-B is methoxy; and
b)
X and Y are each hydrogen;
Z is amino;
R¹ is ethoxycarbonyloxy;
A-B is methyl and
C)
X and Y are each 4-methyl or 6-methyl;
Z is methyl;
R¹ is methoxy;
A-B is methyl and
d)
X and Y are each hydrogen;
Z is methyl;
R' is methoxy;
A-B is phenyl;
and its plant-tolerated acid addition products and base addition products.

2. A carbamate of the formula I as claimed in claim 1, where B is
hydrogen,
C₁-C₆-alkyl, C₂-C₆-alkenyl, C₂-C₆-alkynyl, C₃-C₁₀-cycloalkyl, C₅-C₁₄-cycloalkenyl,
C₆-, C₁₀- or C₁₄-aryl,
hetaryl having 5 to 14 ring atoms, including 1 to 4 heteroatoms selected from the group consisting of N, O and S, heterocyclyl having 5 to 14 ring atoms, including 1 to 4 heteroatoms selected from the group consisting of N, O and S,
C₆-, C₁₀- or C₁₄-aryl-C₁-C₆-alkyl,
hetaryl-C₁-C₆-alkyl having a 5- to 14-membered ring, C₃-C₁₀-cycloalkyl-C₁-C₆-alkyl or C₅-C₁₄-cycloalkenyl-C₁-C₆-alkyl,
where the radicals cycloalkyl, cycloalkenyl, aryl, hetaryl and heterocyclyl may carry 1 to 4 identical or different substituents R⁶;
R⁶ may be substituted by 1 to 4 identical or different substituents R⁷, and R⁶ is hydrogen, halogen, cyano, nitro, haloalkyl, alkyl, haloalkoxy, alkenyl, alkynyl, cycloalkyl, aryl, hetaryl, heterocyclyl, cycloalkenyl, alkoxy, alkenyloxy, alkynyloxy, cycloalkyloxy, aryloxy, hetaryloxy, heterocyclyloxy, cycloalkenyloxy, alkoximino, alkenyloximino, alkynyloximino, cycloalkyloximino, cycloalkenyloximino, aryloximino, hetaryloximino, heterocyclyloximino, alkoxy-carbonyl, alkenyloxycarbonyl, alkynyloxycarbonyl, cycloalkyloxycarbonyl, aryloxycarbonyl, hetaryl-oxycarbonyl, heterocyclyloxycarbonyl, cyclo-alkenyloxycarbonyl, alkylaminocarbonyl, dialkyl-aminocarbonyl, alkenylamino-carbonyl, dialkenyl-aminocarbonyl, alkylthio, alkenylthio, alkynylthio, cycloalkylthio, arylthio, hetarylthio, heterocyclylthio, cycloalkenylthio, alkylamino, alkenylamino, alkynylamino, cycloalkylamino, arylamino, hetarylamino, heterocyclylamino, cycloalkenylamino, alkylcarbonyl, alkenylcarbonyl, alkynylcarbonyl, cycloalkylcarbonyl, arylcarbonyl, hetarylcarbonyl, heterocyclylcarbonyl, cycloalkenylcarbonyl, alkylsulfoxyl, alkenylsulfoxyl, alkynylsulfoxyl, cycloalkylsulfoxyl, arylsulfoxyl, hetarylsulfoxyl, heterocyclylsulfoxyl, cycloalkenylsulfoxyl, alkylsulfonyl, alkenylsulfonyl, alkynylsulfonyl, cycloalkylsulfonyl, arylsulfonyl, hetarylsulfonyl, heterocyclylsulfonyl, cyclo-alkenylsulfonyl, alkylsulfinyl, alkenylsulfinyl, alkynylsulfinyl, cycloalkylsulfinyl, arylsulfinyl, hetarylsulfinyl, heterocyclylsulfinyl or cycloalkenylsulfinyl;
R⁷ is hydrogen, halogen, cyano, nitro, haloalkyl, alkyl, haloalkoxy, alkenyl, alkynyl, cycloalkyl, aryl, hetaryl, heterocyclyl, cycloalkenyl, alkoxy, alkenyloxy, alkynyloxy, cycloalkyloxy, aryloxy, hetaryloxy, heterocyclyloxy, cycloalkenyloxy, alkoximino, alkenyloximino, alkynyloximino, cycloalkyloximino, cycloalkenyloximino, aryloximino, hetaryloximino, heterocyclyloximino, alkoxycarbonyl, alkenyloxycarbonyl, alkynyl-oxycarbonyl, cycloalkyloxycarbonyl, aryloxycarbonyl, hetaryloxycarbonyl, heterocyclyl-oxycarbonyl, cycloalkenyloxycarbonyl, alkylamino-carbonyl, dialkyl-aminocarbonyl, alkenylamino-carbonyl, dialkenylaminocarbonyl, alkylthio, alkenylthio, alkynylthio, cycloalkylthio, arylthio, hetarylthio, heterocyclylthio, cycloalkenylthio, alkylamino, alkenylamino, alkynylamino, cycloalkylamino, arylamino, hetarylamino, heterocyclylamino, cycloalkenylamino, alkylcarbonyl, alkenylcarbonyl, alkynylcarbonyl, cycloalkylcarbonyl, arylcarbonyl, hetarylcarbonyl, heterocyclylcarbonyl, cycloalkenylcarbonyl, alkylsulfoxyl, alkenylsulfoxyl, alkynylsulfoxyl, cycloalkylsulfoxyl, arylsulfoxyl, hetarylsulfoxyl, heterocyclylsulfoxyl, cycloalkenylsulfoxyl, alkylsulfonyl, alkenylsulfonyl, alkynylsulfonyl, cycloalkylsulfonyl, arylsulfonyl, hetarylsulfonyl, heterocyclylsulfonyl, cycloalkenylsulfonyl, alkylsulfinyl, alkenylsulfinyl, alkynylsulfinyl, cycloalkylsulfinyl, arylsulfinyl, hetarylsulfinyl, heterocyclylsulfinyl or cycloalkenylsulfinyl.

3. A carbamate of the formula I as claimed in claim 1, where Z is methoxy and R¹ is OR^{y},
R^{y} being C₁-C₆-alkyl, C₂-C₆-alkenyl, C₂-C₆-alkynyl, C₃-C₁₀-cycloalkyl, C₅-C₁₄-cycloalkenyl or C₁-C₆-alkoxycarbonyl; and
B being unsubstituted or substituted C₁-C₆-alkyl, C₂-C₆-alkenyl, C₂-C₆-alkynyl, C₃-C₁₀-cycloalkyl, C₅-C₁₄-cycloalkenyl, aryl, hetaryl or heterocyclyl.

4. A carbamate of the formula I as claimed in claim 3, where X an Y are each hydrogen.

5. A carbamate of the formula I as claimed in claim 4, where A is CH₂O.

6. A carbamate of the formula I as claimed in claim 4, where A is CH₂O-N=C(CH₃).

7. A fungicide containing an inert carrier and a fungicidal amount of a carbamate of the formula I as claimed in claim 1 or its plant-tolerated acid addition product or base addition product.

8. A method for controlling fungi, wherein the fungi or the plants, seeds, materials threatened by fungal attack or the soil is or are treated with a fungicidal amount of a compound I as claimed in claim 1 or its plant-tolerated acid addition product or base addition product.

9. A compound of the formula A 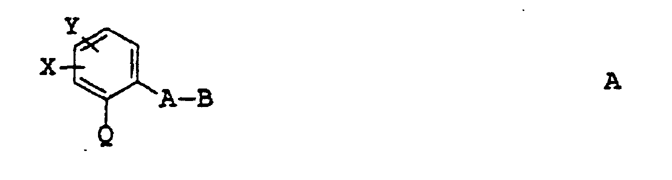 where X and Y are each hydrogen, A and B have the meanings stated in claims 5 and 6 and Q is
NHOH or N(OH)-CO-Z, Z being methoxy, with the exception of the compound of the formula A, where
A is CH₂-O;
B is hydrogen and
Q is NHOH.

10. A compound of the formula B 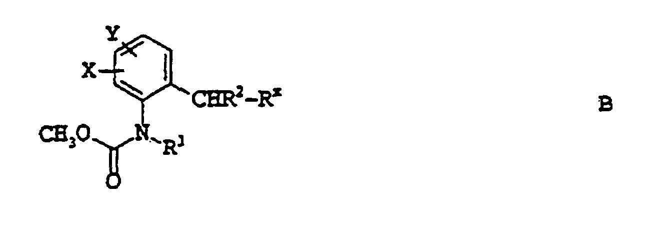 where X and Y are each hydrogen and R¹ and R² have the meanings stated in claim 1 and R^{x} is hydrogen, bromine or chlorine or one of the following groups: [P(C₆H₅)₃⁺Cl⁻], [P(C₆H₅)₃⁺Br⁻] or PO(OAlkyl)₂.

11. A compound of the formula 31 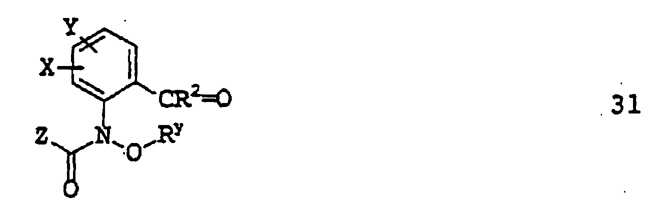 where X, Y, Z and R² have the meanings stated in claim 1 and R^{y} is as defined in claim 3.

12. A compound of the formula C 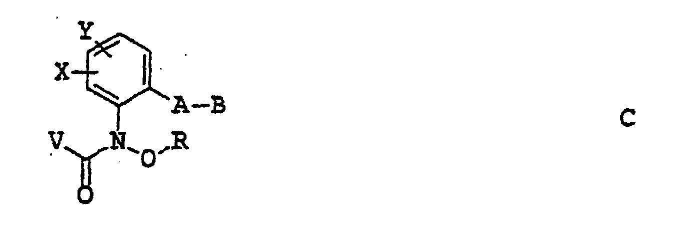 where X, Y, A and B have the meanings stated in claim 1 and
R is hydrogen or a group R⁷,
R^{y} having the meanings stated in claim 3, and
V is trichloromethoxy, phenoxy or p-nitrophenoxy.

## Revendications

1. Carbamates de formule 1 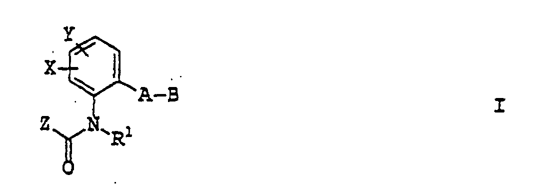 dans laquelle les substituants ont la signification suivante:
Z méthoxy, amino, méthylamino, diméthylamino, méthyle, éthyle, trifluorométhyle ou trichlorométhyle;
X,Y hydrogène, fluor, chlore, brome, trifluorométhyle, cyano, nitro, alkyle en C₁-C₆, alcényle en C₂-C₆, alcynyle en C₂-C₆, alcoxy en C₁-C₆, alcényloxy en C₂-C₆, alcynyloxy en C₂-C₆, ou
X,Y sont ensemble condensés en un noyau aromatique ou hétéroaromatique, alicyclique ou hétérocyclique, partiellement ou totalement hydrogéné et éventuellement substitué;
A une liaison directe, O, S, CR²=CR³, CHR²O, CHR²S, CHR²-ON=CR⁴, CR²=NO, ON=CR⁴, C≡C, CHR²-CHR³, CHR²O-CO ou OCHR²;
R²,R³ hydrogène, alkyle en C₁-C₆, alcényle en C₂-C₆, alcynyle en C₂-C₆ ou cycloalkyle en C₃-C₁₀; et
R⁴ hydrogène, cyano, alkyle en C₁-C₆, alcényle en C₂-C₆, alcynyle en C₂-C₆, alcoxy en C₁-C₆ ou cycloalkyle en C₃-C₁₀;
B hydrogène,
alkyle en C₁-C₆, alcényle en C₂-C₆, alcynyle en C₂-C₆, cycloalkyle en C₃-C₁₀, cycloalcényle en C₅-C₁₄, aryle, hétaryle, hétérocyclyle, aryl-(alkyle en C₁-C₆), hétaryl(alkyle en C₁-C₆), (cycloalkyl en C₃-C₁₀)-alkyle en C₁-C₆) ou (cycloalcényl en C₅-C₁₄)-alkyle en C₁-C₆;
R¹ SR⁵, alcoxy en C₁-C₆, alcényloxy en C₂-C₆, alcynyloxy en C₂-C₆, cycloalcoxy en C₃-C₁₀, cycloalcényloxy en C₅-C₁₄ ou (alcoxy en C₁-C₆)carbonyloxy; et
R⁵ alkyle en C₁-C₆, cyclopropyle, cyclopropylméthyle ou cyclobutyle;
tandis que B ne désigne pas l'hydrogène quand A représente une liaison directe;
à l'exception des carbamates de formule I, dans laquelle les substituants sont
a)
X,Y hydrogène;
Z méthyle;
R¹ méthoxy;
A-B méthoxy; et
b)
X,Y hydrogène;
Z amino;
R¹ éthoxycarbonyloxy;
A-B méthyle; et
c)
X,Y 4-méthyle, 6-méthyle;
Z méthyle;
R¹ méthoxy;
A-B méthyle; et
d)
X,Y hydrogène;
Z méthyle;
R¹ méthoxy;
A-B phényle;
et leur produits d'addition avec des acides et leurs produits d'additions avec des bases, tolérés par les végétaux.

2. Carbamates de formule I selon la revendication 1, dans laquelle B a la signification suivante:
hydrogène,
alkyle en C₁-C₆, alcényle en C₂-C₆, alcynyle en C₂-C₆, cycloalkyle en C₃-C₁₀, cycloalcényle en C₅-C₁₄,
aryle en C₆, C₁₀ ou C₁₄,
hétaryle ayant 5 à 14 atomes dans le cycle, dont 1 à 4 hétéroatomes du groupe N, O ou S, hétérocyclyle ayant 5 à 14 atomes de carbone dans le cycle, dont 1 à 4 hétéroatomes du groupe N, O ou S,
(aryl en C₆, C₁₀ ou C₁₄)-alkyle en C₁-C₆,
(hétaryl à cycle en 5 à 14)-alkyle en C₁-C₆, (cycloalkyl en C₃-C₁₀)-alkyle en C₁-C₆ ou (cycloalcényl en C₅-C₁₄)-alkyle en C₁-C₆,
tandis que les restes: cycloalkyle, cycloalcényle, aryle, hétaryle et hétérocyclyle peuvent porter 1 à 4 substituants R⁴ identiques ou différents;
R⁶ peut être substitué avec 1 à 4 substituants R⁷ identiques ou différents et R⁶ désigne un groupe: hydrogène, halogène, cyano, nitro, halogénoalkyle, alkyle, halogénoalcoxy, alcényle, alcynyle, cycloalkyle, aryle, hétaryle, hétérocyclyle, cycloalcényle, alcoxy, alcényloxy, alcynyloxy, cycloalkyloxy, aryloxy, hétaryloxy, hétérocyclyloxy, cycloalcényloxy, alcoximino, alcényloximino, alcynyloximino, cycloalkyloximino, cycloalcényloximino, aryloximino, hétaryloximino, hétérocyclyloximino, alcoxycarbonyle, alcényloxycarbonyle, alcynyloxycarbonyle, cycloalkyloxycarbonyle, aryloxycarbonyle, hétaryloxycarbonyle, hétérocyclyloxycarbonyle, cycloalcényloxycarbonyle, alkylaminocarbonyle, dialkylaminocarbonyle, alcénylaminocarbonyle, dialcénylaminocarbonyle, alkylthio, alcénylthio, alcynylthio, cycloalkylthio, arylthio, hétarylthio, hétérocyclylthio, cycloalcénylthio, alkylamino, alcénylamino, alcynylamino, cycloalkylamino, arylamino, hétarylamino, hétérocyclylamino, cycloalcénylamino, alkylcarbonyle, alcénylcarbonyle, alcynylcarbonyle, cycloalkylcarbonyle, arylcarbonyle, hétarylcarbonyle, hétérocyclylcarbonyle, cycloalcénylcarbonyle, alkylsulfoxyle, alcénylsulfoxyle, alcynylsulfoxyle, cycloalkylsulfoxyle, arylsulfoxyle, hétarylsulfoxyle, hétérocyclylsulfoxyle, cycloalcénylsulfoxyle, alkylsulfonyle, alcénylsulfonyle, alcynylsulfonyle, cycloalkylsulfonyle, arylsulfonyle, hétarylsulfonyle, hétérocyclylsulfonyle, cycloalcénylsulfonyle, alkylsulfinyle, alcénylsulfinyle, alcynylsulfinyle, cycloalkylsulfinyle, arylsulfinyle, hétarylsulfinyle, hétérocyclylsulfinyle ou cycloalcénylsulfinyle;
R⁷ désigne un groupe: hydrogène, halogène, cyano, nitro, halogénoalkyle, alkyle, halogénoalcoxy, alcényle, alcynyle, cycloalkyle, aryle, hétaryle, hétérocyclyle, cycloalcényle, alcoxy, alcényloxy, alcynyloxy, cycloalkyloxy, aryloxy, hétaryloxy, hétérocyclyloxy, cycloalcényloxy, alcoximino, alcényloximino, alcynyloximino, cycloalkyloximino, cycloalcényloximino, aryloximino, hétaryloximino, hétérocyclyloximino, alcoxycarbonyle, alcényloxycarbonyle, alcynyloxycarbonyle, cycloalkyloxycarbonyle, aryloxycarbonyle, hétaryloxycarbonyle, hétérocyclyloxycaarbonyle, cycloalcényloxycarbonyle, alkylaminocarbonyle, dialkylaminocarbonyle, alcénylaminocarbonyle, dialcénylaminocarbonyle, alkylthio, alcénylthio, alcynylthio, cycloalkylthio, arylthio, hétarylthio, hétérocyclylthio, cycloalcénylthio, alkylamino, alcénylamino, alcynylamino, cycloalkylamino, arylamino, hétarylamino, hétérocyclylamino, cycloalcénylamino, alkylcarbonyle, alcénylcarbonyle, alcynylcarbonyle, cycloalkylcarbonyle, arylcarbonyle, hétarylcarbonyle, hétérocyclylcarbonyle, cycloalcénylcarbonyle, alkylsulfoxyle, alcénylsulfoxyle, alcynylsulfoxyle, cycloalkylsulfoxyle, arylsulfoxyle, hétarylsulfoxyle, hétérocyclylsulfoxyle, cycloalcénylsulfoxyle, alkylsulfonyle, alcénylsulfonyle, alcynylsulfonyle, cycloalkylsulfonyle, arylsulfonyle, hétarylsulfonyle, hétérocyclylsulfonyle, cycloalcénylsulfonyle, alkylsulfinyle, alcénylsulfinyle, alcynylsulfinyle, cycloalkylsulfinyle, arylsulfinyle, hétarylsulfinyle, hétérocyclylsulfinyle ou cycloalcénylsulfinyle.

3. Carbamates de formule I selon la revendication 1, dans laquelle Z désigne le méthoxy et R¹ représente OR^{y}, tandis que
R^{y} désigne un groupe alkyle en C₁-C₆, alcényle en C₂-C₆, alcynyle en C₂-C₆, cycloalkyle en C₃-C₁₀, cycloalcényle en C₅-C₁₄ ou (alcoxy en C₁-C₆)carbonyle; et
B désigne un groupe alkyle en C₁-C₆, alcényle en C₂-C₆, alcynyle en C₂-C₆, cycloalkyle en C₃-C₁₀, cycloalcényle en C₅-C₁₄, aryle, hétaryle ou hétérocyclyle.

4. Carbamates de formule I selon la revendication 1, dans laquelle X et Y désignent l'hydrogène.

5. Carbamates de formule I selon la revendication 4, dans laquelle A représente CH₂O.

6. Carbamates de formule I selon la revendication 4, dans laquelle A représente CH₂O-N=C(CH₃).

7. Fongicide, contenant un véhicule inerte et une quantité active du point de vue fongicide d'un carbamate de formule I selon la revendication 1, ou de son produit d'addition avec des acides et produit d'addition avec des bases, toléré par les végétaux.

8. Procédé pour la lutte contre les mycètes, **caractérisé par le fait que** l'on traite les mycètes ou les végétaux, semences, matières ou sols menacés d'une attaque par les mycètes, avec une quantité efficace du point de vue fongicide d'un composé I selon la revendication 1, ou de son produit d'addition avec des acides et produit d'addition avec des bases, toléré par les végétaux.

9. Composés de formule A 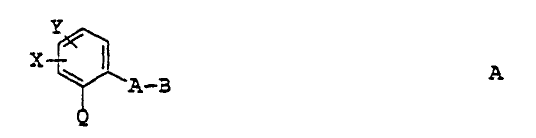 dans laquelle X et Y désignent l'hydrogène, A et B ont la signification indiquée dans la revendication 5 et 6 et Q désigne les groupes suivants:
NHOH et N(OH)-CO-Z, tandis que Z désigne le méthoxy, à l'exception du composé de formule A dans lequel
A est CH₂O;
B est l'hydrogène et
Q désigne NHOH.

10. Composés de formule B 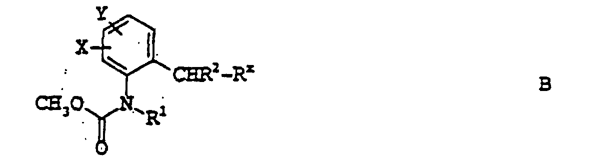 dans laquelle X et Y désignent l'hydrogène et R¹ e R² ont la signification indiquée dans la revendication 1 et R^{x} désigne l'hydrogène, un atome de brome ou de chlore ou l'un des groupes suivants: [P(C₆H₅)₃⁺Cl⁻], [P(C₆H₅)₃⁺Br⁻] ou PO(Oalkyle)₂.

11. Composés de formule 31 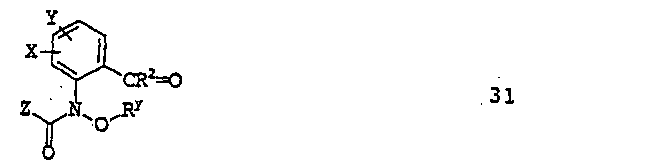 dans laquelle les substituants X, Y, Z et R² ont la signification indiquée dans la revendication 1 et R^{y} est tel que défini dans la revendication 3.

12. Composés de formule C 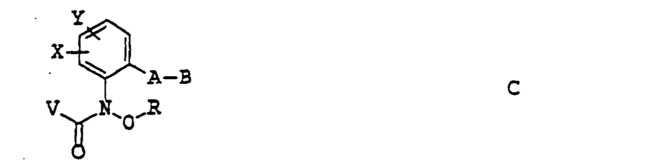 dans laquelle les substituants X, Y, A et B ont la signification indiquée dans la revendication 1 et les restes R et V ont la signification suivante:
R hydrogène ou un groupe R^{y}, tandis que
R^{y} a la signification indiquée dans la revendication 3,
V trichlorométhoxy, phénoxy ou p-nitrophénoxy.
